# EUROPEAN PATENT SPECIFICATION

(11) **EP 4 043 447 B1**
(45) Date of publication and mention of the grant of the patent: **21.01.2026**
(21) Application number: 20860955.2
(22) Date of filing: 03.09.2020
(51) Int. Cl.: C07J 53/00, A61P 9/04, A61P 9/00, A61K 31/341, A61K 31/34, A61K 31/58, C07D 307/04

(54) **HALOGENATED TETRACYCLIC TRITERPENE DERIVATIVE, PREPARATION AND APPLICATION THEREOF**
HALOGENIERTE TETRACYCLISCHE TRITERPENDERIVATE, HERSTELLUNG UND ANWENDUNG DAVON
DÉRIVÉ TRITERPÈNE TÉTRACYCLIQUE HALOGÉNÉ, SA PRÉPARATION ET SON APPLICATION

(30) Priority: 06.09.2019 CN 201910841645
(43) Date of publication of application: 17.08.2022
(73) Proprietor: Shanghai Qingdong Biotechnology Co., Ltd, Shanghai 200120 (CN)
(72) Inventor: ZHANG, Weidong, Shanghai 200003 (CN); SUN, Qingyan, Shanghai 200003 (CN); YUAN, Hu, Shanghai 200003 (CN); LIU, Xia, Shanghai 200003 (CN)
(74) Representative: Vitina, Maruta
(86) International application number: PCT/CN2020/113149
(87) International publication number: WO 2021/043194

(56) References cited:
- WO-A1-2008/063128
- WO-A1-2010/135247
- WO-A1-2011/109657
- CN-A- 1 642 558
- CN-A- 1 793 132
- CN-A- 102 448 308
- CN-A- 102 939 011

## Description

### Technical Field

The invention relates to a series of tetracyclic triterpene derivatives and their preparation and application, in particular to a series of halogenated tetracyclic triterpene derivatives and their preparation and application.

### Background Art

Cardiovascular and cerebrovascular disease is a common disease that seriously threatens the health of human beings, especially the middle-aged and elderly people over 50 years old. Cardiovascular and cerebrovascular diseases are usually result from ischemia or hemorrhage in the heart, brain and systemic tissues caused by hyperlipidemia, blood viscosity, atherosclerosis, hypertension, etc. It has the characteristics of high morbidity, high disability rate and high mortality rate. Even if the most advanced and perfect treatment methods are used, more than 50% of cerebrovascular accident survivors cannot fully take care of themselves. The number of people dying of cardiovascular and cerebrovascular diseases in the world is as high as 15 million every year, ranking first among all causes of death. Therefore, the treatment of cardiovascular and cerebrovascular diseases has become an urgent need.

The main treatment drugs for cardiovascular and cerebrovascular diseases are chemical medicines, including antithrombotic drugs and neuroprotective agents. Although there are some Chinese medicines, there are no small molecular compounds extracted from traditional Chinese medicines with good curative effect, low toxicity and clear structure as active ingredients in clinical practice. Structural modification of natural products derived from natural medicines is one of the most effective approaches to new drug development so far. It is based on maintaining the basic skeletal structure of the natural products with only certain functional groups are chemically modified so as to achieve the purpose of improving the physicochemical properties, increasing the druggability or synergizing the effect and reducing the toxicity.

Heart failure refers to the dysfunction of the systolic and/or diastolic function of the heart. Specifically, the venous return blood volume cannot be fully discharged from the heart, resulting in blood stasis in the venous system and insufficient blood perfusion in the arterial system, called cardiac circulation disorder syndrome, which is manifested as pulmonary congestion and vena cava congestion. Heart failure is not an independent disease, but a terminal stage in the development of heart disease. The vast majority of heart failure begins with left heart failure, which first manifests as pulmonary circulation congestion. Heart failure is the final outcome of a variety of cardiovascular diseases, and its mechanisms are mainly related to the weakening of myocardial diastolic and systolic function, myocardial hypertrophy and fibrosis, activation of the neuro-endocrine system, reduction of myocardial cells and apoptosis.

The tetracyclic triterpenoid saponins of lanolin ester alcohols are natural products clinically used for the treatment of cardiovascular and cerebrovascular diseases such as coronary heart disease, heart failure and hypertension. It has a wide range of cardiovascular pharmacological effects, including lowering blood pressure, strengthening the heart, protecting myocardial cells and vascular endothelium, improving blood rheology, improving left ventricular configuration in patients with chronic heart failure, and up-regulating the expression of vascular and myocardial related genes. Its mechanism of action is related to inducing NO release, resisting lipid peroxidation, scavenging oxygen free radicals, resisting apoptosis, improving energy metabolism and reducing calcium overload. Therefore, such compounds have great potential to be developed as cardiovascular and cerebrovascular drugs. However, these compounds are poorly absorbed after oral administration, and their absolute bioavailability is low in mouse models (3.66% and 2.2% in two different experiments, Eur. J. Drug Metab. Ph., 2005, 30(4): 269-273; Basic. Clin. Pharmacol. Toxicol., 2004, 95(6): 295-298)). In vitro studies of Caco-2 cells suggest that the low absorption rate of these compounds are mainly due to its high molecular weight, low lipophilicity and paracellular transport characteristics (Eur. J. Drug Metab. Ph., 2006, 31(1): 5-10). The aglycones of these compounds also have similar pharmacological activities, their lipophilicity is higher than that of the corresponding glycosides, and the transmembrane absorption capacity is stronger, but their metabolic stability is poor. The metabolic half-lives in human and mouse liver microsomes are respectively 8.4±1.5 minutes and 12.0±4.2 minutes, their clearance are fast (Drug Metab. Pharmacokinet. 2010, 25 (5): 477-486), which limits its development and application. Therefore, it is of great significance to improve the druggability of these compounds for their further development and utilization.

The international application WO2010135247A1 describes compounds for treating diseases (also cardiovascular diseases) by an increase in telomerase activity in cells or tissue of a patient contacting said cell or tissue with an isolated compound including cycloastragenol, C6-(L)-Valyl- cycloastragenol, 6-(L)-Glutamate- cycloastragenol, C6-(L)-phenylalanyl- cycloastragenol.

### Summary of the Invention

The technical problem to be solved by the present invention is to overcome the above deficiencies by structural modifying and activity studies and providing medicines for treating cardiovascular and cerebrovascular diseases.

The present invention is as described in the attached claims. Any information not covered by claims is for information purposes only.

The present invention provides a series of halogenated tetracyclic triterpenoid derivative.

The halogenated tetracyclic triterpenoid derivatives provided by the present invention is (2aR,3R,4S,5aS,5bS,7S,7aR,11aR,12aS)-9,9-difluoro-3-((2R,5S)-5-(2-hydroxypropan-2-yl )-2-methyltetrahydrofuran-2-yl)-2a,5a,8,8-tetramethyltetradecahydro-1H,12H-cyclopenta[a ]cyclopropa[e]phenanthrene-4,7-diol (Compound 2), represented by the following general structural formula:

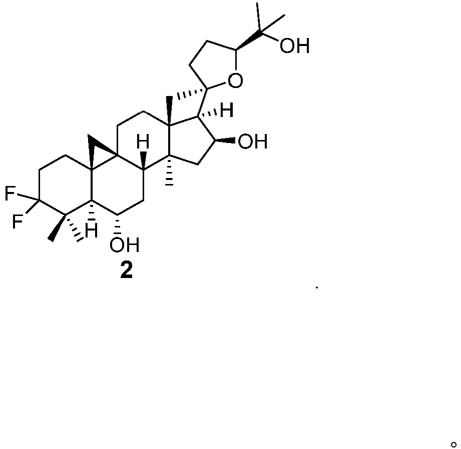

Another object of the present invention is to provide a halogenated tetracyclic triterpenoid derivative as a phosphate prodrug of compound 2 ((2aR,3R,4S,5aS,5bS,7S,7aR,11aR,12aS)-9,9-difluoro-4-hydroxy-3-((2R,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-phosphate, Compound 3). It is represented by the following general structural formula: 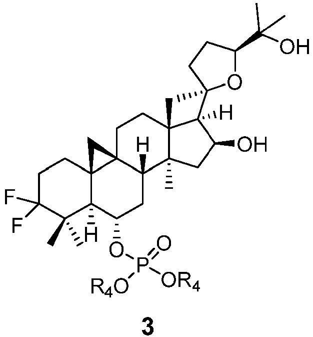 R₄ = Hydrogen, or Sodium, or Potassium, or Calcium, or Magnesium, or Zinc, or Ammonium, or Basic Amino Acids

The following compounds are preferred:

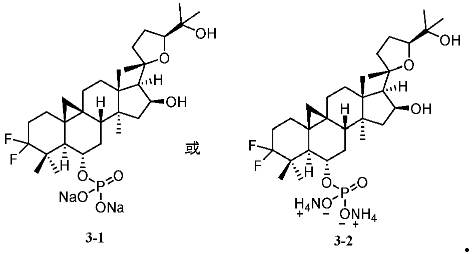

The present invention provides a preparation method of the phosphate prodrug of the compound 2 comprises the following steps, which are represented by the following reaction formula:

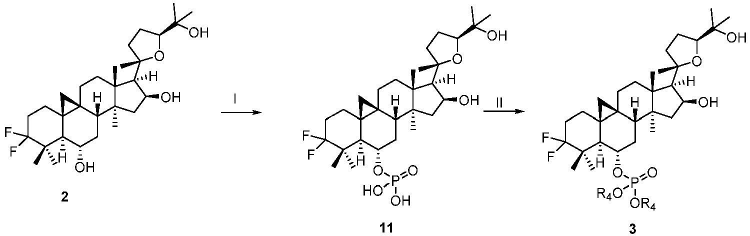

The method includes the following steps:
(I). Phosphate of 6-hydroxy: The reagent is phosphorus oxychloride, and the solvent is trimethyl phosphate. The molar ratio of compound 2 and phosphorus oxychloride is 1:1-1:15, preferably 1:3. The reaction temperature is -80°C-30 °C, preferably -5 °C to 10 °C.
(II). Salt-forming reaction: The reagent is an organic base or an inorganic base, and the organic base is selected from basic amino acids, ammonia water, methylamine, dimethylamine, diethylamine, triethylamine, pyridine, piperidine, pyrrole, ethylenediamine or butanediamine, while the inorganic base is selected from sodium hydroxide, sodium methoxide, potassium hydroxide, potassium carbonate, sodium carbonate, cesium carbonate, potassium bicarbonate, sodium acetate, potassium acetate, calcium carbonate, magnesium carbonate, sodium phosphate or potassium phosphate. The solvent is selected from tetrahydrofuran, 2-methyltetrahydrofuran, ethyl acetate, dichloromethane, dichloroethane, methanol, ethanol, isopropanol, tert-butanol, diethyl ether, methyl tert-butyl ether, acetonitrile or dioxane, preferably methanol. The reaction temperature is -20°C-50°C, preferably 0°C-20°C.

Another object of the present invention is to provide a halogenated tetracyclic triterpenoid derivative as a carboxylate prodrug of compound 2. ((2aR,3R,4S,5aS,5bS,7S,7aR,11aR,12aS)-9,9-difluoro-4-hydroxy-3-((2R,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-ester, Compound 4). It is represented by the following general structural formula: 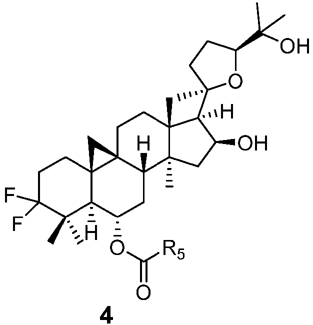 R₅ = amine, or alcohol, or amino acid, or peptide

The following compounds are preferred:

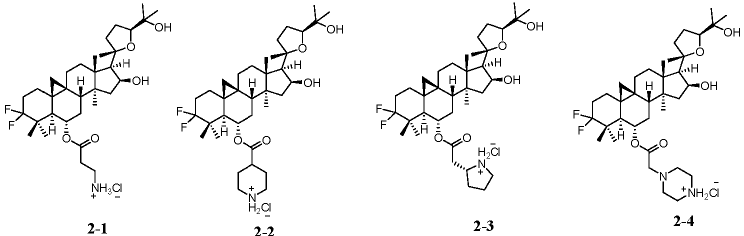

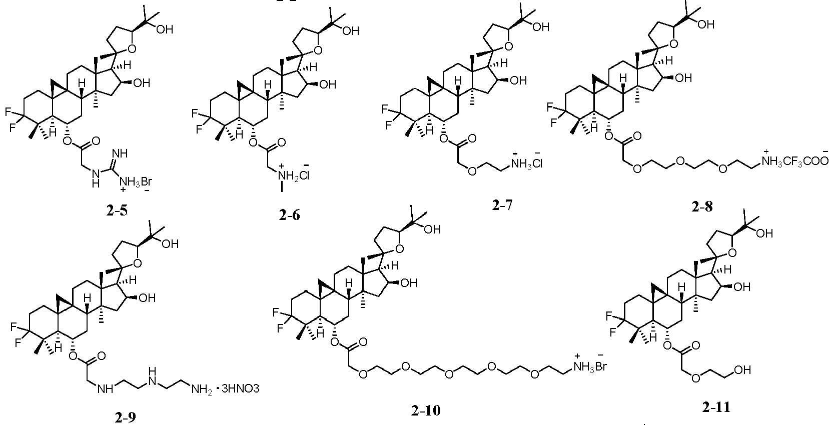

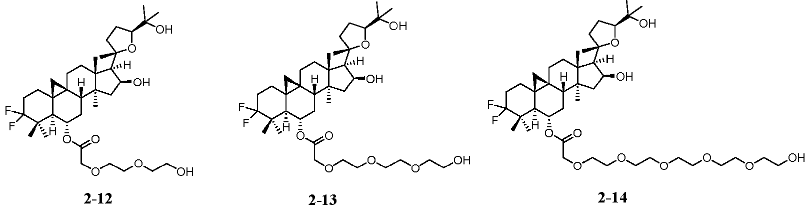

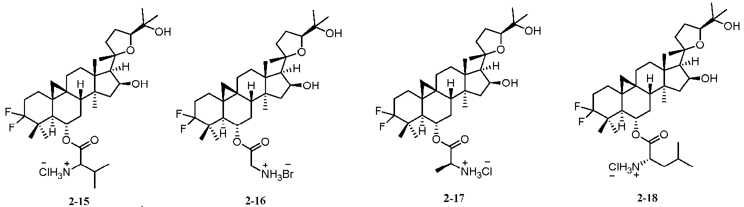

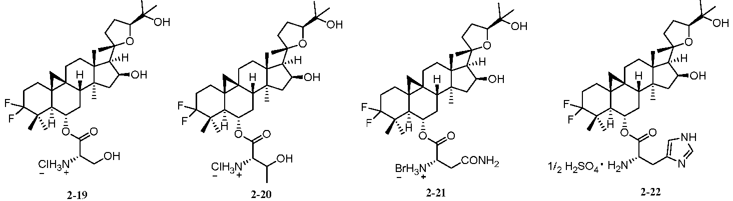

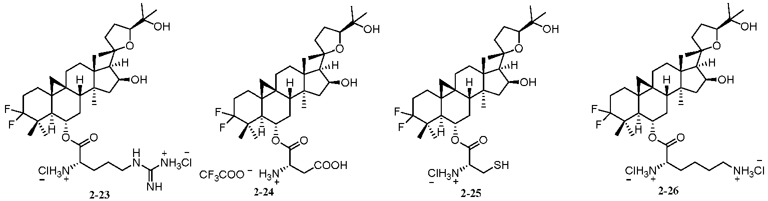

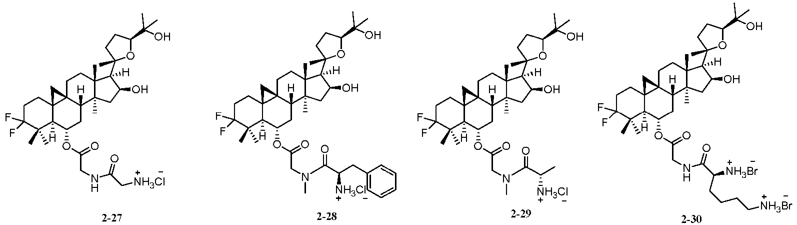

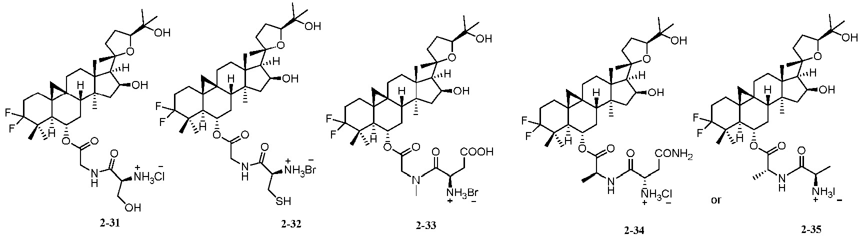

The preparation method of the carboxylate prodrug of the compound 2 is characterized in that the preparation method comprises the following steps which are represented by the following reaction formula:

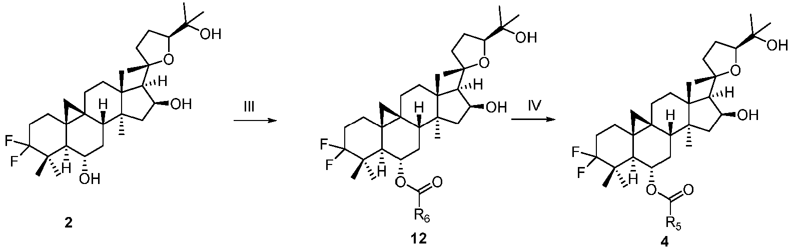

The method includes the following steps:
(III). Esterification: Compound 2 reacts with amines with a carboxylic acid group, polyethylene glycol monoacid, polyethylene diamine monoacid, nitrogen protected amino acid or peptide to get the corresponding ester. The condensing agent is n-propyl phosphoric anhydride, cyclohexylcarbodiimide (DCC), 1-ethyl-3(3-dimethylpropylamine)carbodiimide (EDCI), diisopropylcarbodiimide (DIC), carbonyldiimidazole or succinyl imine carbonate, preferably EDCI. The base is an organic base selected from diethylamine, triethylamine, pyridine, piperidine, imidazole, N,N-diisopropylethylamine, 4-pyrrolidinopyridine, 1,8-diazabicycloundec-7-ene, 4-dimethylaminopyridine or 1,4-diazabicyclo[2.2.2] octane, preferably 4-dimethylaminopyridine. The solvent is an aprotic solvent selected from toluene, xylene, tetrahydrofuran, 2-methyltetrahydrofuran, ethyl acetate, dichloromethane, dichloroethane, dimethylformamide, dimethylacetamide, dimethylsulfoxide, acetonitrile, dioxane or chloroform, preferably dichloromethane or dimethylformamide. The reaction temperature is -50°C-100°C, preferably -10 °C to 40 °C, particularly 0 °C to 20 °C.
(IV). Deprotection: The acid is an organic acid or an inorganic acid including hydrochloric acid, hydrobromic acid, hydroiodic acid, sulfuric acid, nitric acid or trifluoroacetic acid, preferably hydrochloric acid or trifluoroacetic acid. The solvent of the reaction is selected from tetrahydrofuran, 2-methyltetrahydrofuran, methanol, ethanol, isopropanol, tert-butanol, diethyl ether, methyl tert-butyl ether, acetonitrile or a mixture of one or more solvents in dioxane, preferably methanol or methanol-tetrahydrofuran mixed solvent.

Another object of the present invention is to provide the halogenated tetracyclic triterpenoid derivatives for use in the treatment of of a cardiovascular disease.

The halogenated tetracyclic triterpenoid derivatives of the present invention have been studied on pharmacokinetics, and they are relatively stable in rat whole blood. The absolute bioavailability after a single intragastric administration for male and female rats was 15.6% and 28.4% respectively. The mean bioavailability was 22%. The metabolic stability of liver microsomes for the compound 1, 2 and natural product cycloastragenol ((2aR,3R,4S,5aS,5bS,7S,7aR,9S,11aR,12aS)-3-((2S,5S)-5-(2-hydroxypropan-2-yl)-2-meth yltetrahydrofuran-2-yl)-2a,5a,8,8-tetramethyltetradecahydro-1H,12H-cyclopenta[a]cyclopr opa[e]phenanthrene-4,7,9-triol) in humans and rats were evaluated, the results showed that compounds 1 and 2 were more stable than cycloastragenol. Compound 2 significantly increased the ejection fraction (EF), short-axis shortening rate (FS) and CO per minute without increasing heart rate (HR) in mice with heart failure. Taking Enalapril and LZC696 (sacubitril valsartan sodium) as the positive control, compound 2 has a better therapeutic effect on chronic heart failure mice using EF (ejection fraction) as the gold standard, and compound 2 has the most significant effect at the dose of 10 mg/kg. Therefore, the halogenated tetracyclic triterpenoid derivatives provided by the present invention can be used for preparing cardiovascular medicines.

The invention provides the halogenated tetracyclic triterpene derivatives for use in the treatment of heart failure.

The invention provides the application of the halogenated tetracyclic triterpene derivatives in the preparation of medicines for treating chronic heart failure.

The medicine of the present invention is a pharmaceutical composition composed of halogenated tetracyclic triterpene derivatives as the only active ingredient and pharmaceutical excipients.

The active ingredient of the medicine of the present invention are halogenated tetracyclic triterpene derivatives. Preferably, it is selected from the following compounds:
(2aR,3R,4S,5aS,5bS,7S,7aR,9R,11aR,12aS)-9-halogen-3-((2R,5S)-5-(2-hydroxypropan-2-yl)-2-methyltetrahydrofuran-2-yl)-2a,5a,8,8-tetramethyltetradecahydro-1H,12H-cyclope nta[a]cyclopropa[e]phenanthrene-4,7-diol (compound 1),
(2aR,3R,4S,5aS,5bS,7S,7aR,11aR,12aS)-9,9-difluoro-3-((2R,5S)-5-(2-hydroxypropan-2 -yl)-2-methyltetrahydrofuran-2-yl)-2a,5a,8,8-tetramethyltetradecahydro-1H,12H-cyclopent a[a]cyclopropa[e]phenanthrene-4,7-diol (compound 2),
   (2aR,3R,4S,5aS,5bS,7S,7aR,11aR,12aS)-9,9-difluoro-4-hydroxy-3-((2R,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-phosphate (compound 3)
      or
   (2aR,3R,4S,5aS,5bS,7S,7aR,11aR,12aS)-9,9-difluoro-4-hydroxy-3-((2R,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-ester (compound 4).

The effective dose of the medicine of the present invention in mice is 3mg/kg-100mg/kg.

The halogenated tetracyclic triterpene derivatives provided by the invention overcome the disadvantage of poor drug-like properties of natural tetracyclic triterpenes. The glycoside fragments of tetracyclic triterpenes were removed in this invention and replaced the easily oxidized 3-hydroxyl group with halogen, yielding halogenated tetracyclic triterpene derivatives. Meanwhile, tha hydrophilic prodrugs of tetracyclic triterpenoid derivatives were prepared, which greatly improved the solubility in physiological media. The aqueous solubility and its solubility in simulated intestinal fluid of the compound 2 were greatly improved after 6-hydroxyl of compound 2 was linked by ester or phosphate prodrug, thus significantly improved the drugability. PK experiments showed that the peak concentration Cₘₐₓ in rats is 323 ng/mL, the area under the plasma concentration-time curve (AUC₀₋ₜ) is 1916 ng·h/mL, and the elimination half-life (t_{1/2}) is 2.89 h after oral administration of 10 mg/kg compound 2. While the detected peak concentration Cₘₐₓ and the exposure (AUC₀₋ₜ) of the parent drug HHQ16 increased significantly and the half-life was prolonged to varying degrees after intragastric administration of the prodrug of compound 2. The above results show that the prodrug of compound 2 with increased water solubility improve the absorption of the drug and release the parent drug efficiently, increasing its exposure in vivo, which means better druggability.

Through the structural modification of the natural tetracyclic triterpenes, the invention not only retains the biological activity, but also reduces the molecular weight and significantly improves the lipophilicity. Compared with the natural product tetracyclic triterpenes and their glycosides, the bioavailability of the halogenated tetracyclic triterpene derivatives provided by the invention is greatly increased, and the metabolic stability is also significantly improved, and can be used for preparing medicines for cardiovascular and cerebrovascular diseases, which has good clinical prospects and greater social benefits.

### Brief Description Of The Drawings

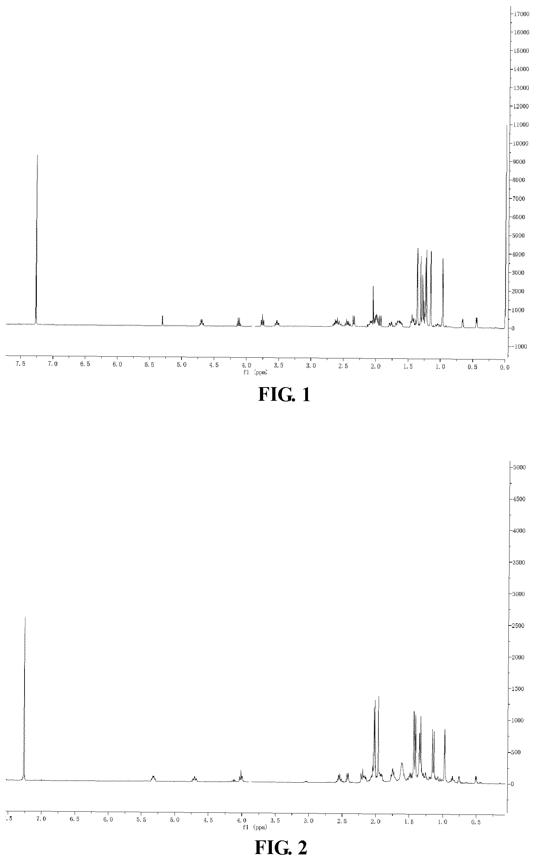

Figure 1 HNMR spectrum of compound 6
Figure 2 HNMR spectrum of compound 7

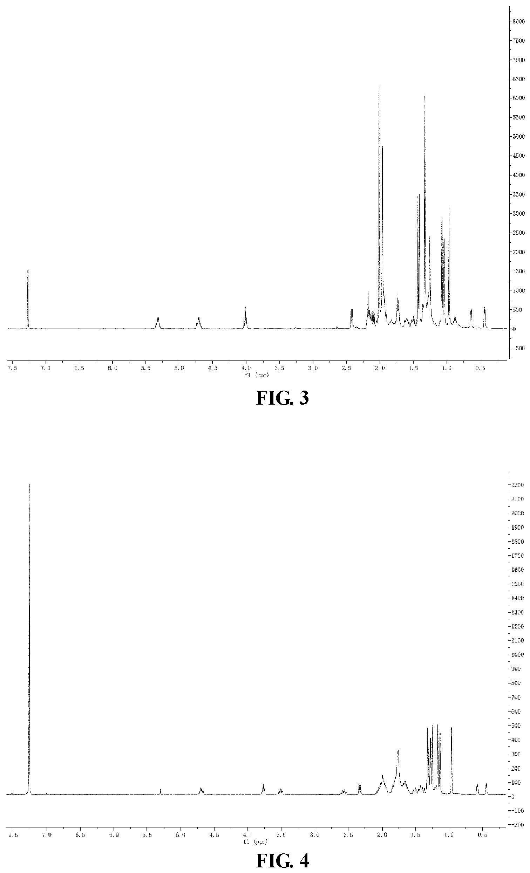

Figure 3 HNMR spectrum of compound 8
Figure 4 HNMR spectrum of compound 2

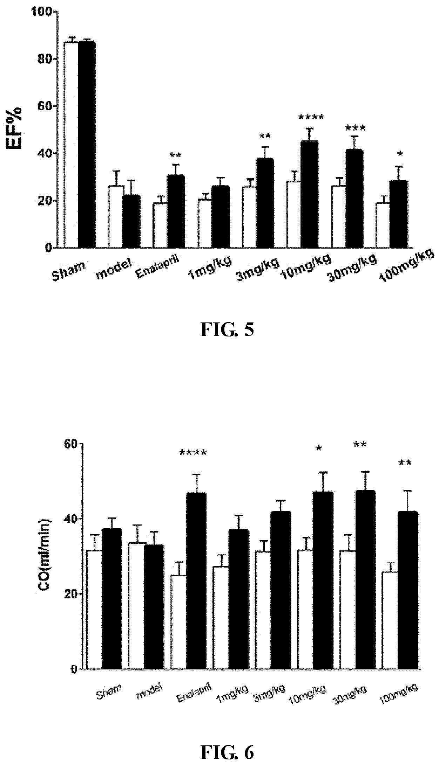

Figure 5. Ejection fraction (EF) of normal mice is 70-90%, and EF of heart failure mice is below 40%. EF is an important indicator for evaluating heart failure. Taking enalapril (p<0.01) as positive control, the EF value of 10mg/kg and 30mg/kg group mice was significantly increased (p<0.0005), while the 10mg/kg group had the highest increase (p<0.0001). The white box in the bar graph represents the 0th day, and the black box represents the 28th day.
Figure 6. Cardiac output per minute (CO), CO=SVxHR. CO decreased during heart failure and all increased after drug administration. The CO of positive control enalapril group (p<0.0001) had a very significant increase, and the CO of 10mg/kg, 30mg/kg and 100mg/kg group of compound 2 also significantly increased (p<0.01 or p<0.05). The white box in the bar graph represents the 0th day, and the black box represents the 28th day.

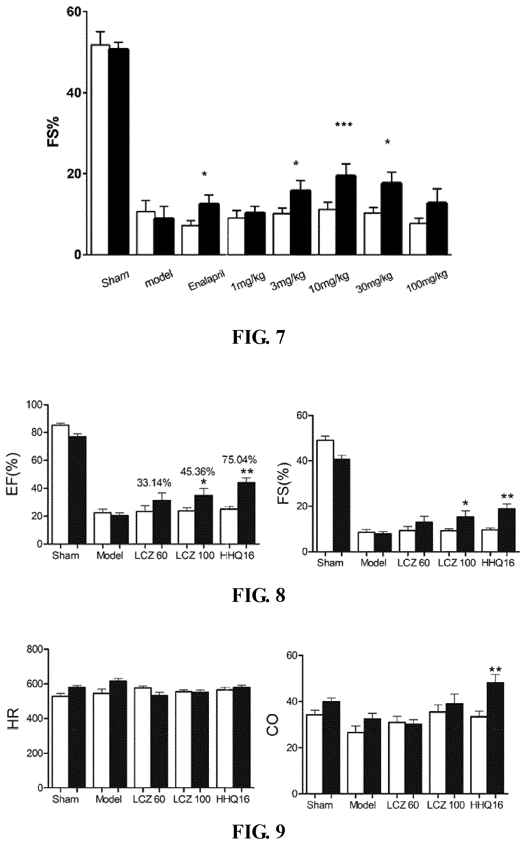

Figure 7. Short axis shortening rate (FS%) will decrease in heart failure model, while it increases after administration of drug. Taking Enalapril (p<0.05) as the positive control drug, the FS of mice in the 10 mg/kg group has significant elevated (p<0.0005). Histogram white box represents day 0, black box represents day 28.
Figure 8. EF, an important indicator for evaluating heart failure, which is is 70-90% in normal mice and below 40% in mice with heart failure. The EF of mice in both LCZ696 and compound 2 groups were all significantly increased (p<0.0005), while the effect of compound 2 was more significant. The short-axis shortening rate (FS%) decreased in heart failure and increased after drug administration. The effect of compound 2 was more significant (p< 0.0005)). The white box in the histogram represents day 0, and the black box represents day 28.
Figure 9. Heart rate (HR). HR of normal mice basically does not change, but it will change significantly in mice with heart failure. The heart rate of mice in the LCZ696 and compound 2 groups had no significant change. Cardiac output per minute (CO), CO=SVxHR. CO decreased during heart failure and all increased after drug administration. However, the increase in LCZ696 group was not statistically significant, while compound 2 group increased significantly (p<0.01)). The white box in the bar graph represents Day 0, black box represents day 28.

### Detailed Description

Unless otherwise specified, the raw materials and reagents used in the examples of the present invention can be obtained commercially.

Of the following examples, examples 1 and 2 are examples of the present invention only in so far as they form part of an overall process for the preparation of compound 2 (this overall process is a combination of the processes of examples 1, 2 and either 3 or 4). Compound 1 of example 5 is present for the purposes of comparison. All other compound-synthesis examples (examples 3, 4 and 6-43) relate directly to the preparation of compounds of the present invention and are thus invention examples. Examples 44-49 relate to the present invention, in as far as they relate to the testing of the final products of examples 3, 4 and 6-43.

### Example 1. Synthesis of compound 6.

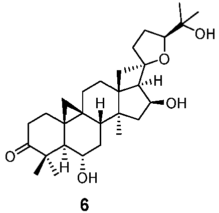

Compound 5 (Cycloastragenol, purchased from Chengdu Jintaihe) (4.9 g, 10 mmol) was dissolved in 70 mL of dry dimethyl sulfoxide. Sulfur trioxide pyridine (4.7 g, 30 mmol) and triethylamine ( 3.03 g, 30 mmol) were added in portions. The mixture was stirred at room temperature (20-25°C) for 8 h, diluted with 100 mL of water, extracted with ethyl acetate three times (80 mL each time), the organic phases were combined, washed with water, concentrated, and then evaporated to remove the solvent by a rotary evaporator at 35°C. Column chromatography (amorphous silica gel, particle size 40-63 µm, pore size 60 Å, washed with petroleum ether/ ethyl acetate = 1/2) or recrystallization in methanol (20 mL methanol) to obtain compound 6 as a white solid (3.2g, 65%) or (2.44 g, 50%), m.p. 222-225°C. ¹H NMR (400 MHz, CDCl₃) δ4.70 (td, *J=* 7.9, 6.2 Hz, 1H), 3.77 (t, *J=* 7.3 Hz, 1H), 3.54 (td, *J* = 10.0, 3.5 Hz, 1H), 2.68 - 2.52 (m, 2H), 2.48 - 2.41 (m, 1H), 2.34 (d, *J* = 7.8 Hz, 1H), 2.09 (dd, *J =* 13.2, 7.3 Hz, 2H), 2.05 - 1.96 (m, 3H), 1.94 (d, *J =* 9.7 Hz, 1H), 1.78 (dd, *J* = 12.3, 4.5 Hz, 1H), 1.70 - 1.60 (m, 3H), 1.49 - 1.36 (m, 3H), 1.36 (s, 3H), 1.31 (s, 3H), 1.28 (s, 3H), 1.24 (s, 3H), 1.22 (s, 3H), 1.16 (s, 3H), 1.05 (ddd, *J* = 14.0, 9.9, 3.4 Hz, 1H), 0.97 (s, 3H), 0.66 (d, *J* = 4.5 Hz, 1H), 0.45 (d, *J* = 4.5 Hz, 1H); ESI-ms: [M+H]⁺ 489.2.

### Example 2. Synthesis of compound 7.

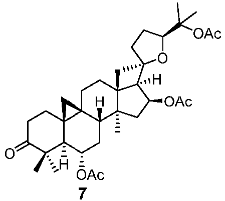

Compound 6 (4.9 g, 10 mmol), 4-pyrrolidinopyridine (1.65 g, 12 mmol) and triethylamine (9 g, 90 mmol) were dissolved in 60 mL of dry toluene, acetic anhydride (9.1 g, 90 mmol) was added and heated to reflux for 10 h. The reaction solution was diluted with 60 mL of ethyl acetate, washed with water, 3 M dilute hydrochloric acid, saturated sodium bicarbonate and saturated brine successively, dried over anhydrous sodium sulfate, filtered and concentrated (evaporated on a rotary evaporator at 35 °C). Remove the solvent to obtain the crude product, which was recrystallized in methanol to obtain a white solid compound 7 (5.5 g, 90%), m.p. 155-157°C, ¹H NMR (400 MHz, CDCl₃) δ 5.35-5.30 (m, 1 H), 4.71 (td, J = 3.6, 10.0 Hz, 1 H), 4.02 (t, J = 7.6 Hz, 1 H), 2.55 (dd, J = 8.0, 13.6 Hz, 1 H), 2.54 (d, J = 8.0 Hz, 1 H, overlapped), 2.43 (d, J = 8.0 Hz, 1 H), 2.22-2.15 (m, 3 H), 2.03 (s, 3 H), 2.02 (s, 3 H), 1.97 (s, 3 H), 2.06-1.91 (m, 2 H, overlapped), 1.76 (t, J = 8.0 Hz, 2 H), 1.43 (s, 3 H), 1.41 (s, 3 H), 1.35 (s, 3 H), 1.33 (s, 3 H), 1.52-1.25 (m, 6 H, overlapped),1.16 (s, 3 H), 1.13 (s, 3 H), 0.97 (s, 3 H), 0.76 (d, J = 4.8 Hz, 1 H), 0.50 (d, J = 4.8 Hz, 1 H); ESI-ms: [M+H]⁺ 615.2.

### Example 3. Synthesis of compound 2.

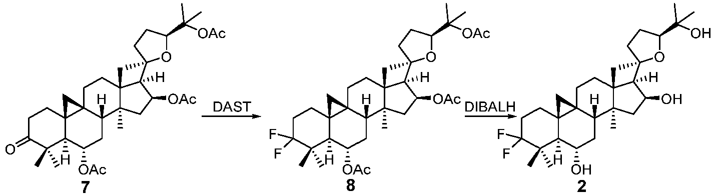

DAST: diethylaminosulfur trifluoride, DABALH: diisobutylaluminum hydride Compound 7 (3.08 g, 5 mmol) was dissolved in 40 mL of dry toluene, diethylaminosulfur trifluoride was added dropwise to the solution at 0 °C (1.61 g, 10 mmol), and then heated it up to 90°C for 6 h. Another two portions of diethylaminosulfur trifluoride (1.61 g, 10 mmol) was added dropwise, and heated at 50 °C for 6 h after each drop. The reaction solution was diluted with 60 mL of ethyl acetate, and 1 M diluted hydrochloric acid was added, and stirred for 1 h. The aqueous phase was separated, and the organic phases were washed successively with 1 M dilute hydrochloric acid, saturated sodium bicarbonate and saturated brine, dried over anhydrous sodium sulfate, filtered, and recrystallized in methanol (20 mL of methanol) to obtain compound 8 as a white solid (2.71g, 85%), m.p. 139-142°C, ¹H NMR (400 MHz, CDCl₃) δ 5.32 (m, 1 H), 4.71 (ddd, J = 5.6, 4.0 Hz, 1 H), 4.01 (t, J = 7.4 Hz, 1 H), 2.42 (d, J = 7.6 Hz, 1 H), 2.19-2.09 (m, 3 H), 2.01 (s, 6 H), 1.97 (s, 3 H), 1.98-1.82 (m, 4 H), 1.73 (t, J = 7.6 Hz, 3 H), 1.63-1.48 (m, 2 H), 1.43 (s, 3 H), 1.41 (s, 3 H), 1.33 (s, 6 H), 1.25 (s, 3 H), 1.36-1.21 (m, 5 H, overlapped), 1.07 (s, 3 H), 1.04 (s, 3 H), 0.97 (s, 3 H), 0.63 (d, J = 4.8 Hz, 1 H), 0.43 (d, J = 4.4, 4.4 Hz, 1 H); ESI-ms: [M+H]⁺ 637.2.

This difluoro-substituted product was dissolved in 60 mL of anhydrous dichloromethane, dropped to 0 °C, and diisobutylaluminum hydride (15 mL, 15 mmol, 1 M) was added dropwise. The reaction was stirred for 3 h after naturally returned to room temperature (20-25 °C). 1 M dilute hydrochloric acid was added dropwise to quench the reaction. The reaction solution was washed with 1 M dilute hydrochloric acid and saturated brine successively, dried over anhydrous sodium sulfate, filtered, and concentrated (35 °C rotary evaporator to remove the solvent). Recrystallized with methanol/water = 1/3 to give the product compound 2 (1.98 g, 92%) as a white solid m.p. 189-192°C, [α]_{D}²⁵ = 118 (c 1.0, CHCl₃); ¹H NMR (400 MHz, CDCl₃) δ 4.69 (q, J = 7.2 Hz, 1 H), 3.77 (t, J = 7.2 Hz, 1 H), 3.51 (td, J = 10.0, 3.6 Hz, 1 H), 2.57 (q, J = 10.6 Hz, 1 H), 2.33 (d, J = 7.6 Hz, 1 H), 2.06-1.94 (m, 6 H), 1.83-1.56 (m, 6 H), 1.46-1.36 (m, 4 H), 1.32 (s, 3 H), 1.30 (s, 3 H), 1.28 (s, 3 H), 1.25 (s, 3 H), 1.17 (s, 3 H), 1.13 (s, 3 H), 0.96 (s, 3 H), 0.58 (d, J = 4.4 Hz, 1 H), 0.44 (d, J = 4.4 Hz, 1 H); ESI-ms: [M+H]⁺ 511.2.

### Example 4. Synthesis of compound 2.

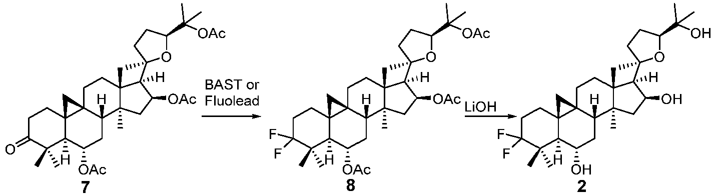

BAST or Fluorolead: bis(2-methoxyethyl)aminosulfur trifluoride or 4-tert-butyl-2,6-dimethylphenylsulfur trifluoride; LiOH: lithium hydroxide Compound 7 (3.08 g, 5 mmol) was dissolved in 40 mL of dry toluene, and BAST (2.21 g, 10 mmol) or fluorolead (2.5 g, 10 mmol) with a catalytic amount (0.15 mL) of pyridine hydrofluoride were added. The mixture was heated to 40 °C for 12 h and diluted with 60 mL of ethyl acetate. 1 M dilute hydrochloric acid was added and stirred for 1 h. The aqueous phase was separated, and the organic phase was washed with 1 M dilute hydrochloric acid, saturated sodium bicarbonate and saturated brine successively. Then dried over anhydrous sodium sulfate, filtered, and recrystallized in methanol (20 mL methanol) to obtain compound 8 as a white solid.

(2.75g, 88%), m.p. 139-142°C, ¹H NMR (400 MHz, CDCl₃) δ 5.32 (m, 1 H), 4.71 (ddd, J = 5.6, 4.0 Hz, 1 H), 4.01 (t, J = 7.4 Hz, 1 H), 2.42 (d, J = 7.6 Hz, 1 H), 2.19-2.09 (m, 3 H), 2.01 (s, 6 H), 1.97 (s, 3 H), 1.98-1.82 (m, 4 H), 1.73 (t, J = 7.6 Hz, 3 H), 1.63-1.48 (m, 2 H), 1.43 (s, 3 H), 1.41 (s, 3 H), 1.33 (s, 6 H), 1.25 (s, 3 H), 1.36-1.21 (m, 5 H, overlapped), 1.07 (s, 3 H), 1.04 (s, 3 H), 0.97 (s, 3 H), 0.63 (d, J = 4.8 Hz, 1 H), 0.43 (d, J = 4.4, 4.4 Hz, 1 H); ESI-ms: [M+H]⁺ 637.2.

This difluoro-substituted product was dissolved in 60 mL of anhydrous dichloromethane, and diisobutylaluminum hydride (15 mL, 15 mmol, 1 M) was added dropwise at 0°C. The reaction was stirred for 3 h after naturally returned to room temperature (20-25 °C). 1 M dilute hydrochloric acid was added dropwise to quench the reaction. The reaction solution was washed with 1 M dilute hydrochloric acid and saturated brine successively, dried over anhydrous sodium sulfate, filtered, and concentrated (35 °C rotary evaporator to remove the solvent). Recrystallized with methanol/water = 1/3 to give the product compound 2 (2.0 g, 92%) as a white solid.

(2.0 g, 92%), m.p. 189-192°C, [α]_{D}²⁵ = 118 (c 1.0, CHCl₃); ¹H NMR (400 MHz, CDCl₃) δ 4.69 (q, J = 7.2 Hz, 1 H), 3.77 (t, J = 7.2 Hz, 1 H), 3.51 (td, J = 10.0, 3.6 Hz, 1 H), 2.57 (q, J = 10.6 Hz, 1 H), 2.33 (d, J = 7.6 Hz, 1 H), 2.06-1.94 (m, 6 H), 1.83-1.56 (m, 6 H), 1.46-1.36 (m, 4 H), 1.32 (s, 3 H), 1.30 (s, 3 H), 1.28 (s, 3 H), 1.25 (s, 3 H), 1.17 (s, 3 H), 1.13 (s, 3 H), 0.96 (s, 3 H), 0.58 (d, J = 4.4 Hz, 1 H), 0.44 (d, J = 4.4 Hz, 1 H); ESI-ms: [M+H]⁺ 511.2.

### Example 5. Synthesis of compound 1.

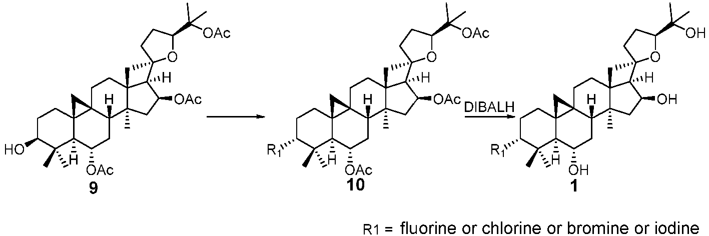

1. Compound 9 (3.1 g, 5 mmol) was dissolved in 50 mL of dry dichloromethane, and 4-tert-butyl-2,6-dimethylphenylsulfur trifluoride (2.5 g, 10 mmol) was added at 0 °C. The reaction was stirred at this temperature for 3 h, quenched by 40 mL of 0.1 M dilute hydrochloric acid, and the aqueous phase was separated. The organic phase was washed with 1 M dilute hydrochloric acid, saturated sodium bicarbonate and saturated brine successively, dried over anhydrous sodium sulfate, filtered, and subjected to column chromatography to obtain fluorinated product as a white solid. ESI-ms: [M+H]⁺ 619.2.
   This fluorinated product was dissolved in 60 mL of anhydrous dichloromethane, dropped to 0 °C, and diisobutylaluminum hydride (15 mL, 15 mmol, 1 M) was added dropwise. 1 M was added dropwise after the reaction was naturally returned to room temperature for 3 h. The reaction was quenched with dilute hydrochloric acid, and the reaction solution was washed with 1 M dilute hydrochloric acid and saturated brine successively, dried over anhydrous sodium sulfate, filtered, concentrated (the solvent was evaporated by a rotary evaporator at 35°C) and column chromatography (amorphous silica gel, particle size 40-63 µm, pore size 60 Å, washed with petroleum ether/ethyl acetate = 2 : 3) to obtain (2aR,3R,4S,5aS,5bS,7S,7aR,9R,11aR,12aS)-9-halogen-3-((2R,5S)-5-(2-hydroxypropan-2-yl)-2-methyltetrahydrofuran-2-yl)-2a,5a,8,8-tetramethyltetradecahydro-1H,12H-cyclopenta [a]cyclopropa[e]phenanthrene-4,7-diol as a white solid. [α]_{D}²⁵ = 188 (c 1.0, CHCl₃); ESI-ms: [M+H]⁺ 493.2.
2. Compound 9 (3.1 g, 5 mmol) was dissolved in 50 mL of dry dichloromethane, carbon tetrabromide (1.66 g, 5 mmol) and triphenylphosphine (1.31 g, 5 mmol) was added at 0 °C and stirred for 6 h. The reaction was quenched by adding 40 mL of 0.1 M dilute hydrochloric acid, and the organic phase was washed with 1 M dilute hydrochloric acid, saturated sodium bicarbonate and saturated brine, dried over anhydrous sodium sulfate, filtered, and subjected to column chromatography (amorphous silica gel, particle size 40-63 µm, pore size 60 Å,) to obtain 2.4g of brominated product as a white solid. ESI-ms: [M+H]⁺ 679.3.
   This brominated product was dissolved in 60 mL of anhydrous dichloromethane, and diisobutylaluminum hydride (15 mL, 15 mmol, 1 M) was added dropwise at 0°C. The reaction was stirred for 3 h after naturally returned to room temperature. 1 M dilute hydrochloric acid was added dropwise to quench the reaction. The reaction solution was washed with 1 M dilute hydrochloric acid and saturated brine successively, dried over anhydrous sodium sulfate, filtered, and concentrated (35 °C rotary evaporator to remove the solvent). Column chromatography (amorphous silica gel, particle size 40-63 µm, pore size 60 Å,washed with petroleum ether/ Ethyl acetate = 2/3) to obtain (2aR,3R,4S,5aS,5bS,7S,7aR,9R,11aR,12aS)-9-bromo-3-((2R,5S)-5-(2-hydroxypropan-2-yl )-2-methyltetrahydrofuran-2-yl)-2a,5a,8,8-tetramethyltetradecahydro-1H,12H-cyclopenta[a ]cyclopropa[e]phenanthrene-4,7-diol (1.77 g, 91%) as a white solid. [α]_{D}²⁵ = 101 (c 1.0, CHCl₃); ESI-ms: [M+H]⁺ 553.3.
3. Compound 9 (3.1 g, 5 mmol) was dissolved in 50 mL of dry dichloromethane, and N-chlorosuccinimide (0.62 g, 5 mmol) and triphenylphosphine (1.31 g, 5 mmol) were added and stirred at 0°C for 6 h. The reaction was quenched by adding 0.1 M diluted hydrochloric acid 40 mL, and the organic phase was sequentially saturated with 1 M diluted hydrochloric acid, washed with sodium bicarbonate and saturated brine, dried over anhydrous sodium sulfate, filtered, and subjected to column chromatography (amorphous silica gel, particle size 40-63 µm, pore size 60 Å) to obtain 2.1 g of chlorinated product as a white solid. ESI-ms: [M+H]⁺ 635.3.
   The chlorinated product was dissolved in 60 mL of anhydrous dichloromethane, and diisobutylaluminum hydride (15 mL, 15 mmol, 1 M) was added dropwise at 0°C. The reaction was stirred for 3 h after naturally returned to room temperature. 1 M dilute hydrochloric acid was added dropwise to quench the reaction. The reaction solution was washed with 1 M dilute hydrochloric acid and saturated brine successively, dried over anhydrous sodium sulfate, filtered, and concentrated (35 °C rotary evaporator to remove the solvent). Column chromatography (amorphous silica gel, particle size 40-63 µm, pore size 60 Å,washed with petroleum ether/ Ethyl acetate = 2/3) to obtain (2aR,3R,4S,5aS,5bS,7S,7aR,9R,11aR,12aS)-9-chloro-3-((2R,5S)-5-(2-hydroxypropan-2-yl )-2-methyltetrahydrofuran-2-yl)-2a,5a,8,8-tetramethyltetradecahydro-1H,12H-cyclopenta[a ]cyclopropa[e]phenanthrene-4,7-diol (1.55 g, 92%) as a white solid. [α]_{D}²⁵ = 133 (c 1.0, CHCl₃); ESI-ms: [M+H]⁺ 509.3.
4. Compound 9 (3.1 g, 5 mmol) was dissolved in 50 mL of dry dichloromethane, and iodine (1.27 g, 5 mmol) and triphenylphosphine (1.31 g, 5 mmol) were added and stirred at 0°C for 6 h. The reaction was quenched by adding 0.1 M diluted hydrochloric acid 40 mL, and the organic phase was sequentially saturated with 1 M diluted hydrochloric acid, washed with sodium bicarbonate and saturated brine, dried over anhydrous sodium sulfate, filtered, and subjected to column chromatography (amorphous silica gel, particle size 40-63 µm, pore size 60 Å) to obtain 2.7 g of iodinated product as a white solid. ESI-ms: [M+H]⁺ 727.3.
   The iodinated product was dissolved in 60 mL of anhydrous dichloromethane, and diisobutylaluminum hydride (15 mL, 15 mmol, 1 M) was added dropwise at 0°C. The reaction was stirred for 3 h after naturally returned to room temperature. 1 M dilute hydrochloric acid was added dropwise to quench the reaction. The reaction solution was washed with 1 M dilute hydrochloric acid and saturated brine successively, dried over anhydrous sodium sulfate, filtered, and concentrated (35 °C rotary evaporator to remove the solvent). Column chromatography (amorphous silica gel, particle size 40-63 µm, pore size 60 Å,washed with petroleum ether/ Ethyl acetate = 2/3) to obtain (2aR,3R,4S,5aS,5bS,7S,7aR,9R,11aR,12aS)-9-iodo-3-((2R,5S)-5-(2-hydroxypropan-2-yl)-2-methyltetrahydrofuran-2-yl)-2a,5a,8,8-tetramethyltetradecahydro-1H,12H-cyclopenta[a] cyclopropa[e]phenanthrene-4,7-diol (2.0 g, 90%) as a white solid. [α]_{D}²⁵ = 87 (c 1.0, CHCl₃); ESI-ms: [M+H]⁺ 601.3.

### Example 6. Synthesis of 3-(((2aR,3R,4S,5aS,5bS,7S,7aR,11aR,12aS)-9,9-difluoro-4-hydroxy-3-((2S,5S)-5-(2-hydr oxypropan-2-yl)-2-methyltetrahydrofuran-2-yl)-2a,5a,8,8-tetramethyltetradecahydro-1H,12 H-cyclopenta[a]cyclopropa[e]phenanthren-7-yl)oxy)-3-oxopropan-1-aminium chloride (compound 2-1).

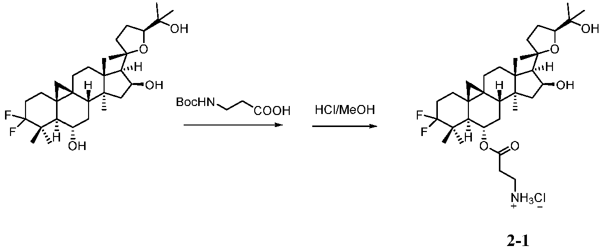

Compound 2 (510mg, 1mmol) and 3-((tert-butoxycarbonyl)amino)propionic acid (200mg, 1.1mmol) were dissolved in 5mL of dry dichloromethane. EDCI (230mg, 1.2mmol) and DMAP ( 24 mg, 0.2 mmol) were added to the mixture at 0°C and stirred at room temperature overnight. The reaction was quenched by adding 0.1 M hydrochloric acid solution, extracted twice with ethyl acetate, washed once with water, concentrated and purified by column chromatography (petroleum ether/ethyl acetate=1/1 ) to obtain a white solid product. The crude product was dissolved in 5 mL of methanol, and 4 M hydrochloric acid dioxane solution was added and stirred at room temperature for 8 h. The methanol was evaporated under reduced pressure after TLC showed the disappearance of the raw material. 10 mL of ether or methyl tert-butyl ether was added and stirred for 2h, and filtered to obtain a white solid product (407mg, 66% yield).

¹H NMR (400 MHz, CDCl₃) δ 4.66 (q, J = 7.2 Hz, 1 H), 3.75 (t, J = 7.2 Hz, 1 H), 3.49 (td, J = 10.0, 3.6 Hz, 1 H), 2.57 (q, J = 10.6 Hz, 1 H), 2.41 (t, J = 7.2 Hz, 2 H), 2.36 (d, J = 7.6 Hz, 1 H), 2.03-1.91 (m, 6 H), 1.86-1.52 (m, 9 H), 1.46-1.36 (m, 4 H), 1.32 (s, 3 H), 1.30 (s, 3 H), 1.28 (s, 3 H), 1.25 (s, 3 H), 1.17 (s, 3 H), 1.15 (s, 3 H), 0.94 (s, 3 H), 0.57 (d, J = 4.5 Hz, 1 H), 0.45 (d, J = 4.4 Hz, 1 H); ESI-ms: [M-H]⁻ 580.2.

### Example 7. Synthesis of 4-((((2aR,3R,4S,5aS,5bS,7S,7aR,11aR,12aS)-9,9-difluoro-4-hydroxy-3-((2S,5S)-5-(2-hydr oxypropan-2-yl)-2-methyltetrahydrofuran-2-yl)-2a,5a,8,8-tetramethyltetradecahydro-1H,12 H-cyclopenta[a]cyclopropa[e]phenanthren-7-yl)oxy)carbonyl)piperidin-1-ium chloride (compound 2-2).

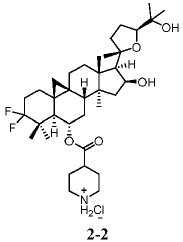

The synthesis of compound 2-2 is similar to that of compound 2-1, except that 1-(tert-butoxycarbonyl)piperidine-4-carboxylic acid was used instead of 3-((tert-butoxycarbonyl)amino)propionic acid.

¹H NMR (400 MHz, CDCl₃) δ 4.63 (q, J = 7.2 Hz, 1 H), 3.72 (t, J = 7.2 Hz, 1 H), 3.43 (td, J = 10.0, 3.3 Hz, 1 H), 2.57 (q, J = 10.3 Hz, 1 H), 2.41 (t, J = 7.1 Hz, 2 H), 2.36 (d, J = 7.6 Hz, 1 H), 2.09-1.93 (m, 7 H), 1.88-1.53 (m, 10 H), 1.51-1.31 (m, 8 H), 1.33 (s, 3 H), 1.29 (s, 6 H), 1.25 (s, 3 H), 1.18 (s, 3 H), 1.16 (s, 3 H), 0.96 (s, 3 H), 0.55 (d, J = 4.4 Hz, 1 H), 0.46 (d, J = 4.4 Hz, 1 H); ESI-ms: [M-H]⁻ 620.2.

### Example 8. Synthesis of (R)-2-(2-(((2aR,3R,4S,5aS,5bS,7S,7aR,11aR,12aS)-9,9-difluoro-4-hydroxy-3-((2S,5S)-5-( 2-hydroxypropan-2-yl)-2-methyltetrahydrofuran-2-yl)-2a,5a,8,8-tetramethyltetradecahydro -1H,12H-cyclopenta[a]cyclopropa[e]phenanthren-7-yl)oxy)-2-oxoethyl)pyrrolidin-1-ium chloride (compound 2-3).

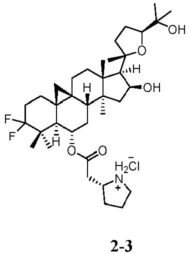

The synthesis of compound 2-3 was similar to that of compound 2-1, except that (R)-2-(1-(tert-butoxycarbonyl)pyrrolidin-2-yl)acetic acid was used instead of 3-((tert-butoxycarbonyl)acetic acid. carbonyl) amino) propionic acid.

¹H NMR (400 MHz, CDCl₃) δ 4.64 (q, J = 7.4 Hz, 1 H), 3.75 (t, J = 7.1 Hz, 1 H), 3.51 (td, J = 10.1, 3.6 Hz, 1 H), 2.99-2.61 (m, 2H),2.56 (q, J = 10.4 Hz, 1 H), 2.34 (d, J = 7.4 Hz, 1 H), 2.29-1.92 (m, 10 H), 1.88-1.59 (m, 9 H), 1.45-1.33 (m, 4 H), 1.32 (s, 6 H),1.28 (s, 3 H), 1.23 (s, 3 H), 1.16 (s, 3 H), 1.11 (s, 3 H), 0.97 (s, 3 H), 0.57 (d, J = 4.5 Hz, 1 H), 0.45 (d, J = 4.5 Hz, 1 H); ESI-ms: [M-H]⁻ 656.3.

### Example 9. Synthesis of (R)-2-(2-(((2aR,3R,4S,5aS,5bS,7S,7aR,11aR,12aS)-9,9-difluoro-4-hydroxy-3-((2S,5S)-5-( 2-hydroxypropan-2-yl)-2-methyltetrahydrofuran-2-yl)-2a,5a,8,8-tetramethyltetradecahydro -1H,12H-cyclopenta[a]cyclopropa[e]phenanthren-7-yl)oxy)-2-oxoethyl)pyrrolidin-1-ium chloride (compound 2-4).

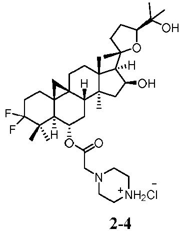

The synthesis of compound 2-4 is similar to that of compound 2-1, except that 2-(4-(tert-butoxycarbonyl)piperazin-1-yl)acetic acid was used instead of 3-((tert-butoxycarbonyl)amino) propionic acid.

¹H NMR (400 MHz, CDCl₃) δ 4.71 (q, J = 7.2 Hz, 1 H), 3.78 (t, J = 7.2 Hz, 1 H), 3.51 (td, J = 10.1, 3.6 Hz, 1 H), 3.28-3.13 (m, 6 H),2.57 (q, J = 10.5 Hz, 1 H), 2.37-1.95 (m, 11 H), 1.87-1.55 (m, 6 H), 1.45-1.36 (m, 4 H), 1.32 (s, 3 H), 1.30 (s, 3 H), 1.28 (s, 3 H), 1.25 (s, 3 H), 1.17 (s, 3 H), 1.13 (s, 3 H), 0.98 (s, 3 H), 0.57 (d, J = 4.4 Hz, 1 H), 0.44 (d, J = 4.5 Hz, 1 H); ESI-ms: [M+H]⁺ 673.2.

### Example 10. Synthesis of 1-(2-(((2aR,3R,4S,5aS,5bS,7S,7aR,11aR,12aS)-9,9-difluoro-4-hydroxy-3-((2S,5S)-5-(2-hy droxypropan-2-yl)-2-methyltetrahydrofuran-2-yl)-2a,5a,8,8-tetramethyltetradecahydro-1H, 12H-cyclopenta[a]cyclopropa[e]phenanthren-7-yl)oxy)-2-oxoethyl)guanidinium bromide (compound 2-5).

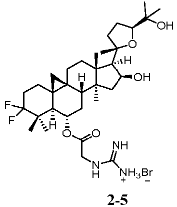

The synthesis of compound 2-5 is similar to that of compound 2-1, except that (N-(tert-butoxycarbonyl)carbamoyl)glycine and hydrobromide acid were used instead of 3-((tert-butoxycarbonyl)amino)propionic acid and hydrochloric acid.

¹H NMR (400 MHz, CDCl₃) δ4.69 (q, J = 7.3 Hz, 1 H), 4.44 (s, 2 H), 3.75 (t, J = 7.2 Hz, 1 H), 3.50 (td, J = 10.0, 3.6 Hz, 1 H), 2.57 (q, J = 10.6 Hz, 1 H), 2.33 (d, J = 7.6 Hz, 1 H), 2.07-1.95 (m, 6 H), 1.85-1.53 (m, 6 H), 1.44-1.31 (m, 4 H), 1.32 (s, 3 H), 1.30 (s, 3 H), 1.28 (s, 3 H), 1.25 (s, 3 H), 1.17 (s, 3 H), 1.13 (s, 3 H), 0.96 (s, 3 H), 0.58 (d, J = 4.5 Hz, 1 H), 0.45 (d, J = 4.5 Hz, 1 H); ESI-ms: [M+H]⁺ 690.2.

### Example 11. Synthesis of 2-(((2aR,3R,4S,5aS,5bS,7S,7aR,11aR,12aS)-9,9-difluoro-4-hydroxy-3-((2S,5S)-5-(2-hydr oxypropan-2-yl)-2-methyltetrahydrofuran-2-yl)-2a,5a,8,8-tetramethyltetradecahydro-1H,12 H-cyclopenta[a]cyclopropa[e]phenanthren-7-yl)oxy)-N-methyl-2-oxoethan-1-aminium chloride (compound 2-6).

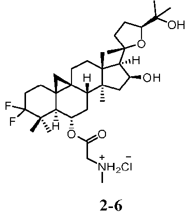

The synthesis of compound 2-6 was similar to that of compound 2-1, except that N-(tert-butoxycarbonyl)sarcosine was used instead of 3-((tert-butoxycarbonyl)amino)propionic acid.

¹H NMR (400 MHz, CDCl₃) δ 4.70 (q, J = 7.2 Hz, 1 H), 4.31 (s, 2H), 3.75 (t, J = 7.2 Hz, 1 H), 3.51 (td, J = 10.1, 3.6 Hz, 1 H), 2.57 (q, J = 10.6 Hz, 1 H), 2.33 (d, J = 7.5 Hz, 1 H), 2.31 (s, 3 H), 2.12-1.99 (m, 6 H), 1.86-1.57 (m, 6 H), 1.50-1.38 (m, 4 H), 1.32 (s, 6 H), 1.28 (s, 3 H), 1.25 (s, 3 H), 1.17 (s, 3 H), 1.13 (s, 3 H), 0.96 (s, 3 H), 0.57 (d, J = 4.5 Hz, 1 H), 0.44 (d, J = 4.5 Hz, 1 H); ESI-ms: [M+H]⁺ 619.2.

### Example 12. Synthesis of 2-(2-(((2aR,3R,4S,5aS,5bS,7S,7aR,11aR,12aS)-9,9-difluoro-4-hydroxy-3-((2S,5S)-5-(2-hy droxypropan-2-yl)-2-methyltetrahydrofuran-2-yl)-2a,5a,8,8-tetramethyltetradecahydro-1H, 12H-cyclopenta[a]cyclopropa[e]phenanthren-7-yl)oxy)-2-oxoethoxy)ethan-1-aminium chloride (compound 2-7).

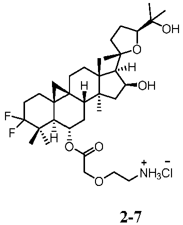

The synthesis of compound 2-7 is similar to that of compound 2-1, except that 2-(2-(((tert-butoxycarbonyl)amino)ethoxy)acetic acid was used instead of 3-((tert-butoxycarbonyl)amino ) propionic acid.

¹H NMR (400 MHz, MeOD) δ 4.73 (q, J = 7.5 Hz, 1 H), 4.36-4.17 (m, 4 H), 3.79 (t, J = 7.1 Hz, 1 H), 3.55 (td, J = 10.3, 3.6 Hz, 1 H), 3.46-3.31 (m, 2 H), 2.58 (q, J = 10.6 Hz, 1 H), 2.34 (d, J = 7.6 Hz, 1 H), 2.05-1.95 (m, 6 H), 1.88-1.62 (m, 6 H), 1.47-1.38 (m, 4 H), 1.34 (s, 3 H), 1.31 (s, 3 H), 1.29 (s, 3 H), 1.25 (s, 3 H), 1.17 (s, 3 H), 1.13 (s, 3 H), 0.98 (s, 3 H), 0.61 (d, J = 4.4 Hz, 1 H), 0.49 (d, J = 4.4 Hz, 1 H); ESI-ms: [M+H]⁺ 649.2.

### Example 13. Synthesis of 2-(2-(2-(2-(((2aR,3R,4S,5aS,5bS,7S,7aR,11aR,12aS)-9,9-difluoro-4-hydroxy-3-((2S,5S)-5 -(2-hydroxypropan-2-yl)-2-methyltetrahydrofuran-2-yl)-2a,5a,8,8-tetramethyltetradecahyd ro-1H,12H-cyclopenta[a]cyclopropale]phenanthren-7-yl)oxy)-2-oxoethoxy)ethoxy)ethoxy) ethan-1-aminium trifluoroacetate (compound 2-8).

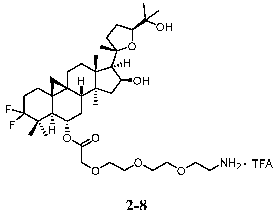

The synthesis of compound 2-8 is similar to that of compound 2-1, except that 2,2-dimethyl-4-oxo-3,8,11,14-tetraoxa-5-azahexadecane-16-oleic acid and trifluoroacetic acid were used instead of 3-((tert-butoxycarbonyl)amino)propionic acid and hydrochloric acid.

¹H NMR (400 MHz, CDCl₃) δ 4.73 (q, J = 7.2 Hz, 1 H), 4.33 (s, 2 H), 3.77 (t, J = 7.3 Hz, 1 H), 3.66-3.26 (m, 11 H), 3.16-3.02 (m, 2 H), 2.57 (q, J = 10.5 Hz, 1 H), 2.36 (d, J = 7.5 Hz, 1 H), 2.06-1.95 (m, 6 H), 1.85-1.59 (m, 6 H), 1.46-1.35 (m, 4 H), 1.32 (s, 3 H), 1.30 (s, 3 H), 1.28 (s, 3 H), 1.25 (s, 3 H), 1.18 (s, 3 H), 1.16 (s, 3 H), 0.99 (s, 3 H), 0.61 (d, J = 4.4 Hz, 1 H), 0.46 (d, J = 4.4 Hz, 1 H); ESI-ms: [M+H]⁺ 700.2.

### Example 14. Synthesis of 2-((2-((2-(((2aR,3R,4S,5aS,5bS,7S,7aR,11aR,12aS)-9,9-difluoro-4-hydroxy-3-((2S,5S)-5-( 2-hydroxypropan-2-yl)-2-methyltetrahydrofuran-2-yl)-2a,5a,8,8-tetramethyltetradecahydro -1H,12H-cyclopenta[a]cyclopropa[e]phenanthren-7-yl)oxy)-2-oxoethyl)amino)ethyl)amino )ethan-1-aminium nitrate (compound 2-9).

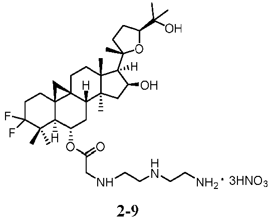

The synthesis of compound 2-9 is similar to that of compound 2-1, except that 8,11-bis(tert-butoxycarbonyl)-2,2-dimethyl-4-oxo-3-oxa-5,8,11-triazacane-13-oleic acid and nitric acid were used instead of 3-((tert-butoxycarbonyl)amino)propionic acid and hydrochloric acid.

¹H NMR (400 MHz, CDCl₃) δ 4.67 (q, J = 7.2 Hz, 1 H), 3.75 (t, J = 7.2 Hz, 1 H), 3.54 (td, J = 10.0, 3.6 Hz, 1 H), 3.43 (s, 2 H), 2.66-2.26 (m, 10 H), 2.09 -1.96 (m, 6 H), 1.87-1.57 (m, 6 H), 1.49-1.37 (m, 4 H), 1.32 (s, 6 H), 1.28 (s, 3 H), 1.25 (s, 3 H), 1.19 (s, 3 H), 1.17 (s, 3 H), 0.97 (s, 3 H), 0.62 (d, J = 4.3 Hz, 1 H), 0.46 (d, J = 4.4 Hz, 1 H); ESI-ms: [M+H]⁺ 654.2.

### Example 15. Synthesis of 17-(((2aR,3R,4S,5aS,5bS,7S,7aR,11aR,12aS)-9,9-difluoro-4-hydroxy-3-((2S,5S)-5-(2-hyd roxypropan-2-yl)-2-methyltetrahydrofuran-2-yl)-2a,5a,8,8-tetramethyltetradecahydro-1H,1 2H-cyclopenta[a]cyclopropa[e]phenanthren-7-yl)oxy)-17-oxo-3,6,9,12,15-pentaoxaheptad ecan-1-aminium bromide (compound 2-10).

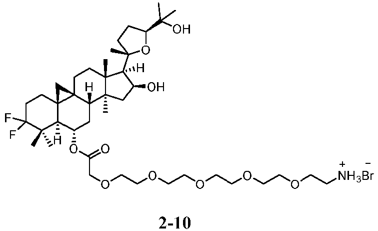

The synthesis of compound 2-10 is similar to that of compound 2-1, except that 17-amino-3,6,9,12,15-pentaoxaheptadecanoic acid and hydrobromide acid were used instead of 3-((tert-butoxycarbonyl)amino)propionic acid and hydrochloric acid.

¹H NMR (400 MHz, CDCl₃) δ 4.72 (q, J = 7.1 Hz, 1 H), 4.41 (s, 2 H), 3.76 (t, J = 7.2 Hz, 1 H), 3.79-3.20 (m, 19 H), 3.16-3.01 (m, 2 H), 2.57 (q, J = 10.4 Hz, 1 H), 2.33 (d, J = 7.4 Hz, 1 H), 2.06-1.95 (m, 6 H), 1.81-1.55 (m, 6 H), 1.45-1.36 (m, 4 H), 1.33 (s, 3 H), 1.30 (s, 3 H), 1.28 (s, 3 H), 1.26 (s, 3 H), 1.17 (s, 3 H), 1.14 (s, 3 H), 0.98 (s, 3 H), 0.59 (d, J = 4.4 Hz, 1 H), 0.47 (d, J = 4.3 Hz, 1 H); ESI-ms: [M+H]⁺ 788.2.

### Example 16. Synthesis of (2aR,3R,4S,5aS,5bS,7S,7aR,11aR,12aS)-9,9-difluoro-4-hydroxy-3-((2S,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-yl-2-(2-hydroxyethoxy)acetate (compound 2-11).

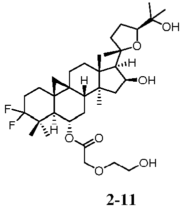

Compound 2-11 was synthesized in a similar manner to compound 2-1, except that 2-(2-(tert-butoxy)ethoxy)acetic acid was used instead of 3-((tert-butoxycarbonyl)amino)propionic acid.

¹H NMR (400 MHz, CDCl₃) δ 4.73 (q, J = 7.2 Hz, 1 H), 4.39 (s, 2 H), 3.78 (t, J = 7.2 Hz, 1 H), 3.76-3.36 (m, 5H), 2.58 (q, J = 10.5 Hz, 1 H), 2.36 (d, J = 7.5 Hz, 1 H), 2.10-1.99 (m, 6 H), 1.85-1.57 (m, 6 H), 1.49-1.37 (m, 4 H), 1.32 (s, 3 H), 1.31 (s, 3 H), 1.26 (s, 3 H), 1.23 (s, 3 H), 1.18 (s, 3 H), 1.13 (s, 3 H), 0.99 (s, 3 H), 0.59 (d, J = 4.4 Hz, 1 H), 0.47 (d, J = 4.4 Hz, 1 H); ESI-ms: [M+H]⁺ 613.2.

### Example 17. Synthesis of (2aR,3R,4S,5aS,5bS,7S,7aR,11aR,12aS)-9,9-difluoro-4-hydroxy-3-((2S,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-yl-2-(2-(2-hydroxyethoxy)ethoxy)acetate (compound 2-12).

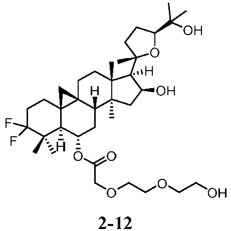

The synthesis of compound 2-12 is similar to that of compound 2-1, except that 2-(2-(2-(2-(tert-butoxy)ethoxy)ethoxy)acetic acid was used instead of 3-((tert-butoxycarbonyl)amino)propionic acid.

¹H NMR (400 MHz, CDCl₃) δ 4.70 (q, J = 7.3 Hz, 1 H), 4.36 (s, 2 H), 3.75 (t, J = 7.2 Hz, 1 H), 3.68-3.22 (m, 9H), 2.57 (q, J = 10.6 Hz, 1 H), 2.33 (d, J = 7.5 Hz, 1 H), 2.12-2.00 (m, 6 H), 1.86-1.59 (m, 6 H), 1.50-1.39 (m, 4 H), 1.32 (s, 3 H), 1.31 (s, 3 H), 1.26 (s, 3 H), 1.23 (s, 3 H), 1.19 (s, 3 H), 1.13 (s, 3 H), 0.96 (s, 3 H), 0.59 (d, J = 4.4 Hz, 1 H), 0.45 (d, J = 4.4 Hz, 1 H); ESI-ms: [M+H]⁺ 657.1.

### Example 18. Synthesis of (2aR,3R,4S,5aS,5bS,7S,7aR,11aR,12aS)-9,9-difluoro-4-hydroxy-3-((2S,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-yl-2-(2-(2-(2-hydroxyethoxy)ethoxy)ethoxy)acet ate (compound 2-13).

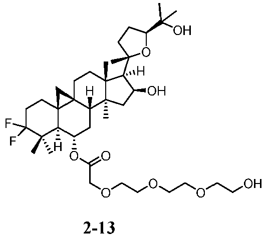

The synthesis of compound 2-13 is similar to that of compound 2-1, except that 13,13-dimethyl-3,6,9,12-tetraoxadecanoic acid was used instead of 3-((tert-butoxycarbonyl)amino)propionic acid.

¹H NMR (400 MHz, CDCl₃) δ 4.68 (q, J = 7.4 Hz, 1 H), 4.35 (s, 2 H), 3.78-3.19 (m, 14H), 2.59 (q, J = 10.5 Hz, 1 H), 2.32 (d, J = 7.4 Hz, 1 H), 2.10-2.00 (m, 6 H), 1.89-1.63 (m, 6 H), 1.53-1.41 (m, 4 H), 1.35 (s, 3 H), 1.31 (s, 3 H), 1.28 (s, 3 H), 1.23 (s, 3 H), 1.19 (s, 3 H), 1.15 (s, 3 H), 1.01 (s, 3 H), 0.59 (d, J = 4.4 Hz, 1 H), 0.44 (d, J = 4.4 Hz, 1 H); ESI-ms: [M+H]⁺ 700.1.

### Example 19. Synthesis of (2aR,3R,4S,5aS,5bS,7S,7aR,11aR,12aS)-9,9-difluoro-4-hydroxy-3-((2S,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-yl-17-hydroxy-3,6,9,12,15-pentaoxaheptadecano ate (compound 2-14).

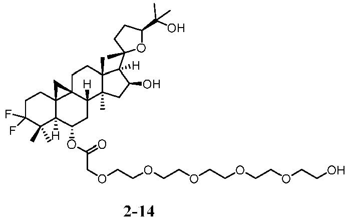

The synthesis of compound 2-14 is similar to that of compound 2-1, except that 19,19-dimethyl-3,6,9,12,15,18-hexaoxyeicosanoic acid was used instead of 3-((tert-butoxycarbonyl)amino)propionic acid.

¹H NMR (400 MHz, CDCl₃) δ 4.66 (q, J = 7.4 Hz, 1 H), 4.31 (s, 2 H), 3.77-3.13 (m, 22H), 2.55 (q, J = 10.4 Hz, 1 H), 2.37 (d, J = 7.5 Hz, 1 H), 2.14-2.02 (m, 6 H), 1.93-1.66 (m, 6 H), 1.56-1.44 (m, 4 H), 1.35 (s, 3 H), 1.33 (s, 3 H), 1.27 (s, 3 H), 1.23 (s, 3 H), 1.19 (s, 3 H), 1.15 (s, 3 H), 0.99 (s, 3 H), 0.57 (d, J = 4.4 Hz, 1 H), 0.44 (d, J = 4.4 Hz, 1 H); ESI-ms: [M+H]⁺ 789.1.

### Example 20. Synthesis of 1-(((2aR,3R,4S,5aS,5bS,7S,7aR,11aR,12aS)-9,9-difluoro-4-hydroxy-3-((2S,5S)-5-(2-hydr oxypropan-2-yl)-2-methyltetrahydrofuran-2-yl)-2a,5a,8,8-tetramethyltetradecahydro-1H,12 H-cyclopenta[a]cyclopropa[e]phenanthren-7-yl)oxy)-3-methyl-1-oxobutan-2-aminium chloride (compound 2-15).

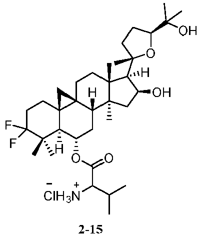

The synthesis of compound 2-15 is similar to that of compound 2-1, except that (tert-butoxycarbonyl)-DL-Valine was used instead of 3-((tert-butoxycarbonyl)amino)propionic acid.

dr=1:1,¹H NMR (400 MHz, Methanol-*d*₄) δ 4.93 (td, *J* = 9.2, 4.5 Hz, 2H), 4.66 (q, *J=* 7.7 Hz, 2H), 3.94 (d, *J =* 3.4 Hz, 1H), 3.88 (d, *J =* 3.9 Hz, 1H), 3.76 (dd, *J =* 8.4, 5.9 Hz, 2H), 2.61 (q, *J=* 10.7 Hz, 2H), 2.46 - 2.28 (m, 4H), 2.21 (dd, *J* = 13.8, 9.7 Hz, 2H), 2.11 - 1.81 (m, 15H), 1.77 - 1.57 (m, 8H), 1.55 - 1.32 (m, 8H), 1.28 (s, 3H), 1.27 (s, 3H), 1.25 (s, 6H), 1.21 (s, 6H), 1.15 (s, 2H), 1.13 (d, *J* = 1.2 Hz, 12H), 1.10 (s, 2H), 1.09 (d, *J* = 2.0 Hz, 3H), 1.07 (d, *J* = 3.0 Hz, 4H), 1.04 - 0.96 (m, 12H), 0.70 (s, 2H), 0.59 (d, *J* = 4.8 Hz, 1H), 0.51 (d, *J=* 4.9 Hz, 1H); ESI-ms: [M+H]⁺ 610.2.

### Example 21. Synthesis of 2-(((2aR,3R,4S,5aS,5bS,7S,7aR,11aR,12aS)-9,9-difluoro-4-hydroxy-3-((2S,5S)-5-(2-hydr oxypropan-2-yl)-2-methyltetrahydrofuran-2-yl)-2a,5a,8,8-tetramethyltetradecahydro-1H,12 H-cyclopenta[a]cyclopropa[e]phenanthren-7-yl)oxy)-2-oxoethan-1-aminium bromide (compound 2-16).

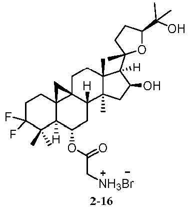

The synthesis of compound 2-16 is similar to that of compound 2-1, except that (tert-butoxycarbonyl)-glycine and hydrobromide acid were used instead of 3-((tert-butoxycarbonyl)amino)propionic acid and hydrochloric acid.

¹H NMR (400 MHz, Methanol-*d*₄) δ 4.96 (td, *J* = 9.2, 4.4 Hz, 1H), 4.65 (td, *J =* 7.9, 6.4 Hz, 1H), 4.06 (q, *J =* 7.2 Hz, 1H), 3.76 (dd, *J* = 8.4, 5.9 Hz, 1H), 3.58 (td, *J =* 6.5, 4.5 Hz, 1H), 2.62 (q, *J =* 10.8 Hz, 1H), 2.36 (d, *J =* 7.5 Hz, 1H), 2.20 (d, *J =* 9.6 Hz, 1H), 2.12 - 1.77 (m, 8H), 1.74 - 1.58 (m, 4H), 1.45 - 1.31 (m, 4H), 1.27 (s, 3H), 1.25 (s, 3H), 1.21 (s, 3H), 1.14 (s, 6H), 1.04 (s, 3H), 1.01 (s, 3H), 0.72 (d, *J* = 4.5 Hz, 1H), 0.55 (d, *J* = 4.5 Hz, 1H); ESI-ms: [M+H]⁺ 568.2.

### Example 22. Synthesis of (S)-1-(((2aR,3R,4S,5aS,5bS,7S,7aR,11aR,12aS)-9,9-difluoro-4-hydroxy-3-((2S,5S)-5-(2-h ydroxypropan-2-yl)-2-methyltetrahydrofuran-2-yl)-2a,5a,8,8-tetramethyltetradecahydro-1 H,12H-cyclopenta[a]cyclopropa[e]phenanthren-7-yl)oxy)-1-oxopropan-2-aminium chloride (compound 2-17).

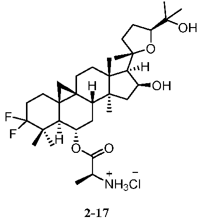

The synthesis of compound 2-17 is similar to that of compound 2-1, except that (tert-butoxycarbonyl)-L-alanine was used instead of 3-((tert-butoxycarbonyl)amino)propionic acid.

¹H NMR (400 MHz, Methanol-*d*₄) δ 4.95 (td, *J =* 9.2, 4.5 Hz, 1H), 4.66 (td, *J* = 7.9, 6.4 Hz, 1H), 4.06 (q, *J =* 7.2 Hz, 1H), 3.76 (dd, *J =* 8.4, 5.9 Hz, 1H), 3.58 (td, *J* = 6.5, 4.5 Hz, 1H), 2.61 (q, *J =* 10.8 Hz, 1H), 2.38 (d, *J =* 7.8 Hz, 1H), 2.20 (d, *J =* 9.7 Hz, 1H), 2.15 - 1.79 (m, 8H), 1.75 - 1.60 (m, 4H), 1.51 (d, *J =* 7.3 Hz, 3H), 1.44 - 1.30 (m, 4H), 1.27 (s, 3H), 1.25 (s, 3H), 1.21 (s, 3H), 1.13 (s, 6H), 1.02 (s, 3H), 1.01 (s, 3H), 0.90 (t, *J* = 7.2 Hz, 1H), 0.70 (d, *J* = 4.9 Hz, 1H), 0.56 (d, *J =* 4.9 Hz, 1H); ESI-ms: [M+H]⁺ 582.2.

### Example 23. Synthesis of (S)-1-(((2aR,3R,4S,5aS,5bS,7S,7aR,11aR,12aS)-9,9-difluoro-4-hydroxy-3-((2S,5S)-5-(2-h ydroxypropan-2-yl)-2-methyltetrahydrofuran-2-yl)-2a,5a,8,8-tetramethyltetradecahydro-1 H,12H-cyclopenta[a]cyclopropa[e]phenanthren-7-yl)oxy)-4-methyl-1-oxopentan-2-aminiu m chloride (compound 2-18).

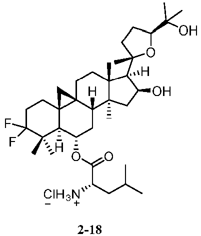

The synthesis of compound 2-18 is similar to that of compound 2-1, except that (tert-butoxycarbonyl)-L-leucine was used instead of 3-((tert-butoxycarbonyl)amino)propionic acid.

¹H NMR (400 MHz, Methanol-*d*₄) δ 4.93 (td, *J =* 9.2, 4.5 Hz, 1H), 4.63 (td, *J* = 7.9*,* 6.4 Hz, 1H), 4.03 (q, *J =* 7.2 Hz, 1H), 3.78 (dd, *J =* 8.4, 5.9 Hz, 1H), 3.57 (td, *J =* 6.5, 4.5 Hz, 1H), 2.62 (q, *J =* 10.8 Hz, 1H), 2.37 (d, *J =* 7.8 Hz, 1H), 2.21 (d, *J =* 9.7 Hz, 1H), 2.15 - 1.79 (m, 8H), 1.77 - 1.55 (m, 6H), 1.53 (d, *J =* 7.3 Hz, 6H), 1.44 - 1.30 (m, 4H), 1.27 (s, 3H), 1.25 (s, 3H), 1.21 (s, 3H), 1.13 (s, 6H), 1.03 (s, 3H), 1.01 (s, 3H), 0.91 (t, *J =* 7.2 Hz, 1H), 0.69 (d, *J =* 4.5 Hz, 1H), 0.53 (d, *J =* 4.5 Hz, 1H); ESI-ms: [M+H]⁺ 624.3.

### Example 24. Synthesis of (S)-1-(((2aR,3R,4S,5aS,5bS,7S,7aR,11aR,12aS)-9,9-difluoro-4-hydroxy-3-((2S,5S)-5-(2-h ydroxypropan-2-yl)-2-methyltetrahydrofuran-2-yl)-2a,5a,8,8-tetramethyltetradecahydro-1 H,12H-cyclopenta[a]cyclopropa[e]phenanthren-7-yl)oxy)-3-hydroxy-1-oxopropan-2-amini um chloride (compound 2-19).

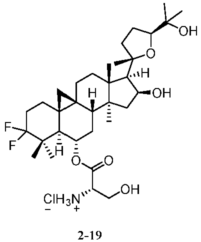

The synthesis of compound 2-19 is similar to that of compound 2-1, except that (tert-butoxycarbonyl)-L-serine was used instead of 3-((tert-butoxycarbonyl)amino)propionic acid.

¹H NMR (400 MHz, Methanol-*d*₄) δ 4.90 (td, *J =* 9.0, 4.4 Hz, 1H), 4.62 (td, *J* = 7.7, 6.4 Hz, 1H), 4.44 - 4.32 (m, 2H), 4.03 (q, *J* = 7.2 Hz, 1H), 3.73 (dd, *J* = 8.4, 5.9 Hz, 1H), 3.53 (td, *J =* 6.5, 4.5 Hz, 1H), 2.59 (q, *J=* 10.6 Hz, 1H), 2.37 (d, *J =* 7.8 Hz, 1H), 2.21 (d, *J* = 9.7 Hz, 1H), 2.13 - 1.75 (m, 8H), 1.74 - 1.58 (m, 4H), 1.47 - 1.33 (m, 4H), 1.28 (s, 3H), 1.25 (s, 3H), 1.21 (s, 3H), 1.14 (s, 6H), 1.02 (s, 3H), 0.99 (s, 3H), 0.66 (d, *J* = 4.4 Hz, 1H), 0.54 (d, *J =* 4.4 Hz, 1H); ESI-ms: [M+H]⁺ 598.2.

### Example 25. Synthesis of (2S,3S)-1-(((2aR,3R,4S,5aS,5bS,7S,7aR,11aR,12aS)-9,9-difluoro-4-hydroxy-3-((2S,5S)-5-(2-hydroxypropan-2-yl)-2-methyltetrahydrofuran-2-yl)-2a,5a,8,8-tetramethyltetradecahydr o-1H,12H-cyclopenta[a]cyclopropa[e]phenanthren-7-yl)oxy)-3-hydroxy-1-oxobutan-2-ami nium chloride (compound 2-20).

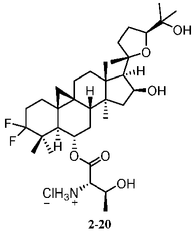

The synthesis of compound 2-20 is similar to that of compound 2-1, except that (tert-butoxycarbonyl)-L-threonine was used instead of 3-((tert-butoxycarbonyl)amino)propionic acid.

¹H NMR (400 MHz, Methanol-*d*₄) δ 4.91 (td, *J =* 9.2, 4.5 Hz, 1H), 4.62 (td, *J =* 7.8, 6.3 Hz, 1H), 4.05 (q, *J =* 7.2 Hz, 1H), 3.76 (dd, *J =* 8.4, 5.9 Hz, 1H), 3.58 (td, *J =* 6.5, 4.5 Hz, 1H), 2.63 (q, *J =* 10.7 Hz, 1H), 2.38 (d, *J =* 7.8 Hz, 1H), 2.20 (d, *J =* 9.7 Hz, 1H), 2.19- 1.83 (m, 8H), 1.79 - 1.62 (m, 4H), 1.53 (d, *J =* 7.3 Hz, 3H), 1.49 - 1.34 (m, 4H), 1.29 (s, 3H), 1.25 (s, 3H), 1.21 (s, 3H), 1.13 (s, 6H), 1.06 (s, 3H), 1.01 (s, 3H), 0.66 (d, *J =* 4.6 Hz, 1H), 0.53 (d, *J =* 4.8 Hz, 1H); ESI-ms: [M+H]⁺ 612.2.

### Example 26. Synthesis of (S)-4-amino-1-(((2aR,3R,4S,5aS,5bS,7S,7aR,11aR,12aS)-9,9-difluoro-4-hydroxy-3-((2S,5 S)-5-(2-hydroxypropan-2-yl)-2-methyltetrahydrofuran-2-yl)-2a,5a,8,8-tetramethyltetradeca hydro-1H,12H-cyclopenta[a]cyclopropa[e]phenanthren-7-yl)oxy)-1,4-dioxobutan-2-aminiu m bromide (compound 2-21).

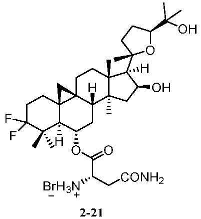

The synthesis of compound 2-21 is similar to that of compound 2-1, except that (tert-butoxycarbonyl)-L-asparagine and hydrobromide acid were used instead of 3-((tert-butoxycarbonyl)amino)propionic acid and hydrochloric acid.

¹H NMR (400 MHz, Methanol-*d*₄) δ 4.96 (td, *J =* 9.0, 4.5 Hz, 1H), 4.66 (td, *J =* 7.8, 6.4 Hz, 1H), 4.02 (q, *J =* 7.2 Hz, 1H), 3.75 (dd, *J* = 8.4, 5.5 Hz, 1H), 3.55 (td, *J =* 6.5, 4.5 Hz, 1H), 2.62 (q, *J =* 10.4 Hz, 1H), 2.38 (d, *J* = 7.8 Hz, 1H), 2.20 (d, *J* = 9.7 Hz, 1H), 2.15 - 1.79 (m, 10H), 1.75 - 1.60 (m, 4H), 1.47 - 1.33 (m, 4H), 1.27 (s, 3H), 1.25 (s, 3H), 1.21 (s, 3H), 1.14 (s, 6H), 1.04 (s, 3H), 1.00 (s, 3H), 0.68 (d, *J =* 4.6 Hz, 1H), 0.55 (d, *J =* 4.6 Hz, 1H); ESI-ms: [M+H]⁺ 625.2.

### Example 27. Synthesis of (2aR,3R,4S,5aS,5bS,7S,7aR,11aR,12aS)-9,9-difluoro-4-hydroxy-3-((2S,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-yl-L-histidinate sulfate (compound 2-22).

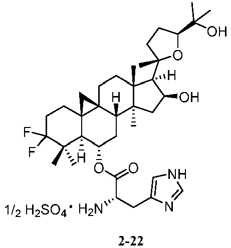

The synthesis of compound 2-22 is similar to that of compound 2-1, except that (tert-butoxycarbonyl)-histidine and sulfuric acid were used instead of 3-((tert-butoxycarbonyl)amino)propionic acid and hydrochloric acid.

¹H NMR (400 MHz, Methanol-*d*₄) δ 8.57 (s, 1H), 7.57 (s, 1H), 4.97 (td, *J =* 9.1, 4.4 Hz, 1H), 4.67 (td, *J =* 7.7, 6.4 Hz, 1H), 4.03 (q, *J* = 7.2 Hz, 1H), 3.76 (dd, *J* = 8.4, 5.9 Hz, 1H), 3.55 (td, *J =* 6.2, 4.5 Hz, 1H), 2.61 (q, *J =* 10.8 Hz, 1H), 2.35 (d, *J =* 7.8 Hz, 1H), 2.20 (d, *J* = 9.7 Hz, 1H), 2.17 - 1.73 (m, 8H), 1.71 - 1.57 (m, 4H), 1.45 - 1.31 (m, 4H), 1.27 (s, 3H), 1.25 (s, 3H), 1.21 (s, 3H), 1.15 (s, 3H), 1.11 (s, 3H),1.02 (s, 3H), 1.01 (s, 3H), 0.71 (d, *J =* 4.5 Hz, 1H), 0.55 (d, *J =* 4.5 Hz, 1H); ESI-ms: [M+H]⁺ 648.3.

### Example 28. Synthesis of 1-((S)-4-ammonio-5-(((2aR,3R,4S,5aS,5bS,7S,7aR,11aR,12aS)-9,9-difluoro-4-hydroxy-3-( (2S,5S)-5-(2-hydroxypropan-2-yl)-2-methyltetrahydrofuran-2-yl)-2a,5a,8,8-tetramethyltetr adecahydro-1H,12H-cyclopenta[a]cyclopropa[e]phenanthren-7-yl)oxy)-5-oxopentyl)guani dinium chloride (compound 2-23).

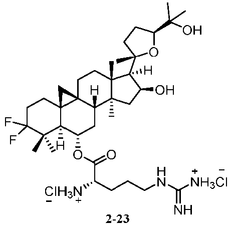

The synthesis of compound 2-23 is similar to that of compound 2-1, except that (tert-butoxycarbonyl)-L-arginine was used instead of 3-((tert-butoxycarbonyl)amino)propionic acid.

¹H NMR (400 MHz, Methanol-*d*₄) δ 4.94 (td, *J =* 9.1*,* 4.2 Hz, 1H), 4.64 (td, *J =* 7.2, 6.2 Hz, 1H), 4.04 (q, *J = 7.2* Hz, 1H), 3.76 (dd, *J* = 8.4, 5.9 Hz, 1H), 3.55 (td, *J* = 6.5, 4.5 Hz, 1H), 3.15 - 2.99 (m, 2H), 2.62 (q, *J =* 10.8 Hz, 1H), 2.38 (d, *J =* 7.8 Hz, 1H), 2.36 - 2.26 (m, 2H), 2.20 (d, *J =* 9.7 Hz, 1H), 2.15 - 1.79 (m, 10H), 1.77 - 1.62 (m, 4H), 1.49 - 1.35 (m, 4H), 1.27 (s, 3H), 1.25 (s, 3H), 1.21 (s, 3H), 1.13 (s, 3H), 1.05 (s, 3H), 1.02 (s, 3H), 0.97 (s, 3H), 0.68 (d, *J =* 4.5 Hz, 1H), 0.54 (d, *J =* 4.5 Hz, 1H); ESI-ms: [M+H]⁺ 667.2.

### Example 29. Synthesis of (S)-3-carboxy-1-(((2aR,3R,4S,5aS,5bS,7S,7aR,11aR,12aS)-9,9-difluoro-4-hydroxy-3-((2S, 5S)-5-(2-hydroxypropan-2-yl)-2-methyltetrahydrofuran-2-yl)-2a,5a,8,8-tetramethyltetradec ahydro-1H,12H-cyclopenta[a]cyclopropa[e]phenanthren-7-yl)oxy)-1-oxopropan-2-aminiu m 2,2,2-trifluoroacetate (compound 2-24).

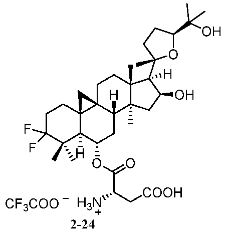

The synthesis of compound 2-8 is similar to that of compound 2-1, except that (tert-butoxycarbonyl)-L-aspartic acid and trifluoroacetic acid were used instead of 3-((tert-butoxycarbonyl)amino)propionic acid and hydrochloric acid.

¹H NMR (400 MHz, Methanol-*d*₄) δ 4.93 (td, *J =* 9.4, 4.3 Hz, 1H), 4.63 (td, *J =* 7.6, 6.1 Hz, 1H), 4.03 (q, *J =* 7.2 Hz, 1H), 3.76 (dd, *J* = 8.4, 5.9 Hz, 1H), 3.58 (td, *J =* 6.5, 4.5 Hz, 1H), 2.60 (q, *J =* 10.8 Hz, 1H), 2.39 (d, *J =* 7.8 Hz, 1H), 2.20 (d, *J =* 9.7 Hz, 1H), 2.13 - 1.76 (m, 10H), 1.73 - 1.57 (m, 4H), 1.46 - 1.31 (m, 4H), 1.27 (s, 3H), 1.25 (s, 3H), 1.21 (s, 3H), 1.13 (s, 3H), 1.08 (s, 3H), 1.02 (s, 3H), 0.98 (s, 3H), 0.67 (d, *J =* 4.4 Hz, 1H), 0.55 (d, *J =* 4.5 Hz, 1H); ESI-ms: [M-H]⁻ 624.2.

### Example 30. Synthesis of (R)-1-(((2aR,3R,4S,5aS,5bS,7S,7aR,11aR,12aS)-9,9-difluoro-4-hydroxy-3-((2S,5S)-5-(2-h ydroxypropan-2-yl)-2-methyltetrahydrofuran-2-yl)-2a,5a,8,8-tetramethyltetradecahydro-1 H,12H-cyclopenta[a]cyclopropa[e]phenanthren-7-yl)oxy)-3-mercapto-1-oxopropan-2-amin ium chloride (compound 2-25).

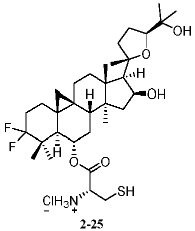

The synthesis of compound 2-25 is similar to that of compound 2-1, except that (tert-butoxycarbonyl)-L-cysteine was used instead of 3-((tert-butoxycarbonyl)amino)propionic acid.

¹H NMR (400 MHz, Methanol-*d*₄) δ 4.88 (td, *J =* 9.2, 4.4 Hz, 1H), 4.64 (td, *J* = 7.7, 6.4 Hz, 1H), 4.03 (q, *J =* 7.2 Hz, 1H), 3.73 (dd, *J* = 8.4, 5.9 Hz, 1H), 3.58 - 3.32 (m, 3H), 2.60 (q, *J =* 10.7 Hz, 1H), 2.38 (d, *J* = 7.8 Hz, 1H), 2.23 (d, *J =* 9.7 Hz, 1H), 2.15 - 1.57 (m, 12H), 1.49 - 1.35 (m, 4H), 1.29 (s, 3H), 1.25 (s, 3H), 1.21 (s, 3H), 1.14 (s, 3H), 1.07 (s, 3H), 1.02 (s, 3H), 0.99 (s, 3H), 0.68 (d, *J* = 4.6 Hz, 1H), 0.55 (d, *J* = 4.6 Hz, 1H); ESI-ms: [M-H]⁻ 614.2.

### Example 31. Synthesis of (S)-6-(((2aR,3R,4S,5aS,5bS,7S,7aR,11aR,12aS)-9,9-difluoro-4-hydroxy-3-((2S,5S)-5-(2-h ydroxypropan-2-yl)-2-methyltetrahydrofuran-2-yl)-2a,5a,8,8-tetramethyltetradecahydro-1 H,12H-cyclopenta[a]cyclopropa[e]phenanthren-7-yl)oxy)-6-oxohexane-1,5-diaminium chloride (compound 2-26).

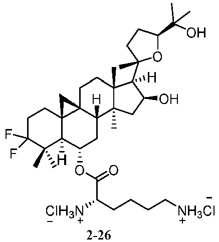

The synthesis of compound 2-26 is similar to that of compound 2-1, except that (tert-butoxycarbonyl)-L-lysine was used instead of 3-((tert-butoxycarbonyl)amino)propionic acid.

¹H NMR (400 MHz, Methanol-*d*₄) δ 4.95 (td, *J =* 9.2, 4.4 Hz, 1H), 4.64 (td, *J =* 7.8*,* 6.4 Hz, 1H), 4.09 (q, *J =* 7.2 Hz, 1H), 3.76 (dd, *J =* 8.4, 5.9 Hz, 1H), 3.58 (td, *J =* 6.5, 4.5 Hz, 1H), 2.60 (q, *J* = 10.6 Hz, 1H), 2.38 (d, *J* = 7.8 Hz, 1H), 2.23 (d, *J =* 9.6 Hz, 1H), 2.19 - 1.77 (m, 10H), 1.75 - 1.56 (m, 4H), 1.52- 1.27 (m, 10H), 1.25 (s, 3H), 1.23 (s, 3H), 1.21 (s, 3H), 1.13 (s, 3H), 1.09 (s, 3H), 1.02 (s, 3H), 1.01 (s, 3H), 0.69 (d, *J* = 4.8 Hz, 1H), 0.55 (d, *J=* 4.7 Hz, 1H); ESI-ms: [M+H]⁺ 639.2.

### Example 32. Synthesis of 2-((2-(((2aR,3R,4S,5aS,5bS,7S,7aR,11aR,12aS)-9,9-difluoro-4-hydroxy-3-((2S,5S)-5-(2-h ydroxypropan-2-yl)-2-methyltetrahydrofuran-2-yl)-2a,5a,8,8-tetramethyltetradecahydro-1 H,12H-cyclopenta[a]cyclopropa[e]phenanthren-7-yl)oxy)-2-oxoethyl)amino)-2-oxoethan-1 -aminium chloride (compound 2-27).

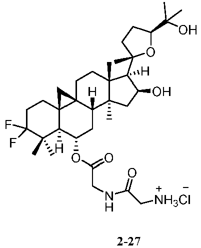

The synthesis of compound 2-27 is similar to that of compound 2-1, except that (tert-butoxycarbonyl)glycylglycine was used instead of 3-((tert-butoxycarbonyl)amino)propionic acid.

¹H NMR (400 MHz, Methanol-*d*₄) δ 4.82 (td, *J=* 9.2, 4.5 Hz, 1H), 4.65 (q, *J =* 7.5 Hz, 1H), 4.30 (d, *J* = 22.7 Hz, 1H), 3.97 (d, *J* = 9.8 Hz, 1H), 3.90 (d, *J =* 6.2 Hz, 1H), 3.78 - 3.73 (m, 3H), 2.61 (q, *J =* 10.7 Hz, 1H), 2.37 (d, *J =* 7.8 Hz, 1H), 2.13 (d, *J =* 10.3 Hz, 1H), 2.09 - 1.55 (m, 12H), 1.50 - 1.29 (m, 5H), 1.28 (s, 3H), 1.25 (s, 3H), 1.21 (s, 3H), 1.12 (s, 3H), 1.09 (s, 3H), 1.04 (s, 3H), 1.01 (s, 3H), 0.68 (d, *J =* 4.8 Hz, 1H), 0.56 (d, *J =* 4.8 Hz, 1H); ESI-ms: [M+H]⁺ 625.2.

### Example 33. Synthesis of (R)-1-((2-(((2aR,3R,4S,5aS,5bS,7S,7aR,11aR,12aS)-9,9-difluoro-4-hydroxy-3-((2S,5S)-5-(2-hydroxypropan-2-yl)-2-methyltetrahydrofuran-2-yl)-2a,5a,8,8-tetramethyltetradecahydr o-1H,12H-cyclopenta[a]cyclopropa[e]phenanthren-7-yl)oxy)-2-oxoethyl)(methyl)amino)-1 -oxo-3-phenylpropan-2-aminium chloride (compound 2-28).

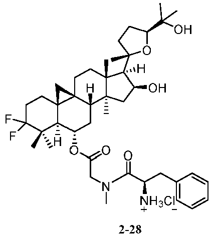

The synthesis of compound 2-27 is similar to that of compound 2-1, except that N-((tert-butoxycarbonyl)-D-phenylalanyl)-N-methylglycine was used instead of 3-((tert-butoxycarbonyl)amino)propionic acid.

¹H NMR (400 MHz, Methanol-*d*₄) δ 7.43 - 7.29 (m, 5H), 4.82 (td, *J =* 9.2, 4.5 Hz, 1H), 4.66 (qd, *J* = 7.3, 4.0 Hz, 2H), 4.08 (s, 2H), 3.76 (dd, *J* = 8.2, 5.9 Hz, 1H), 3.25 - 3.17 (m, 1H), 3.10 - 3.02 (m, 1H), 2.92 (s, 3H), 2.61 (q, *J =* 10.7 Hz, 1H), 2.37 (d, *J =* 7.8 Hz, 1H), 2.13 (d, *J =* 10.1 Hz, 1H), 2.12 - 1.87 (m, 8H), 1.84 - 1.76 (m, 1H), 1.74 - 1.55 (m, 4H), 1.50 - 1.30 (m, 5H), 1.28 (s, 3H), 1.25 (s, 3H), 1.21 (s, 3H), 1.13 (s, 3H), 1.09 (s, 3H), 1.07 (s, 3H), 1.01 (s, 3H), 0.68 (d, *J =* 4.8 Hz, 1H), 0.55 (d, *J =* 4.7 Hz, 1H); ESI-ms: [M+H]⁺ 729.2.

### Example 34. Synthesis of (S)-1-((2-(((2aR,3R,4S,5aS,5bS,7S,7aR,11aR,12aS)-9,9-difluoro-4-hydroxy-3-((2S,5S)-5-( 2-hydroxypropan-2-yl)-2-methyltetrahydrofuran-2-yl)-2a,5a,8,8-tetramethyltetradecahydro -1H,12H-cyclopenta[a]cyclopropa[e]phenanthren-7-yl)oxy)-2-oxoethyl)(methyl)amino)-1-oxopropan-2-aminium chloride (compound 2-29).

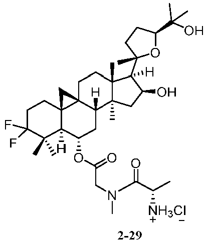

The synthesis of compound 2-27 is similar to that of compound 2-1, except that N-((tert-butoxycarbonyl)-L-alanyl)-N-methylglycine was used instead of 3-((tert-butoxycarbonyl)amino)propionic acid.

¹H NMR (400 MHz, Methanol-*d*₄) δ 4.87 (td, *J* = 9.2, 4.5 Hz, 1H), 4.64 (qd, *J* = 7.3, 4.0 Hz, 2H), 4.08 (s, 2H), 3.76 (dd, *J =* 8.2, 5.9 Hz, 1H), 3.25 - 3.18 (m, 1H), 3.11 - 3.02 (m, 1H), 2.92 (s, 3H), 2.60 (q, *J =* 10.5 Hz, 1H), 2.37 (d, *J =* 7.8 Hz, 1H), 2.15 (d, *J =* 10.0 Hz, 1H), 2.12 - 1.78 (m, 9H), 1.75 - 1.56 (m, 4H), 1.52 (d, *J* = 7.3 Hz, 1H), 1.48 - 1.31 (m, 5H), 1.27 (s, 3H), 1.25 (s, 3H), 1.21 (s, 3H), 1.13 (s, 3H), 1.07 (s, 3H), 1.07 (s, 3H), 1.01 (s, 3H), 0.67 (d, *J =* 4.8 Hz, 1H), 0.54 (d, *J =* 4.7 Hz, 1H); ESI-ms: [M+H]⁺ 653.2.

### Example 35. Synthesis of (S)-6-((2-(((2aR,3R,4S,5aS,5bS,7S,7aR,11aR,12aS)-9,9-difluoro-4-hydroxy-3-((2S,5S)-5-( 2-hydroxypropan-2-yl)-2-methyltetrahydrofuran-2-yl)-2a,5a,8,8-tetramethyltetradecahydro -1H,12H-cyclopenta[a]cyclopropa[e]phenanthren-7-yl)oxy)-2-oxoethyl)amino)-6-oxohexa ne-1,5-diaminium bromide (compound 2-30).

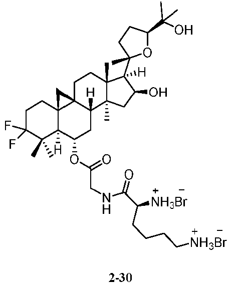

The synthesis of compound 2-30 is similar to that of compound 2-1, except that N,N-bis(tert-butoxycarbonyl)-L-lysylglycine and hydrobromide acid were used instead of 3-((tert-butoxycarbonyl)amino)propionic acid and hydrochloric acid.

¹H NMR (400 MHz, Methanol-*d*₄) δ 4.85 (td, *J* = 9.2, 4.4 Hz, 1H), 4.65 (qd, *J* = 7.3, 4.0 Hz, 2H), 4.05 (s, 2H), 3.75 (dd, *J =* 8.2, 5.7 Hz, 1H), 3.29 - 3.18 (m, 3H), 3.09 - 3.00 (m, 1H), 2.62 (q, *J =* 10.5 Hz, 1H), 2.36 (d, *J* = 7.8 Hz, 1H), 2.15 (d, *J =* 10.1 Hz, 1H), 2.11 - 1.76 (m, 11H), 1.73 - 1.53 (m, 6H), 1.51 - 1.31 (m, 7H), 1.28 (s, 3H), 1.25 (s, 3H), 1.20 (s, 3H), 1.12 (s, 3H), 1.09 (s, 3H), 1.07 (s, 3H), 0.98 (s, 3H), 0.68 (d, *J* = 4.7 Hz, 1H), 0.53 (d, *J= 4.7* Hz, 1H); ESI-ms: [M+H]⁺ 696.3.

### Example 36. Synthesis of (S)-1-((2-(((2aR,3R,4S,5aS,5bS,7S,7aR,11aR,12aS)-9,9-difluoro-4-hydroxy-3-((2S,5S)-5-( 2-hydroxypropan-2-yl)-2-methyltetrahydrofuran-2-yl)-2a,5a,8,8-tetramethyltetradecahydro -1H,12H-cyclopenta[a]cyclopropa[e]phenanthren-7-yl)oxy)-2-oxoethyl)amino)-3-hydroxy-1-oxopropan-2-aminium chloride (compound 2-31).

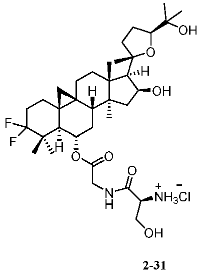

The synthesis of compound 2-31 is similar to that of compound 2-1, except that (tert-butoxycarbonyl)-L-serylglycine was used instead of 3-((tert-butoxycarbonyl)amino)propionic acid.

¹H NMR (400 MHz, Methanol-*d*₄) δ 4.83 (td, *J =* 9.2, 4.4 Hz, 1H), 4.64 (qd, *J =* 7.2, 4.0 Hz, 2H), 4.06 (s, 2H), 3.99 (dd, *J* = 8.5, 4.6 Hz, 1H), 3.82 (dd, *J* = 8.5, 4.6 Hz, 1H), 3.75 (dd, *J =* 8.2, 5.6 Hz, 1H), 3.26 - 3.16 (m, 1H), 3.11 - 3.03 (m, 1H), 2.60 (q, *J* = 10.7 Hz, 1H), 2.35 (d, *J =* 7.8 Hz, 1H), 2.15 (d, *J* = 10.1 Hz, 1H), 2.11 - 1.76 (m, 9H), 1.73 - 1.32 (m, 9H), 1.28 (s, 3H), 1.23 (s, 3H), 1.19 (s, 3H), 1.12 (s, 3H), 1.09 (s, 3H), 1.05 (s, 3H), 0.99 (s, 3H), 0.65 (d, *J* = 4.87 Hz, 1H), 0.53 (d, *J* = 4.6 Hz, 1H); ESI-ms: [M+H]⁺ 655.3.

### Example 37. Synthesis of (R)-1-((2-(((2aR,3R,4S,5aS,5bS,7S,7aR,11aR,12aS)-9,9-difluoro-4-hydroxy-3-((2S,5S)-5-(2-hydroxypropan-2-yl)-2-methyltetrahydrofuran-2-yl)-2a,5a,8,8-tetramethyltetradecahydr o-1H,12H-cyclopenta[a]cyclopropa[e]phenanthren-7-yl)oxy)-2-oxoethyl)amino)-3-mercap to-1-oxopropan-2-aminium bromide (compound 2-32).

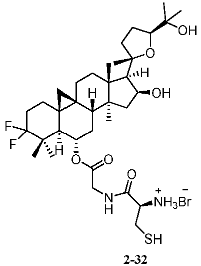

The synthesis of compound 2-32 is similar to that of compound 2-1, except that (tert-Butoxycarbonyl)-L-cysteinylglycine and hydrobromide acid were used instead of 3-((tert-butoxycarbonyl)amino)propionic acid and hydrochloric acid.

¹H NMR (400 MHz, Methanol-*d*₄) δ 4.89 (td, *J* = 9.3, 4.5 Hz, 1H), 4.63 (qd, *J* = 7.2, 4.0 Hz, 2H), 4.02 (s, 2H), 3.74 (dd, *J =* 8.2, 5.5 Hz, 1H), 3.34 - 3.19 (m, 2H), 3.16 - 3.00 (m, 2H), 2.61 (q, *J* = 10.5 Hz, 1H), 2.37 (d, *J* = 7.8 Hz, 1H), 2.20 - 1.87 (m, 9H), 1.85 - 1.55 (m, 5H), 1.53 - 1.29 (m, 5H), 1.27 (s, 3H), 1.25 (s, 3H), 1.21 (s, 3H), 1.10 (s, 3H), 1.08 (s, 3H), 1.03 (s, 3H), 0.98 (s, 3H), 0.70 (d, *J =* 4.8 Hz, 1H), 0.55 (d, *J =* 4.7 Hz, 1H); ESI-ms: [M+H]⁺ 671.3.

### Example 38. Synthesis of (R)-3-carboxy-1-((2-(((2aR,3R,4S,5aS,5bS,7S,7aR,11aR,12aS)-9,9-difluoro-4-hydroxy-3-( (2S,5S)-5-(2-hydroxypropan-2-yl)-2-methyltetrahydrofuran-2-yl)-2a,5a,8,8-tetramethyltetr adecahydro-1H,12H-cyclopenta[a]cyclopropa[e]phenanthren-7-yl)oxy)-2-oxoethyl)(methy l)amino)-1-oxopropan-2-aminium bromide (compound 2-33).

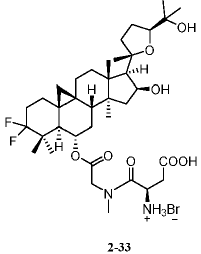

The synthesis of compound 2-33 is similar to that of compound 2-1, except that N-(4-(tert-butoxy)-2-((tert-butoxycarbonyl)amino)-4-oxobutyryl)-N-methylglycine and hydrobromide acid were used instead of 3-((tert-butoxycarbonyl)amino)propionic acid and hydrochloric acid.

¹H NMR (400 MHz, Methanol-*d*₄) δ 4.90 (td, *J =* 9.2, 4.5 Hz, 1H), 4.66 (qd, *J =* 7.3, 4.0 Hz, 2H), 4.05 (s, 2H), 3.75 (dd, *J =* 8.2, 5.9 Hz, 1H), 3.31 - 3.15 (m, 2H), 3.13 - 2.99 (m, 2H), 2.88 (s, 3H), 2.62 (q, *J =* 10.6 Hz, 1H), 2.35 (d, *J =* 7.8 Hz, 1H), 2.19 - 1.89 (m, 9H), 1.86 - 1.56 (m, 5H), 1.52 - 1.28 (m, 5H), 1.28 (s, 3H), 1.25 (s, 3H), 1.20 (s, 3H), 1.11 (s, 3H), 1.09 (s, 3H), 1.04 (s, 3H), 0.96 (s, 3H), 0.65 (d, *J =* 4.8 Hz, 1H), 0.53 (d, *J =* 4.7 Hz, 1H); ESI-ms: [M-H]⁻ 695.3.

### Example 39. Synthesis of (S)-4-amino-1-(((S)-1-(((2aR,3R,4S,5aS,5bS,7S,7aR,11aR,12aS)-9,9-difluoro-4-hydroxy-3 -((2S,5S)-5-(2-hydroxypropan-2-yl)-2-methyltetrahydrofuran-2-yl)-2a,5a,8,8-tetramethylte tradecahydro-1H,12H-cyclopenta[a]cyclopropa[e]phenanthren-7-yl)oxy)-1-oxopropan-2-yl )amino)-1,4-dioxobutan-2-aminium chloride (compound 2-34).

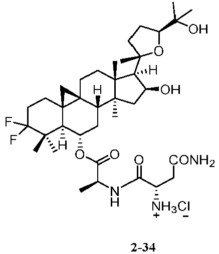

The synthesis of compound 2-34 is similar to that of compound 2-1, except that (tert-butoxycarbonyl)-L-asparagine-L-alanine was used instead of 3-((tert-butoxycarbonyl)amino)propionic acid.

¹H NMR (400 MHz, Methanol-*d*₄) δ 4.87 (td, *J* = 9.2, 4.5 Hz, 1H), 4.65 (qd, *J* = 7.2, 4.0 Hz, 2H), 3.77 (dd, *J =* 8.2, 5.9 Hz, 1H), 3.27 - 3.13 (m, 2H), 3.10 - 2.96 (m, 2H), 2.62 (q, *J* = 10.6 Hz, 1H), 2.37 (d, *J* = 7.8 Hz, 1H), 2.23 - 1.92 (m, 8H), 1.89 - 1.60 (m, 4H), 1.56 - 1.32 (m, 8H, overlapped), 1.29 (s, 3H), 1.25 (s, 3H), 1.20 (s, 3H), 1.12 (s, 3H), 1.09 (s, 3H), 1.05 (s, 3H), 0.98 (s, 3H), 0.66 (d, *J* = 4.7 Hz, 1H), 0.56 (d, *J =* 4.7 Hz, 1H); ESI-ms: [M+H]⁺ 696.3.

### Example 40. Synthesis of (R)-1-(((R)-1-(((2aR,3R,4S,5aS,5bS,7S,7aR,11aR,12aS)-9,9-difluoro-4-hydroxy-3-((2S,5S )-5-(2-hydroxypropan-2-yl)-2-methyltetrahydrofuran-2-yl)-2a,5a,8,8-tetramethyltetradecah ydro-1H,12H-cyclopenta[a]cyclopropa[e]phenanthren-7-yl)oxy)-1-oxopropan-2-yl)amino) -1-oxopropan-2-aminium iodide (compound 2-35).

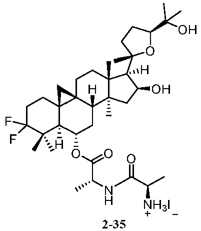

The synthesis of compound 2-35 is similar to that of compound 2-1, except that (tert-butoxycarbonyl)-D-propionamido-D-alanine was used instead of 3-((tert-butoxycarbonyl)amino)propionic acid.

¹H NMR (400 MHz, Methanol-*d*₄) δ 4.90 (td, *J =* 9.2, 4.5 Hz, 1H), 4.66 (td, *J =* 7.9, 6.4 Hz, 1H), 4.05 (q, *J* = 7.2 Hz, 1H), 3.75 (dd, *J* = 8.4, 5.9 Hz, 1H), 3.55 (td, *J* = 6.5, 4.5 Hz, 1H), 2.63 (q, *J =* 10.8 Hz, 1H), 2.38 (d, *J =* 7.8 Hz, 1H), 2.20 (d, *J =* 9.7 Hz, 1H), 2.16 - 1.62 (m, 12H), 1.54 (d, *J =* 7.2 Hz, 3H), 1.51 (d, *J =* 7.3 Hz, 3H), 1.47 - 1.33 (m, 4H), 1.28 (s, 3H), 1.25 (s, 3H), 1.21 (s, 3H), 1.13 (s, 6H), 1.02 (s, 3H), 1.0 (s, 3H), 0.93 (t, *J* = 7.2 Hz, 1H), 0.70 (d, *J* = 4.5 Hz, 1H), 0.55 (d, *J* = 4.7 Hz, 1H); ESI-ms: [M+H]⁺ 653.1.

### Example 41. Synthesis of (2aR,3R,4S,5aS,5bS,7S,7aR,11aR,12aS)-9,9-difluoro-4-hydroxy-3-((2S,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-yl dihydrogen phosphate (compound 11).

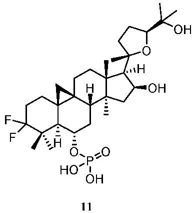

510 mg of compound 2 was dissolved in 3 mL of trimethyl phosphate, and 0.1 mL of phosphorus oxychloride dropped into the solution in an ice-water bath. The mixture was stirred for 6 hours at room temperature, then 10 mL of 2 M sodium bicarbonate was added dropwise to quench the reaction, followed by extracting twice with ethyl acetate. 2 M hydrochloric acid was added dropwise to the aqueous phase until pH=1. The mixture was placed in the refrigerator overnight to precipitate a solid, filtered and dried to obtain a white solid (295mg, 50%).

¹H NMR (400 MHz, Methanol-*d*₄) δ 4.65 (q, J = 7.0 Hz, 1 H), 3.73 (t, J = 7.2 Hz, 1 H), 3.54 (td, J = 10.2, 3.4 Hz, 1 H), 2.52 (q, J = 10.4 Hz, 1 H), 2.35 (d, J = 7.5 Hz, 1 H), 2.05-1.92 (m, 6 H), 1.80-1.55 (m, 6 H), 1.47-1.37 (m, 4 H), 1.33 (s, 3 H), 1.32 (s, 3 H), 1.28 (s, 3 H), 1.25 (s, 3 H), 1.17 (s, 3 H), 1.13 (s, 3 H), 0.95 (s, 3 H), 0.57 (d, J = 4.4 Hz, 1 H), 0.45 (d, J = 4.4 Hz, 1 H); ESI-ms: [M-H]⁻ 589.2.

### Example 42. Synthesis of monosodium mono((2aR,3R,4S,5aS,5bS,7S,7aR,11aR,12aS)-9,9-difluoro-4-hydroxy-3-((2S,5S)-5-(2-hy droxypropan-2-yl)-2-methyltetrahydrofuran-2-yl)-2a,5a,8,8-tetramethyltetradecahydro-1H, 12H-cyclopenta[a]cyclopropa[e]phenanthren-7-yl phosphate) (compound 3-1).

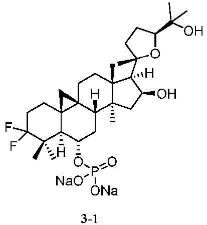

Compound 11 (590 mg, 1 mmol) was dissolved in 10 mL of methanol. 110 mg of sodium methoxide was added to the solution and stirred at room temperature overnight. 550 mg of white solid was obtained after 20 mL of acetone was added and filtered.

¹H NMR (400 MHz, Methanol-*d*₄) δ 4.68 (q, J = 7.2 Hz, 1 H), 3.76 (t, J = 7.3 Hz, 1 H), 3.52 (td, J = 10.2, 3.6 Hz, 1 H), 2.57 (q, J = 10.5 Hz, 1 H), 2.37 (d, J = 7.6 Hz, 1 H), 2.04-1.93 (m, 6 H), 1.81-1.53 (m, 6 H), 1.42-1.34 (m, 4 H), 1.32 (s, 3 H), 1.30 (s, 3 H), 1.28 (s, 3 H), 1.25 (s, 3 H), 1.17 (s, 3 H), 1.13 (s, 3 H), 0.97 (s, 3 H), 0.59 (d, J = 4.4 Hz, 1 H), 0.44 (d, J = 4.4 Hz, 1 H); ESI-ms: [M-H]⁻ 589.2.

### Example 43. Synthesis of ammonium (2aR,3R,4S,5aS,5bS,7S,7aR,11aR,12aS)-9,9-difluoro-4-hydroxy-3-((2S,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-yl phosphate (compound 3-2).

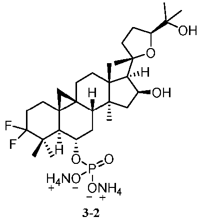

Compound 11 (590 mg, 1 mmol) was dissolved in 10 mL of methanol. 2mL of 30% ammonia was added to the solution and stirred at room temperature overnight. 620 mg of white solid was obtained after the solvent was concentrated and dried under vacuum.

¹H NMR (400 MHz, Methanol-*d*₄) δ 4.71 (q, J = 7.2 Hz, 1 H), 3.79 (t, J = 7.2 Hz, 1 H), 3.52 (td, J = 10.2, 3.6 Hz, 1 H), 2.59 (q, J = 10.5 Hz, 1 H), 2.39 (d, J = 7.5 Hz, 1 H), 2.11-1.98 (m, 6 H), 1.84-1.58 (m, 6 H), 1.49-1.39 (m, 4 H), 1.32 (s, 3 H), 1.30 (s, 3 H), 1.25 (s, 3 H), 1.21 (s, 3 H), 1.15 (s, 3 H), 1.11 (s, 3 H), 0.99 (s, 3 H), 0.58 (d, J = 4.4 Hz, 1 H), 0.43 (d, J = 4.4 Hz, 1 H); ESI-ms: [M-H]⁻ 589.2.

### Example 44. Pharmacokinetic study of compound 2.

### 1. Stability test of compound 2 in fresh rat whole blood.

### 1.1 Materials and Methods

### 1.1.1 Experimental procedure and incubation conditions

Preparation of the tested product: Compound 2 was dissolved with an appropriate amount of dimethyl sulfoxide (DMSO) to obtain a 400 µM solution.

**Table 1-1 Reagents for stability research**

| Incubation solution | Added volume/µL | Final concentration |
|---|---|---|
| compound 2 | 7 | 2 µM |
| Rat whole blood/PBS solution (with 0.5% bovine serum albumin) | 1393 | -- |

| | | |
|---|---|---|
| 1) Fresh rat whole blood is added to the EP tube and shaken gently to mix. 2) Pre-incubate the EP tube in a water bath at 37 °C for 5 minutes. 3) Add Compound 2 and incubate at 37 °C. 4) Every 50 µL of sample was taken from the sample tube to the stop tube (containing 5 µL internal standard, 5 µg/mL tolbutamide) at 0, 3 min, 6 min, 15 min, 30 min, and 60 min. 150 µL of acetonitrile was added and shaked to stop reaction, which was incubated in PBS system for 0 and 60 min. 5) Centrifuge for 5 min at 4°C(13000 r/min), aspirate the supernatant, and analyze the sample. NOTE: Duplicate tubes for each sample. | | |

### 1.2 Determination method

### 1.2.1 Instruments

Dionex's Ultimate3000 liquid chromatography system (including Ultimate3000 ternary infusion pump, Ultimate3000 column thermostat, Ultimate3000 autosampler and Ultimate3000 vacuum degasser) and AB SCIEX's 4000 Q-Trap tandem mass spectrometer equipped with electrospray ionization source (ESI source) and Analyst (version 1.6.3) data acquisition and analysis software. Electrothermal constant temperature water bath, HHS-21-6.

### 1.2.2 Measurement conditions

### 1.2.2.1 Preparation of standard solutions

Stock solution: 5 mg of compound 2 was precisely weight into a 5 mL polypropylene tube and dissolved in DMSO. 50% methanol-H₂O was added to form a stock solution containing 1 mg/mL of compound 2, which was stored in a refrigerator at 4°C.

### 1.2.2.2 Chromatographic conditions

Column: Xtimate^{™} C₁₈ (50 × 2.1mm); Injection volume: 5 µL; Mobile phase: C (0.05% ammonia), A (acetonitrile), gradient elution (see table below); Flow rate: 0.3 mL/min; Column temperature: 40°C; Sample tray temperature: 10°C

| Time/min | C% | A% |
|---|---|---|
| 0 | 50 | 50 |
| 0.1 | 5 | 95 |
| 3 | 5 | 95 |
| 3.1 | 50 | 50 |
| 5 | 50 | 50 |

### 1.2.2.3 Mass spectrometry conditions

The ion source was electrospray ionization (ESI). The drying gas (N2) temperature was 450 °C. The electrospray voltage was 4500 V. The detection method was negative ion detection. The scanning method was the selective reaction monitoring (MRM) method, and the scanning time was 0.15 s. The mass spectrometry parameters are as follows:

| Compound | parent ion /Da (Q 1 Mass) | daughter ion /Da (Q 3 Mass) | declustering voltage /V (DP) | collision voltage /eV (CE) |
|---|---|---|---|---|
| Compound 2 | 509.1 | 403.4 | -113.0 | -40.0 |
| Tolbutamide (internal standard) | 268.9 | 169.8 | -80.0 | -26.0 |

### 1.2.3 Sample processing

50 µL of rat whole blood was added with 5 µL of internal standard working solution (5 µg/mL tolbutamide). 150 µL of acetonitrile was used to precipitate the proteins. The sample was centrifuged at 13,000 rpm and 4°C for 5 min. The upper organic phase was taken and filtered with a 0.22 µM microporous membrane, which was then analyzed by LC-MS/MS.

### 1.2.4 Linear

50 µL of blank rat whole blood was taken, and 5 µL of working solutions with different concentrations were added to make a series of solutions with concentrations of 0.02, 0.05, 0.1, 0.2, 0.5, 1.0, and 2.0 µM, which was then operated according to the sample processing method.

Perform a linear regression on the concentration of the analyte in the biological sample with the ratio of the peak area of the analyte Compound 2 to the peak area of the internal standard, and use the inverse of the square of the concentration (1/x/x) as the weighting coefficient to obtain the determination of regression equation of effect and concentration.

### 2. Results

### 2.1 The stability of compound 2 in rat whole blood

There was no significant change in the concentration of compound 2 after incubation in rat whole blood for 3, 6, 15, 30, and 60 min, which means that compound 2 is relatively stable in rat whole blood. The ln residual rate of compound 2 in the incubation system was plotted against the incubation time, and the slope k was obtained by linear regression. T_{1/2} (min) was calculated according to the formula (T_{1/2}=-0.693/k).

In vitro T_{1/2} was 639.77 min.

### 2. Pharmacokinetic study

### 2.1 Materials and methods

### 2.1.1 Medicines

Compound 2.

### 2.1.2 Experimental animals

Strain: SD rat; Gender: half male and female. Weight: about 200-250 g. Source: Shanghai Slac Laboratory Animal Co., Ltd.. Laboratory Animal Production License No.: SCXK (Shanghai) 2017-0005. Laboratory Animal Use License Certificate number: SYXK (Shanghai) 2014-0018. Breeding: constant temperature purification and ventilation animal room, 20-26 °C, free food and water, humidity 40-70%, light for 12 h.

### 2.1.3 Test method

### 2.1.3.1 Method of administration

Route A: single oral administration. Volume: calculated according to 10 mL/kg body weight. Preparation: an appropriate amount of compound 2 is added to a small amount of 0.5% CMC-Na and grinded, and another portion of 0.5% CMC-Na was added to form a suspension.

Route B: single intravenous injection. Volume: calculated according to 5 mL/kg body weight. Preparation: an appropriate amount of compound 2 is dissolved in 3% total volume of DMSO. 3% total volume of castor oil is added to the solution and mixed by vortexing, an appropriate amount of normal saline is added to form an intravenous drug solution (0.4 mg/mL clear solution).

### 2.1.3.2 Administration and sample collection

12 SD rats were divided into 2 groups, 6 rats in each group, half male and half male. Fasting for 12 h before administration, free drinking water. The doses of 10 mg/kg were administered as a single gavage, and the doses of 2 mg/kg were administered as a single intravenous injection. About 100 µL orbital blood was collected before administration, 3 min, 10 min, 20 min, 30 min, 1 h, 2 h, 3 h, 4 h, 6 h, 8 h, 11 h, and 24 h after administration. For intravenous administration, orbital blood was collected before before administration, 3 min, 8 min, 15 min, 30 min, 1 h, 2 h, 3 h, 4 h, 6 h, 8 h, 11 h, 24 h after injection. The blood was anticoagulated with 1% heparin, centrifuged at 8000 rpm for 4 min, and the plasma was separated. Store at -20 °C. High concentration samples were diluted with blank plasma.

### 2.1.4 Determination method of plasma samples

### 2.1.4.1 Instrument (LC-MS/MS)

Liquid chromatography system: Ultimate3000 liquid chromatography system (including Ultimate3000 ternary infusion pump, Ultimate3000 column oven, Ultimate3000 autosampler and Ultimate3000 vacuum degasser), Dionex Corporation.
MS/MS system: 4000 Q-Trap tandem mass spectrometer with electrospray ionization source (ESI source), AB Company
Data Acquisition: Analyst (version 1.6.3), AB Company

### 2.1.4.2 Measurement Conditions

### 2.1.4.2.1 Preparation of standard solutions

Compound 2 stock solution: Precisely weigh compound 2 into a 5 mL polypropylene tube, dissolve in a small amount of methanol, and add 50% methanol-H₂O to the volume to prepare a 1 mg/mL stock solution of compound 2, which is stored in a refrigerator at 4°C away from light.

Tolbutamide stock solution: Accurately weigh 5 mg of tolbutamide into a 5 mL polypropylene tube, dissolve a small amount of methanol, and add 50% methanol-H₂O to the volume to prepare a 1 mg/mL tolbutamide stock solution, which is stored in a refrigerator at 4°C.

### 2.1.4.2.2 Chromatographic conditions

Column: Athena C₁₈-WP 100A (2.1×50 mm, 5µm); Injection volume: 10 µL; Mobile phase: A: acetonitrile; C: 0.05% ammonia, gradient elution (see table below); Flow rate: 0.3 mL /min

| Time/min | A% | C% |
|---|---|---|
| 0 | 50 | 50 |
| 0.1 | 95 | 5 |
| 3 | 95 | 5 |
| 3.1 | 50 | 50 |
| 5 | 50 | 50 |

### 2.1.4.2.3 Mass spectrometry conditions

The ion source is electrospray ionization source (ESI), the drying gas (N2) temperature is 500 °C, the electrospray voltage is -4500 V, the detection method is negative ion detection, the scanning method is the selective reaction monitoring (MRM) method and the scanning time is 0.15 s. The mass spectrometry parameters are as follows:

| Compound | parent ion /Da (Q 1 Mass) | daughter ion /Da (Q 3 Mass) | declustering voltage /V (DP) | collision voltage /eV (CE) |
|---|---|---|---|---|
| Compound 2 | 509.1 | 403.4 | -113.0 | -40.0 |
| Tolbutamide (internal standard) | 268.9 | 169.8 | -80.0 | -26.0 |

### 2.1.4.3 Sample processing

50 µL of rat whole blood was added with 2.5 µL of internal standard working solution (250 ng/mL tolbutamide). 150 µL of acetonitrile was used to precipitate the proteins. The sample was centrifuged at 13,000 rpm and 4°C for 5 min. The upper organic phase was taken and filtered with a 0.22 µM microporous membrane, which was then analyzed by LC-MS/MS.

### 2.2 Results

### 2.2.1 Methodological assessment

Under the test conditions, the test substance and the components in the sample were well separated without interference from endogenous impurity peaks.

### 2.2.1.1 Linear

The concentration of compound 2 in rat plasma was in the range of 5-500 ng/mL. The concentration of compound 2 (X) was linear with the peak area ratio (Y) of compound 2 and internal standard (tolbutamide), and the regression equations were Y = 0.0015X-0.0061, the correlation coefficient is 0.9958. The deviation between the measured value and the marked value at the lowest concentration point of the standard curve is within ±20%, and the deviation of other concentration points is within ±15%.

### 2.2.1.2 Precision and Accuracy

The accuracy and precision of the QC samples in validation met the acceptance criteria (accuracy (%REC) 104.3-107.8, precision (%RSD) 2.3-10.5.).

### 2.2.1.3 Stability test

Before the quality control samples were processed, they were placed for 3 h (room temperature), and then the stability was examined. The results show that compound 2 is stable under this condition.

The LC-MS/MS assay established in this experiment has good linearity and high sensitivity, with a linear range of 5-500 ng/mL, and impurities in plasma do not interfere with sample peaks. The intra-assay precision is <15%, and the accuracy is within 85-115%. The deviation of the plasma concentration point is within ±15% in the stability study. These results comply with the relevant regulations for the determination of biological samples.

### 2.2.2 Pharmacokinetic parameters

### 2.2.2.1 Intravenous administration

The blood concentration of compound 2 after intravenous injection of 2 mg/kg in rats is shown in Table 1. It summarizes the pharmacokinetic parameters of compound 2 after a single intravenous injection of 2 mg/kg in rats.

**Table 1 Plasma pharmacokinetic parameters after intravenous injection of compound 2 at 2 mg/kg in rats (analyzed by non-compartmental model, n=3 or 6)**

| Gender | AUC₀₋ₜ ng·h/mL | AUC_{0-∞} ng·h/mL | MRT_{0-∞} h | t_{1/2} h |
|---|---|---|---|---|
| Mean±SD♂ | 1064±113 | 1081±106 | 1.07±0.09 | 0.90±0.13 |
| Mean±SD♀ | 2117±114 | 2249±206 | 2.03±0.41 | 1.47±0.40 |
| Mean±SD | 1591±585 | 1665±656 | 1.55±0.59 | 1.18±0.41 |

| Gender | CL L/h/kg | V_{z} L/kg | C_{0.05} ng/mL | |
|---|---|---|---|---|
| Mean±SD ♂ | 1.86±0.193 | 2.39±0.168 | 1540±233 | |
| Mean±SD ♀ | 0.894±0.084 | 1.86±0.348 | 1387±250 | |
| Mean±SD | 1.38±0.547 | 2.13±0.380 | 1463±232 | |

### 2.2.2.2 Single intragastric administration

The pharmacokinetic parameters of compound 2 after a single gavage administration of 10 mg/kg to rats are summarized in Table 2 below.

**Table 2 Plasma pharmacokinetic parameters of compound 2 after gavage in rats (analyzed by non-compartmental model, n=3 or 6)**

| Gender | AUC₀₋ₜ ng·h/mL | AUC_{0-∞} ng·h/L | | MRT_{0-∞} h | t_{1/2} h | Tₘₐₓ h |
|---|---|---|---|---|---|---|
| Mean±S D♂ | 831±302 | 859±297 | | 3.78±0. 24 | 2.14±0.64 | 2.00±1.00 |
| Mean±S D♀ | 3001±2432 | 4042±3987 | | 5.01±1. 71 | 3.64±2.54 | 2.33±0.58 |
| Mean±S D | 1916±1953 | 2450 ±3071 | | 4.40±1. 28 | 2.89±1.85 | 2.17±0.75 |

| Gender | CL/F | V_{z}/F | Cₘₐₓ | F | | |
|---|---|---|---|---|---|---|
| | L/h/kg | L/kg | ng/mL | % | | |
| Mean±S D♂ | 12.5±3.6 3 | 40.0±19. 5 | 187±37. 0 | 15.6±5.7 | | |
| Mean±S D♀ | 4.27±2.7 9 | 15.8±9.2 4 | 459±277 | 28.4±23. 0 | | |
| Mean±S D | 8.38±5.3 5 | 27.9±19. 0 | 323±231 | 22.0±16. 5 | | |

### 2.3 Conclusions

The pharmacokinetic parameters (except C_{0.05}) of male and female rats showed statistical differences (P < 0.05) after intravenous injection of compound 2 at 2 mg/kg in rats. The clearance rates of male and female rats was 1.86 L/h/kg and 0.894 L/h/kg respectively. The compound was moderately cleared in male rats and was slowly cleared in the female rats. The mean plasma elimination half-lives was 0.90 h and 1.47 h respectively.

The main pharmacokinetic parameters of male and female rats did not show statistical differences (P > 0.05) after intragastric administration of 10 mg/kg compound 2. The plasma concentration peak time (Tₘₐₓ) in rats was 2.17 h, the apeak concentration (Cₘₐₓ) was 323 ng/mL, and the area under the plasma concentration-time curve (AUC₀₋ₜ) was 1916 ng·h/mL (831 ng·h/mL for males, 3001±2432 ng-h/mL for the females). The elimination half-life (t_{1/2}) was 2.89 h. The absolute bioavailability after a single intragastric administration for male and female rats was 15.6% and 28.4% respectively. The mean bioavailability was 22%.

### Example 45. Study on metabolic stability of compounds on liver microsomes

### 1. Research method:

| Main reagents | | |
|---|---|---|
| reagents | Batch number | Manufacturer |
| Human and rat liver microsomes | YAO | Rild |
| Acetonitrile | 148670 | Fisher Scientific |
| Formic acid | A0287990 | ACROS |

Preparation of the test sample: Compound 1 (prepared in Example 5), 2 (prepared in Example 4), and 5 (cycloastragenol, purchased from Chengdu Jintaihe Company) were dissolved with an appropriate amount of DMSO to obtain a concentration of 10 mM solution.

### 2. Experimental procedure and incubation conditions

**Table 1. Reagents for Stability Study of Human and Rat Liver Microsomes**

| Assay Reagent | Volumes (µl) for a 600 ul incubation volume | Final concentrations |
|---|---|---|
| Compound 1, 2, and 5 | 60 | 5 µM |
| HLM | 30 | 1 mg/mL |
| PBS | 510 | -- |

| | | |
|---|---|---|
| 1) The compound was diluted with DMSO to 0.5 mM, and diluted to 1/20 with PBS before the experiment. 2) Human liver microsomes and potassium phosphate buffer were added to the EP tube respectively and mixed by gentle shaking. 3) Pre-incubate the EP tube in a metal bath at 37 °C for 5 minutes. 4) Compound 1, 2 or 5 were added and incubated at 37 °C. 5) Take 100 µl of sample from the tube at 0, 60, 120, 240, and 360 minutes respectively, and add the sample to the stop tube (containing 100 µl of cooled acetonitrile solution). The mixture was shaken vigorously to terminate the reaction and then placed it in a -30°C refrigerator until analysis. 6) Centrifuge at 14300 r/min and 4 °C for 10 minutes, aspirate the supernatant and place it in a -30 °C refrigerator until analysis. | | |

### 3. Analysis method

### UFLC: LC-20A

Column: Shim-pack XR-ODS II, 2.0 × 75 mm; mobile phase: mobile phase B: aqueous solution containing 0.2% formic acid; mobile phase A: acetonitrile solution; mobile phase ratio:

| time (min) | 0.01 | 2.00 | 9.00 | 9.01 | 12.00 | 12.01 | 16.00 |
|---|---|---|---|---|---|---|---|
| Mobile phase B(%) | 90 | 90 | 45 | 5 | 5 | 90 | 90 |

Auto sampler temperature: 4 °C; flow rate: 0.4 ml/min; injection volume: 15 µl; run time: 16 min

### 4. Results

| | Metabolic half-life of human liver microsomes (min) | Metabolic half-life of human liver microsomes (min) |
|---|---|---|
| Compound 5 | 18.5 | 17.2 |
| Compound 1 | 233.6 | 288. |
| Compound 2 | 438.2 | 518 |

The metabolic stability of compounds 1, 2 and 5 in human and rat liver microsomes was evaluated, and it was found that compounds 1 and 2 were relatively stable in human and rat liver microsomes, which is more stable than natural product (compound 5, Cycloastragenol).

Example 46. In vivo efficacy study of compound 2 (also named HHQ16) on chronic heart failure in mice

### 1. Purpose

C57 mouse coronary artery ligation method was used in this experiment to prepare the mouse CHF model. The differences in echocardiography, hemodynamics and histopathology of the model were observed after administration.

### 2. Materials

### 2.1. Test substance

Test substance: compound 2. Solvent: 0.3% sodium carboxymethyl cellulose. Dosage: 1 mg/kg, 3 mg/kg, 10 mg/kg, 30 mg/kg and 100 mg/kg. Route of administration: intragastric. Control drug: enalapril 2mg/kg.

### 2.2. Experimental animals

Strain: C57 mouse; Age: 7 weeks; Weight: 18-20g; Gender: Male; Grade: SPF
Source: Provided by the Second Military Medical University; Rearing conditions: room temperature 20-25 °C, humidity 40-70%, standard feed, free diet and drinking water. Total number of animals: 100.

### 2.3. Test method selection and basis

Since the etiology and pathogenesis of chronic heart failure are very complex, the changes and characteristics of cardiac function in animal models of CHF prepared by different methods are different. Therefore, the preparation of CHF animal models is difficult, which also limits further research on the mechanism of drugs against heart failure.

C57 mouse CHF model was established by coronary artery ligation method in this experiment. The differences in echocardiography, hemodynamics and histopathology of the model were observed after administration.

### 2.4. Dosage setting and basis

5 dose groups were set for compound 2 in this experiment. Enalapril was selected as the positive control. Once daily for 28 consecutive days. The doses for each group was 1 mg/kg, 3 mg/kg, 10 mg/kg, 30 mg/kg and 100 mg /kg.

### 2.5. Control

Sham-operated group: sham-operated mice were given 0.3% sodium carboxymethyl cellulose.

Model control group: 0.3% sodium carboxymethyl cellulose was given after CHF was established.

Positive control group: Enalapril (2 mg/kg) was administered after CHF was established.

### 2.6. Instruments and reagents

0.9% sodium chloride injection: Zhejiang Tianrui Pharmaceutical Co., Ltd., specification: 500 ml: 4.5 g, batch number: 716022605.

H-600 transmission electron microscope: Hitachi, Japan.

Medical degreased gauze: Shanghai Honglong Medical Equipment Co., Ltd., batch number: 121112.

Disposable nursing mask: Shanghai Honglong Medical Equipment Co., Ltd., batch number: 140703.

Ruyi powder-free latex gloves: Haimen Ruyi Experimental Equipment Factory, batch number: 160606.

Disposable sterile syringe with needle (1 ml, 2 ml, 5 ml): Shanghai Kindly Enterprise Development Group Co., Ltd., batch number: K20160318.

CP-8000 Cortex pet clippers, Shenzhen Cortex Electric Co., Ltd..

YP1201N Electronic Balance: Shanghai Precision Scientific Instrument Co., Ltd..Ultrasound instrument: Esaote MyLabTMone/Touch.

Anesthesia machine: Huide Wanbang IBIS200. Isoflurane: Wald, batch number: 217170301; 217161103.

### 3. Experimental method

### 3.1. CHF animal model

The mice were anesthetized in a box filled with isoflurane. After the mice were anesthetized, the left 4th intercostal space was opened to expose the left atrial appendage and left ventricle. It can be seen that the left coronary artery runs downward from the middle of the left atrial appendage to the anterior wall of the left ventricle. The coronary artery is sutured at a high position about 1 mm from the lower border of the left atrial appendage. After ligation, the myocardium gradually changes from fleshy red to pale white, indicating myocardial infarction. The model is successfully established, and the chest is closed layer by layer. After the spontaneous breathing of the mice recovered, the mice were caged and reared in a day-night cycle with free access to water and food. After being reared for 30 days, the chronic heart failure model was detected by echocardiography, and the mice with successful chronic heart failure model were selected.

### 3.2. Experiment grouping design

Qualified CHF mice screened by echocardiography were randomly grouped. The details of this test grouping are shown in the table below:

| Groups | test substance | Dose | Route of administration | Amount |
|---|---|---|---|---|
| Sham control | 0.3%CMC | 10ml/kg | intragastric | 8 |
| Model control | 0.3%CMC | 10ml/kg | intragastric | 8 |
| positive control | Enalapril | 2mg/kg | intragastric | 8 |
| testing sample | Compound 2 | 1mg/kg | intragastric | 8 |
| testing sample | Compound 2 | 3mg/kg | intragastric | 8 |
| testing sample | Compound 2 | 10mg/kg | intragastric | 8 |
| testing sample | Compound 2 | 30mg/kg | intragastric | 9 |
| testing sample | Compound 2 | 100mg/kg | intragastric | 9 |

### 3.3. Method of administration

Mice were given compound 2 by intragastric administration once daily for 28 consecutive days.

### 3.4. Detection indicators

The body weight of mice was measured every week, and echocardiography was performed after 28 days of administration. After the mices were anesthetized, 1 mm³ tissue was taken from the hearts of 3 mice in each group, fixed with electron microscope fixative, and detected by transmission electron microscope to observe the ultrastructure of the heart. All remaining heart tissues were fixed with 10% formaldehyde, and stained with HE for histopathology to observe histopathological changes.

### 4. Experimental results and conclusions

Compound 2 significantly increased the ejection fraction (EF), short-axis shortening rate (FS), but not heart rate (HR) in mice with heart failure. The most significant effect at 10mg/kg was higher than that of the clinical gold standard drug enalapril.

The short-axis shortening rate FS% decreases in heart failure, while the FS% increased after administration of medicines. Taking enalapril (p<0.05) as the positive control, 10 mg/kg group of compound 2 has a significant increase (p<0.0005).

Example 47. Pharmacodynamic comparison between LZC696 and compound 2 (HHQ16).

### (1) Experiment 1: Selecting a mouse model of chronic heart failure by echocardiography

Compared with the sham operation group, the left ventricular systolic diameters (LVIDs) in the heart failure model group were significantly increased, and the left ventricular posterior wall thickness (LVPWd) was significantly decreased. The left ventricular ejection fraction (EF%) was significantly decreased, and the short-axis shortening rate (FS%) was significantly decreased. With EF<50%, chronic heart failure mice were selected.

**Table 1 Screening of chronic heart failure mice by echocardiography**

| group | EF (%) | FS (%) | CO (ml/min) | HR (imes/min) |
|---|---|---|---|---|
| Sham control | 85.00±6.74 | 48.98±8.05 | 34.18±0.02 | 528.31±68.65 |
| Model control | 22.57±12.86** | 8.39±5.74** | 26.65±0.02 | 544.17±120.0 1 |

| | | | | |
|---|---|---|---|---|
| Compared with the sham operation group, **P* < 0.05, ** *P* < 0.01 | | | | |

### (2) Experiment 2: Compound 2 (HHQ16) significantly improved the indicators of heart failure.

The mice were detected by cardiac ultrasound after 28 days of administration. Compared with the sham group, the heart failure model group had significantly higher LVIDs, lower LVPWd and lower short axis shortening rate (FS%). The left ventricular enlargement and the ventricular diameter is enlarged in chronic heart failure mice. The left ventricular ejection function of the chronic heart failure mice was significantly reduced, behaving as the significantly reduced EF% value. The positive control drug LCZ696 at 60mg/kg and 100mg/kg both increased the left ventricular ejection fraction EF% (increased by 33.14% and 45.36%, respectively), and the difference was significant at 100mg/kg. Meanwhile, 100mg/kg LCZ696 significantly increased the FS%. Compound 2 in the present invention significantly improved left ventricular ejection fraction EF% (increased by 75.04%), FS% and per minute cardiac output (CO), the effects were better than that of the positive control drug LCZ696 (See table 2 and the corresponding statistics).

**Table 2 Echocardiography results of compound 2**

| Group | EF (%) | | FS (%) | |
|---|---|---|---|---|
| Time | Day0 | Day28 | Day0 | Day28 |
| Sham control | 85.00±1.55 | 76.81±2.11 | 48.98±1.85 | 40.53±1.86 |
| Model control | 22.57±2.48 | 20.39±2.09 | 8.39±1.17 | 7.89±0.93 |
| Positive control 1 | 23.49±3.96 | 31.27±5.36 | 9.31±1.8 | 12.93±2.66 |
| Positive control 2 | 22.89±2.06 | 34.72±5.06 | 9.24±0.86* | 14.76±2.86 |
| Compound 2 | 25.04±1.87 | 43.83±3.62** | 9.65±0.84** | 18.86±2.17 |
| | | | | |

| Group | CO (ml/min) | | HR (times/min) | |
|---|---|---|---|---|
| Time | Day 0 | Day28 | Day 0 | Day28 |
| Sham control | 34.18±2.06 | 39.87±1.67 | 528.31±15.75 | 579±11.88 |
| Model control | 26.65±2.72 | 32.30±2.56 | 544.17±24.5 | 615.14±15.02 |
| Positive control 1 | 30.83±2.77 | 30.17±1.85 | 575.58±11.44 | 531.4±19.6 |
| Positive control 2 | 35.4±3.19 | 39.02±4.26 | 553.5±10.76 | 550.17±12.72 |
| Compound 2 | 33.39:1:2.45 | 48.09±3.69** | 564.5±14.96 | 579.3±12.44 |

| | | | | |
|---|---|---|---|---|
| Compare 28 days with 0 days, P < 0.05, ** *P < 0.01* | | | | |

### 5. Conclusions

Compound 2 showed a obvious therapeutic effect on chronic heart failure mice. Taking EF as the gold standard, compound 2 had the most significant effect at a dose of 10 mg/kg, which was better than the existing standard drug enalapril and the new drug LCZ696.

Figure 5. Ejection fraction (EF) of normal mice is 70-90%, and EF of heart failure mice is below 40%. EF is an important indicator for evaluating heart failure. Taking enalapril (p<0.01) as positive control, the EF value of 10mg/kg and 30mg/kg group mice was significantly increased (p<0.0005), while the 10mg/kg group had the highest increase (p<0.0001). The white box in the bar graph represents the 0th day, and the black box represents the 28th day.

Figure 6. Cardiac output per minute (CO), CO=SVxHR. CO decreased during heart failure and all increased after drug administration. The CO of positive control enalapril group (p<0.0001) had a very significant increase, and the CO of 10mg/kg, 30mg/kg and 100mg/kg group of compound 2 also significantly increased (p<0.01 or p<0.05). The white box in the bar graph represents the 0th day, and the black box represents the 28th day.

Figure 7. Short axis shortening rate (FS%) will decrease in heart failure model, while it increases after administration of drug. Taking Enalapril (p<0.05) as the positive control drug, the FS of mice in the 10 mg/kg group had significant elevated (p<0.0005). Histogram white box represents day 0, black box represents day 28.

Figure 8. EF, an important indicator for evaluating heart failure, which is is 70-90% in normal mice, and below 40% in mice with heart failure. The EF of mice in both LCZ696 and compound 2 groups were all significantly increased (p<0.0005), while the effect of compound 2 was more significant. The short-axis shortening rate (FS%) decreased in heart failure and increased after drug administration. The effect of compound 2 was more significant (p< 0.0005)). The white box in the histogram represents day 0, and the black box represents day 28.

Figure 9. (Heart rate (HR). HR of normal mice basically does not change, but it will change significantly in mice with heart failure. The heart rate of mice in the LCZ696 and compound 2 groups had no significant change. Cardiac output per minute (CO), CO=SVxHR. CO decreased during heart failure and all increased after drug administration. However, the increase in LCZ696 group was not statistically significant, and compound 2 group increased significantly (p<0.01)). The white box in the bar graph represents Day 0, black box represents day 28.

### Example 48. Solubility test in water and FaSSIF

### 1. Preparation of simulated intestinal fluid

FaSSIF buffer solution: 42 mg sodium hydroxide, 388.6 mg anhydrous sodium dihydrogen phosphate, and 618.6 mg sodium chloride were dissolved in water and diluted to 100 mL, pH 6.5.

FaSSIF: Add 25 mL of FaSSIF buffer solution to 56 mg of SIF powder to fully dissolve it. The solution was stirred and equilibrated in the dark for 2 hours before use.

### 2. Solubility test

The solubility of the sample in water and FaSSIF was tested at a simulated human body temperature of 37°C, and the maximum duration of the test was 24 hours. 7.5 mg of each sample was weighed, and then 1.5 mL of the above vehicle (5 mg/mL) was added to form a suspension. It was placed in a constant temperature shaker (37°C, 500 rpm) for 24 hours, and the samples were filtered at 0.5, 2 and 24 hours. Finally, the drug concentration in the filtrate was analyzed by establishing a linear curve.

### Solubility in water and FaFFIF

| Compound | Solvent | Solubility(mg/mL) | Compound | Solvent | Solubility(mg/mL) |
|---|---|---|---|---|---|
| **2** | Water | 0.002 | 5(Cycloastragenol) | Water | 0.008 |
| | FaSSIF | 0.015 | | FaSSIF | 0.049 |
| **2-1** | Water | 1.13 | **2-2** | Water | 0.88 |
| | FaSSIF | 2.18 | | FaSSIF | 1.66 |
| **2-3** | Water | 0.95 | **2-4** | Water | 1.22 |
| | FaSSIF | 1.8 | | FaSSIF | 2.5 |
| **2-5** | Water | 1.31 | **2-6** | Water | 0.89 |
| | FaSSIF | 2.41 | | FaSSIF | 1.69 |
| **2-7** | Water | 0.79 | **2-8** | Water | 2.05 |
| | FaSSIF | 1.23 | | FaSSIF | 4.02 |
| **2-9** | Water | 2.11 | **2-10** | Water | 2.28 |
| | FaSSIF | 4.52 | | FaSSIF | 4.39 |
| **2-11** | Water | 0.55 | **2-12** | Water | 0.68 |
| | FaSSIF | 1.05 | | FaSSIF | 1.11 |
| **2-13** | Water | 0.85 | **2-14** | Water | 1.23 |
| | FaSSIF | 1.15 | | FaSSIF | 2.31 |
| **2-15** | Water | 0.77 | **2-16** | Water | 0.95 |
| | FaSSIF | 3.02 | | FaSSIF | 5.3 |
| **2-17** | Water | 0.88 | **2-18** | Water | 0.6 |
| | FaSSIF | 2.39 | | FaSSIF | 1.85 |
| **2-19** | Water | 1.24 | **2-20** | Water | 1.2 |
| | FaSSIF | 3.98 | | FaSSIF | 2.8 |
| **2-21** | Water | 0.9 | **2-22** | Water | 0.7 |
| | FaSSIF | 3.5 | | FaSSIF | 1.25 |
| **2-23** | Water | 2.3 | **2-24** | Water | 1.5 |
| | FaSSIF | 6.6 | | FaSSIF | 5.45 |
| **2-25** | Water | 1.02 | **2-26** | Water | 2.1 |
| | FaSSIF | 3.35 | | FaSSIF | 6.6 |
| **2-27** | Water | 1.3 | **2-28** | Water | 0.65 |
| | FaSSIF | 4.3 | | FaSSIF | 2.85 |
| **2-29** | Water | 0.95 | **2-30** | Water | 1.9 |
| | FaSSIF | 3.13 | | FaSSIF | 5.95 |
| **2-31** | Water | 1.65 | **2-32** | Water | 1.31 |
| | FaSSIF | 6.25 | | FaSSIF | 4.96 |
| **2-33** | Water | 2.1 | **2-34** | Water | 1.18 |
| | FaSSIF | 6.8 | | FaSSIF | 3.2 |
| **2-35** | Water | 1.35 | **11** | Water | 0.79 |
| | FaSSIF | 3.14 | | FaSSIF | 3.2 |
| **3-1** | Water | 11.5 | **3-2** | Water | 12.6 |
| | FaSSIF | 15.8 | | FaSSIF | 22.3 |

### 3. Conclusion

From the results in the table, it can be seen that the aqueous solution of the compounds and their solubility in simulated intestinal fluid were greatly improved after the 6-hydroxyl of compound 2 was esterified to link a hydrophilic group or made into a phosphate prodrug, which improved the druggability significantly.

### Example 49. Pharmacokinetic Studies

1. The materials and methods for oral absorption test are the same as described in example 44.
2. Results

### 2.1 Pharmacokinetic parameters

### 2.1.1 Single intragastric administration of compound 2

The pharmacokinetic parameters of compound 2 after a single intragastric administration of 10 mg/kg to rats are summarized in Table 2 below.

| | AUC₀₋ₜ ng·h/mL | t_{1/2} h | Cₘₐₓ ng/mL |
|---|---|---|---|
| Compound 2 | 1916 | 2.89 | 323 |

### 2.1.2 Single intragastric administration of the prodrug of compound 2

The pharmacokinetic parameters of HHQ-16 measured after a single intragastric administration of 10 mg/kg of compound 2 prodrug in rats are summarized in Table 2 below.

| | AUC₀₋ₜ ng·h/mL | t_{1/2} h | Cₘₐₓ ng/mL |
|---|---|---|---|
| **Compound 2-1** | 2955 | 3.52 | 455 |
| **Compound 2-3** | 3200 | 3.98 | 460 |
| **Compound 2-6** | 3525 | 4.2 | 433 |
| **Compound 2-12** | 3873 | 2.95 | 626 |
| **Compound 2-15** | 4500 | 8.2 | 451 |
| **Compound 2-16** | 4623 | 5.2 | 510 |
| **Compound 2-17** | 5222 | 7.8 | 501 |
| **Compound 2-19** | 4400 | 3.5 | 602 |
| **Compound 2-20** | 4005 | 3.6 | 582 |
| **Compound 2-24** | 3755 | 3.2 | 425 |
| **Compound 2-27** | 3005 | 7.5 | 358 |
| **Compound 2-28** | 4332 | 6.2 | 475 |
| **Compound 2-29** | 5221 | 5.6 | 605 |
| **Compound 2-31** | 3888 | 4.5 | 403 |
| **Compound 11** | 5871 | 4.1 | 708 |

### 3. Conclusions

After oral administration of 10 mg/kg Compound 2, the Cₘₐₓ in rats was 323 ng/mL, the AUC₀₋ₜ was 1916 ng·h/mL, and the t_{1/2} was 2.89 h. The detected Cₘₐₓ and the exposure (AUC₀₋ₜ) of the parent drug HHQ-16 increased significantly after intragastric administration of the prodrug of compound 2 (the dose converted to HHQ-16 by molecular weight is 10 mg/kg). The half-life was prolonged to varying degrees. The above results show that the prodrugs of compound 2 with increased water solubility improved the absorption and increased the exposure of the parent drug in vivo.

## Claims

1. A halogenated tetracyclic triterpene derivative, **characterized in that** the derivative is compound 2: (2aR,3R,4S,5aS,5bS,7S,7aR,11aR,12aS)-9,9-difluoro-3-((2R,5S)-5-(2-hydroxypropan-2-yl )-2-methyltetrahydrofuran-2-yl)-2a,5a,8,8-tetramethyltetradecahydro-1H,12H-cyclopenta[a ]cyclopropa[e]phenanthrene-4,7-diol, represented by the following general structural formula:

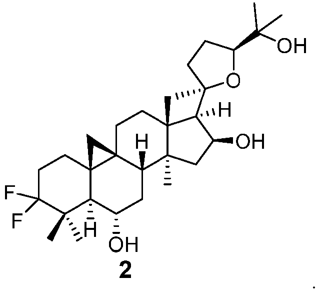

2. A halogenated tetracyclic triterpene derivative, **characterized in that** the derivative is compound 3: (2aR,3R,4S,5aS,5bS,7S,7aR,11aR,12aS)-9,9-difluoro-4-hydroxy-3-((2R,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-phosphate, represented by the following general structural formula:

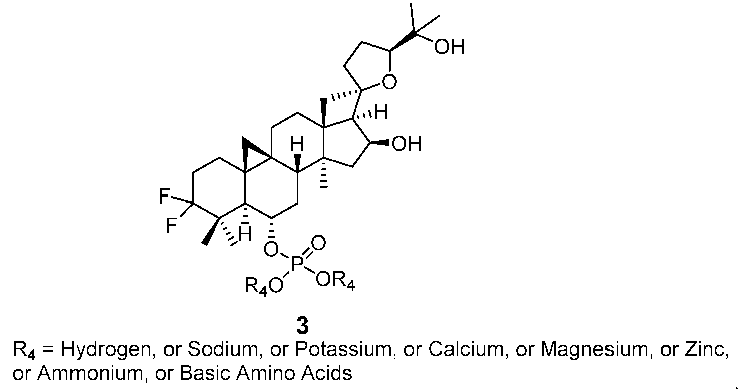

3. The halogenated tetracyclic triterpene derivative according to claim 2, charactrerized in that the derivative is a compound selected from

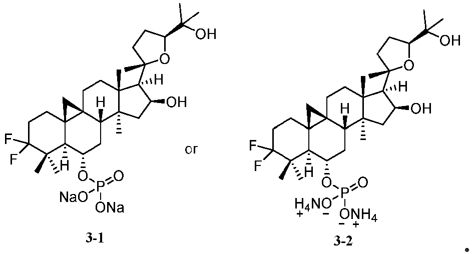

4. A halogenated tetracyclic triterpene derivative, **characterized in that** the derivative is compound 4: (2aR,3R,4S,5aS,5bS,7S,7aR,11aR,12aS)-9,9-difluoro-4-hydroxy-3-((2R,5S)-5-(2-hydroxy propan-2-yl)-2-methyltetrahydrofuran-2-yl)-2a,5a,8,8-tetramethyltetradecahydro-1H,12H-c yclopenta[a]cyclopropa[e]phenanthren-7-ester, represented by the following general structural formula:

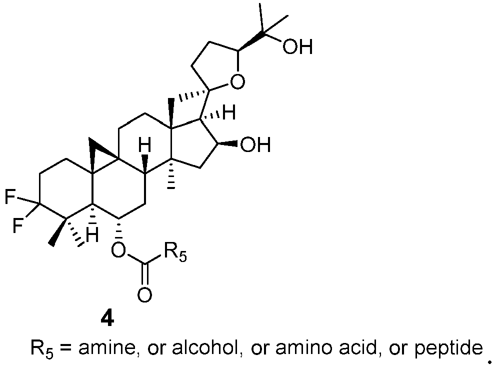

5. The halogenated tetracyclic triterpene derivative according to claim 4, **characterized in that** the derivative is a compound selected from

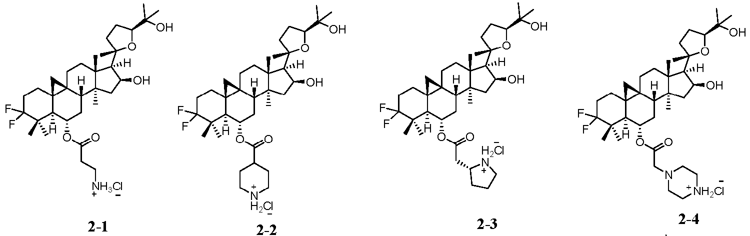

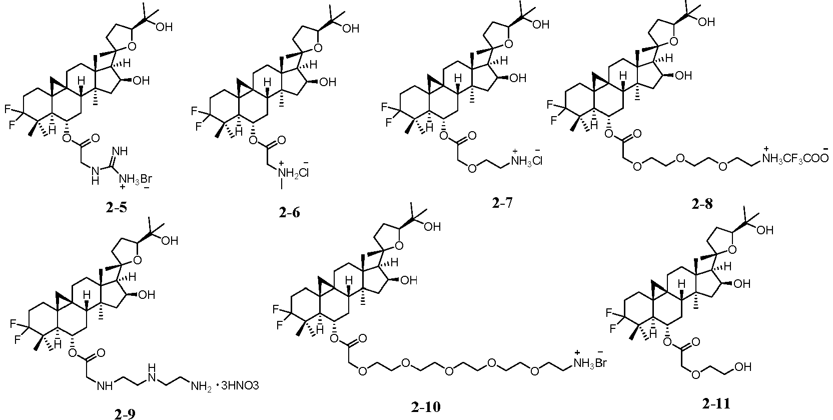

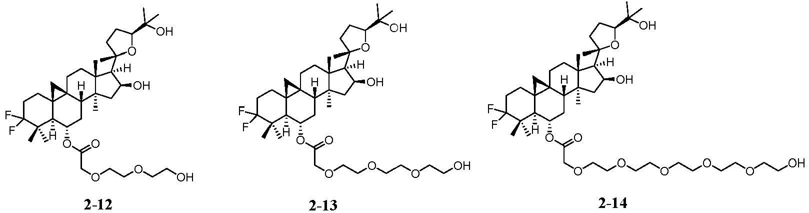

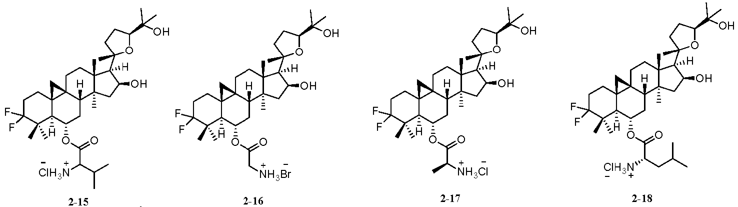

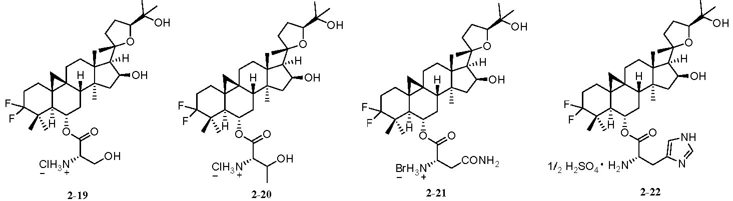

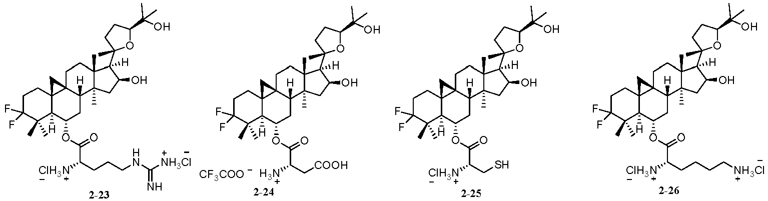

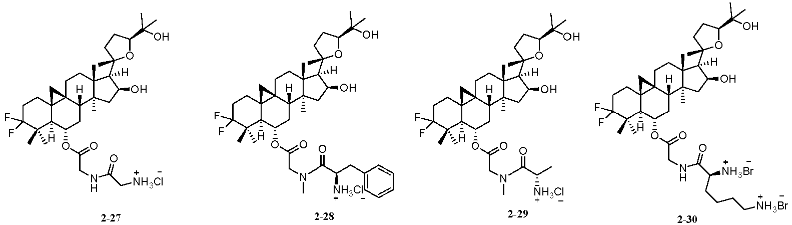

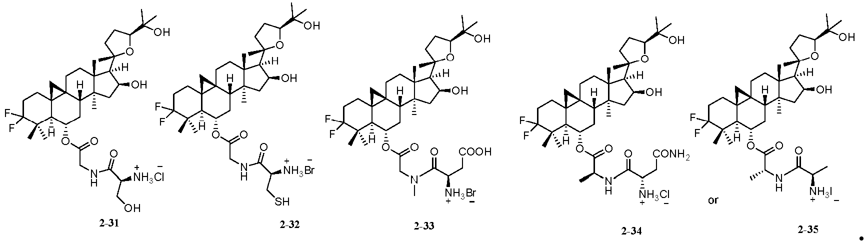

6. A preparation method of the halogenated tetracyclic triterpenoid derivative as claimed in claim 2, **characterized in that** the preparation method comprises the following steps and is represented by the following reaction formula: 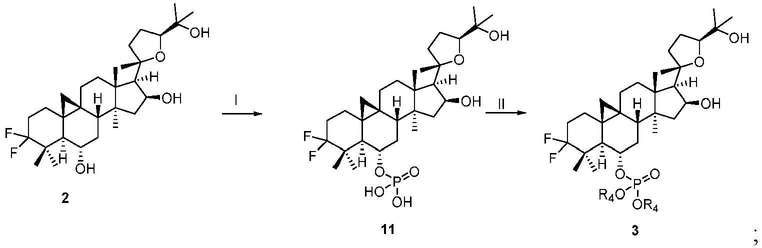 the method includes the following steps:
(I). Phosphate of 6-hydroxy: the reagent is phosphorus oxychloride, and the solvent is trimethyl phosphate; the molar ratio of compound 2 and phosphorus oxychloride is 1:1-1:15, preferably 1:3; the reaction temperature is -80°C-30 °C, preferably -5 °C to 10 °C;
(II). Salt-forming reaction: the reagent is an organic base or an inorganic base, and the organic base is selected from basic amino acids, ammonia water, methylamine, dimethylamine, diethylamine, triethylamine, pyridine, piperidine, pyrrole, ethylenediamine or butanediamine, while the inorganic base is selected from sodium hydroxide, sodium methoxide, potassium hydroxide, potassium carbonate, sodium carbonate, cesium carbonate, potassium bicarbonate, sodium acetate, potassium acetate, calcium carbonate, magnesium carbonate, sodium phosphate or potassium phosphate; the solvent is selected from tetrahydrofuran, 2-methyltetrahydrofuran, ethyl acetate, dichloromethane, dichloroethane, methanol, ethanol, isopropanol, tert-butanol, diethyl ether, methyl tert-butyl ether, acetonitrile or dioxane, preferably methanol; the reaction temperature is -20°C-50°C, preferably 0°C-20°C.

7. A preparation method of the halogenated tetracyclic triterpenoid derivative as claimed in claim 3, **characterized in that** the preparation method comprises the following steps which are represented by the following reaction formula: 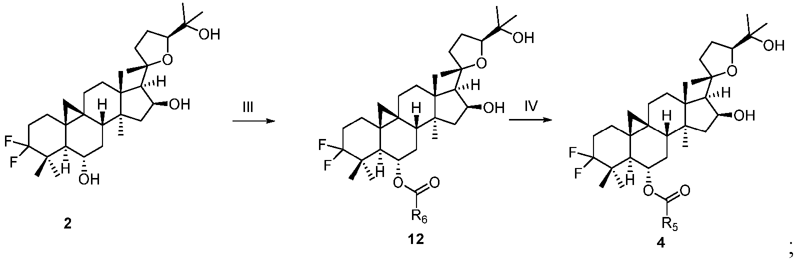 the method includes the following steps:
(III). Esterification: Compound 2 reacts with amines with a carboxylic acid group, polyethylene glycol monoacid, polyethylene diamine monoacid, nitrogen protected amino acid or peptide to get the corresponding ester; the condensing agent is n-propyl phosphoric anhydride, cyclohexylcarbodiimide (DCC), 1-ethyl-3(3-dimethylpropylamine)carbodiimide (EDCI), diisopropylcarbodiimide (DIC), carbonyldiimidazole or succinyl imine carbonate, preferably EDCI; the base is an organic base selected from diethylamine, triethylamine, pyridine, piperidine, imidazole, N,N-diisopropylethylamine, 4-pyrrolidinopyridine, 1,8-diazabicycloundec-7-ene, 4-dimethylaminopyridine or 1,4-diazabicyclo[2.2.2] octane, preferably 4-dimethylaminopyridine, the solvent is an aprotic solvent selected from toluene, xylene, tetrahydrofuran, 2-methyltetrahydrofuran, ethyl acetate, dichloromethane, dichloroethane, dimethylformamide, dimethylacetamide, dimethylsulfoxide, acetonitrile, dioxane or chloroform, preferably dichloromethane or dimethylformamide, the reaction temperature is -50°C-100°C, preferably -10 °C to 40 °C, particularly 0 °C to 20 °C;
(IV). Deprotection: the acid is an organic acid or an inorganic acid including hydrochloric acid, hydrobromic acid, hydroiodic acid, sulfuric acid, nitric acid or trifluoroacetic acid, preferably hydrochloric acid or trifluoroacetic acid; the solvent of the reaction is selected from tetrahydrofuran, 2-methyltetrahydrofuran, methanol, ethanol, isopropanol, tert-butanol, diethyl ether, methyl tert-butyl ether, acetonitrile or a mixture of one or more solvents in dioxane, preferably methanol or methanol-tetrahydrofuran mixed solvent.

8. The halogenated tetracyclic triterpene derivative according to any one of claims 1-5 for use in the treatment of a cardiovascular disease.

9. The halogenated tetracyclic triterpene derivative according to any one of claims 1-5 for use in the treatment of heart failure.

## Patentansprüche

1. Halogeniertes tetrazyklisches Triterpen-Derivat, **dadurch gekennzeichnet, dass** es sich bei dem Derivat um Verbindung 2 handelt: (2aR,3R,4S,5aS,5bS,7S,7aR,11aR,12aS)-9,9-Difluor-3-((2R,5S)-5-(2-hydroxypropan-2-yl)-2-methyltetrahydrofuran-2-yl)-2a,5a,8,8-tetramethyletradecahydro-1H,12H-cyclopenta[a]cyclopropa[e]phenanthren-4,7-diol, dargestellt durch die folgende allgemeine Strukturformel:

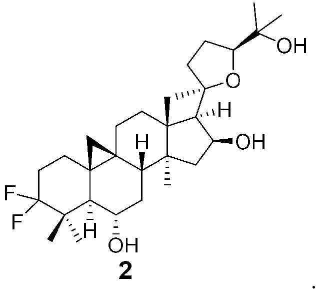

2. Halogeniertes tetrazyklisches Triterpen-Derivat, **dadurch gekennzeichnet, dass** es sich bei dem Derivat um Verbindung 3 handelt: (2aR,3R,4S,5aS,5bS,7S,7aR,11aR,12aS)-9,9-Difluor-4-hydroxy-3-((2R,5S)-5-(2-hydroxypropan-2-yl)-2-methyltetrahydrofuran-2-yl)-2a,5a,8,8-tetramethyletradecahydro-1H,12H-cyclopenta[a]cyclopropa[e]phenanthren-7-phosphat, dargestellt durch die folgende allgemeine Strukturformel:

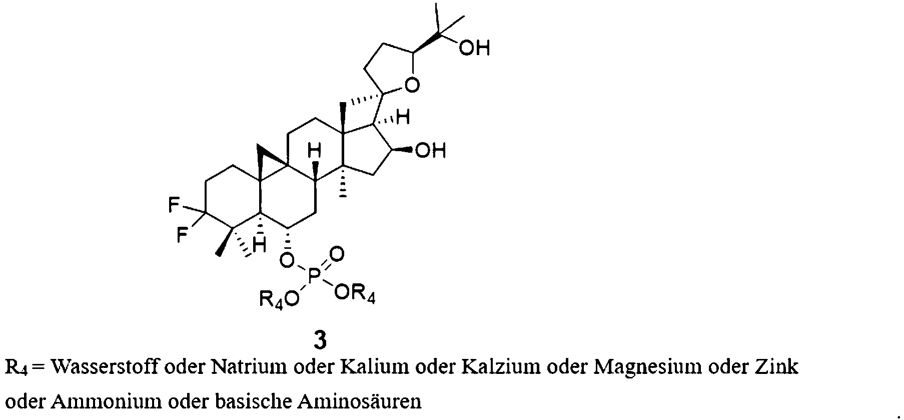

3. Halogeniertes tetrazyklisches Triterpen-Derivat nach Anspruch 2, **dadurch gekennzeichnet, dass** das Derivat eine Verbindung ist, ausgewählt aus

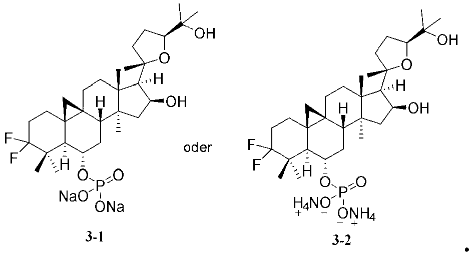

4. Halogeniertes tetrazyklisches Triterpen-Derivat, **dadurch gekennzeichnet, dass** es sich bei dem Derivat um Verbindung 4 handelt: (2aR,3R,4S,5aS,5bS,7S,7aR,11aR,12aS)-9,9-Difluor-4-hydroxy-3-((2R,5S)-5-(2-hydroxypropan-2-yl)-2-methyltetrahydrofuran-2-yl)-2a,5a,8,8-tetramethyletradecahydro-1H,12H-cyclopenta[a]cyclopropa[e]phenanthren-7-ester, dargestellt durch die folgende allgemeine Strukturformel:

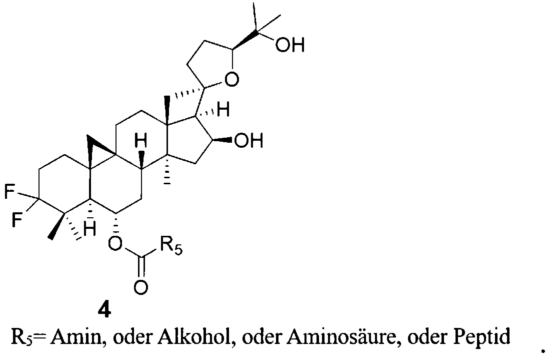

5. Halogeniertes tetrazyklisches Triterpen-Derivat nach Anspruch 4, **dadurch gekennzeichnet, dass** das Derivat eine Verbindung ist, ausgewählt aus

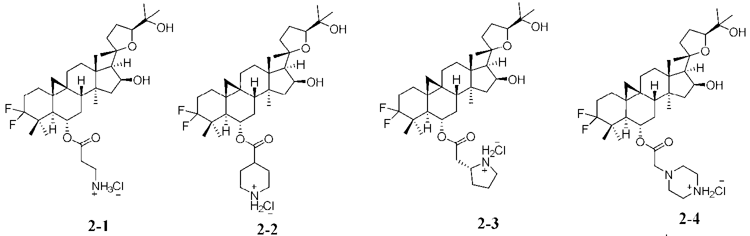

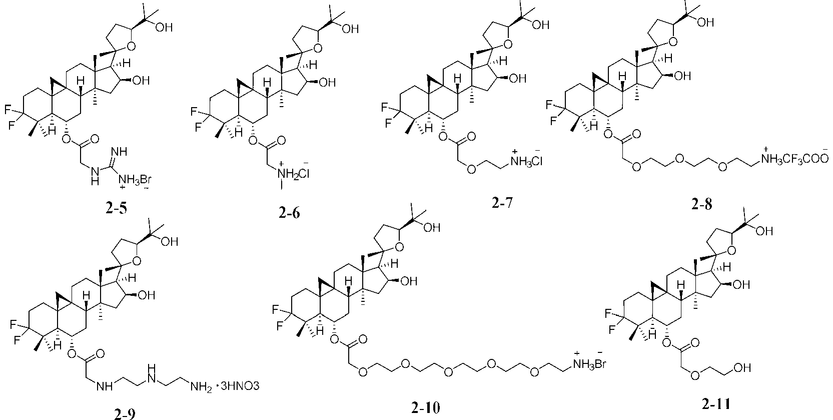

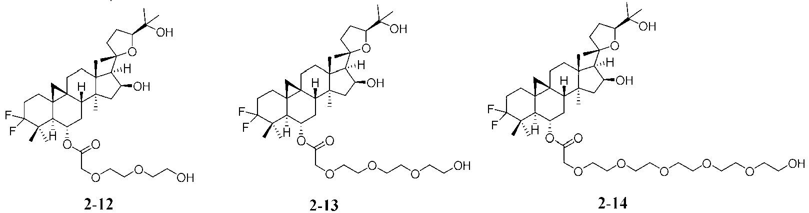

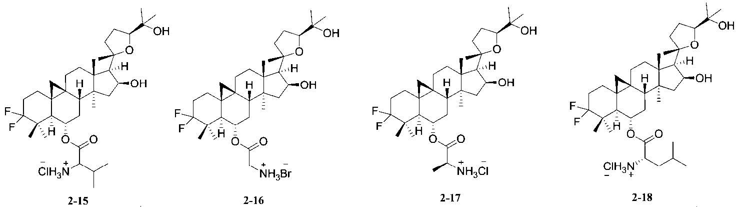

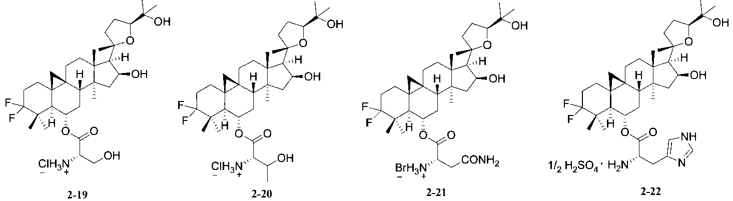

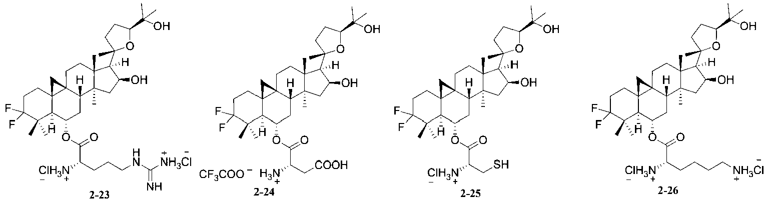

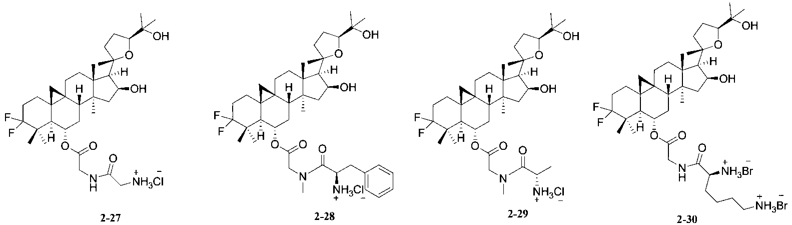

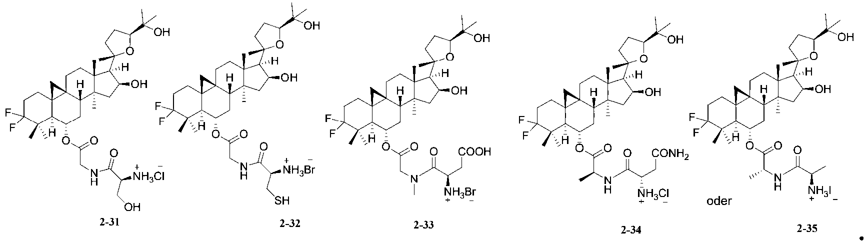

6. Herstellungsverfahren eines halogenierten tetracyclischen Triterpen-Derivats nach Anspruch 2, **dadurch gekennzeichnet, dass** das Herstellungsverfahren die folgenden Schritte umfasst und durch die folgende Reaktionsformel dargestellt wird: 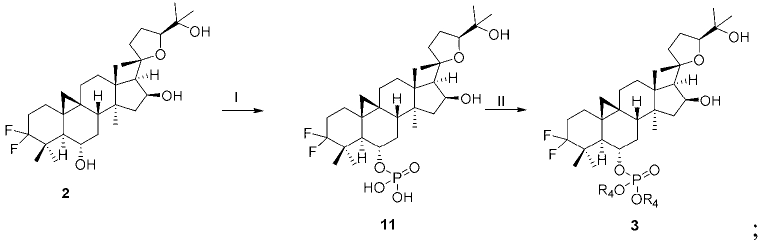 wobei das Verfahren die folgenden Schritte umfasst:
(I). Phosphorylierung von 6-Hydroxy: Das Reagenz ist Phosphoroxychlorid und das Lösungsmittel ist Trimethylphosphat; das Molverhältnis von Verbindung 2 zu Phosphoroxychlorid beträgt 1:1 bis 1:15, vorzugsweise 1:3; die Reaktionstemperatur liegt zwischen -80 °C und 30 °C, vorzugsweise zwischen -5 °C und 10 °C;
(II). Salzbildende Reaktion: das Reagenz ist eine organische Base oder eine anorganische Base, und die organische Base ist ausgewählt aus basischen Aminosäuren, Ammoniakwasser, Methylamin, Dimethylamin, Diethylamin, Triethylamin, Pyridin, Piperidin, Pyrrol, Ethylendiamin oder Butandiamin, während die anorganische Base ausgewählt ist aus Natriumhydroxid, Natriummethoxid, Kaliumhydroxid, Kaliumcarbonat, Natriumcarbonat, Cäsiumcarbonat, Kaliumbicarbonat, Natriumacetat, Kaliumacetat, Calciumcarbonat, Magnesiumcarbonat, Natriumphosphat oder Kaliumphosphat; das Lösungsmittel ausgewählt ist aus Tetrahydrofuran, 2-Methyltetrahydrofuran, Ethylacetat, Dichlormethan, Dichlorethan, Methanol, Ethanol, Isopropanol, tert-Butanol, Diethylether, Methyl-tert-Butylether, Acetonitril oder Dioxan, vorzugsweise Methanol; die Reaktionstemperatur - 20°C-50°C, vorzugsweise 0°C-20°C beträgt.

7. Herstellungsverfahren eines halogenierten tetracyclischen Triterpen-Derivats nach Anspruch 3, **dadurch gekennzeichnet, dass** das Herstellungsverfahren die folgenden Schritte umfasst, die durch die folgende Reaktionsformel dargestellt werden: 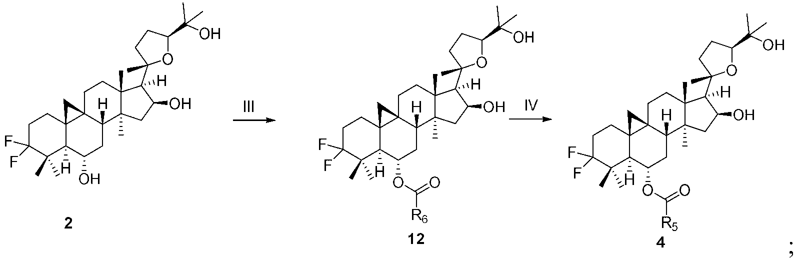 wobei das Verfahren die folgenden Schritte umfasst:
(III). Veresterung: Verbindung 2 reagiert mit Aminen mit einer Carbonsäuregruppe, Polyethylenglykol-Monosäure, Polyethylendiamin-Monosäure, stickstoffgeschützter Aminosäure oder Peptid, um den entsprechenden Ester zu erhalten; das Kondensierungsmittel ist n-Propylphosphorsäureanhydrid, Cyclohexylcarbodiimid (DCC), 1-Ethyl-3(3-dimethylpropylamin)carbodiimid (EDCI), Diisopropylcarbodiimid (DIC), Carbonydiimidazol oder Succinyimidcarbonat, vorzugsweise EDCI; die Base ist eine organische Base ausgewählt aus Diethylamin, Triethylamin, Pyridin, Piperidin, Imidazol, N,N-Diisopropylethylamin, 4-Pyrrolidinopyridin, 1,8-Diazabicycloundec-7-en, 4-Dimethylaminopyridin oder 1,4-Diazabicyclo[2.2.2]octan, vorzugsweise 4-Dimethylaminopyridin, das Lösungsmittel ist ein aprotisches Lösungsmittel ausgewählt aus Toluol, Xylol, Tetrahydrofuran, 2-Methyltetrahydrofuran, Ethylacetat, Dichlormethan, Dichlorethan, Dimethylformamid, Dimethylacetamid, Dimethylsulfoxid, Acetonitril, Dioxan oder Chloroform, vorzugsweise Dichlormethan oder Dimethylformamid, die Reaktionstemperatur beträgt -50°C bis 100°C, vorzugsweise -10 °C bis 40 °C, insbesondere 0 °C bis 20 °C;
(IV) Entschützung: Die Säure ist eine organische Säure oder eine anorganische Säure, einschließlich Salzsäure, Bromwasserstoffsäure, Iodwasserstoffsäure, Schwefelsäure, Salpetersäure oder Trifluoressigsäure, vorzugsweise Salzsäure oder Trifluoressigsäure; das Lösungsmittel der Reaktion ausgewählt wird aus Tetrahydrofuran, 2-Methyltetrahydrofuran, Methanol, Ethanol, Isopropanol, tert-Butanol, Diethylether, Methyl-tert-Butylether, Acetonitril oder einer Mischung aus einem oder mehreren Lösungsmitteln in Dioxan, vorzugsweise Methanol oder einem Methanol-Tetrahydrofuran-Mischlösungsmittel.

8. Halogeniertes tetrazyklisches Triterpen-Derivat nach einem der Ansprüche 1 bis 5 zur Verwendung bei der Behandlung einer Herz-Kreislauf-Erkrankung.

9. Halogeniertes tetrazyklisches Triterpen-Derivat nach einem der Ansprüche 1 bis 5 zur Verwendung bei der Behandlung von Herzinsuffizienz.

## Revendications

1. Dérivé triterpénoïde tétracyclique halogéné, **caractérisé en ce que** le dérivé est un composé 2: (2aR,3R,4S,5aS,5bS,7S,7aR,11aR,12aS)-9,9-difluoro-3-((2R,5S)-5-(2-hydroxypropan-2-yl)-2-méthyltétrahydrofuran-2-yl)-2a,5a,8,8-tétraméthyltétradécahydro-1H,12H-cyclopenta[a]cyclopropa[e]phénanthrène-4,7-diol, représenté par la formule structurale générale suivante :

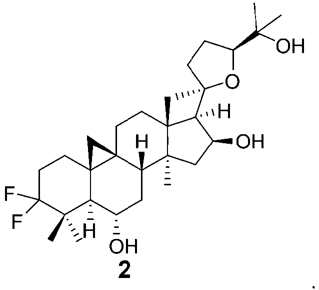

2. Dérivé triterpénoïde tétracyclique halogéné, **caractérisé en ce que** le dérivé est un composé 3: (2aR,3R,4S,5aS,5bS,7S,7aR,11aR,12aS)-9,9-difluoro-4-hydroxy-3-((2R,5S)-5-(2-hydroxypropan-2-yl)-2-méthyltétrahydrofuran-2-yl)-2a,5a,8,8-tétraméthyltétradécahydro-1H,12H-cyclopenta[a]cyclopropa[e]phénanthrène-7-phosphate, représenté par la formule structurale générale suivante :

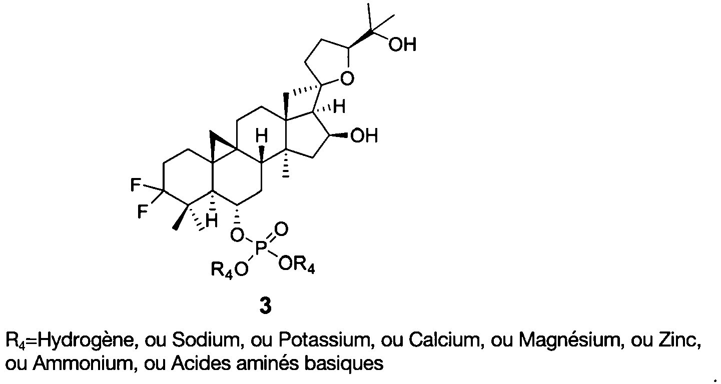

3. Dérivé triterpénoïde tétracyclique halogéné selon la revendication 2, **caractérisé en ce que** le dérivé est un composé choisi parmi

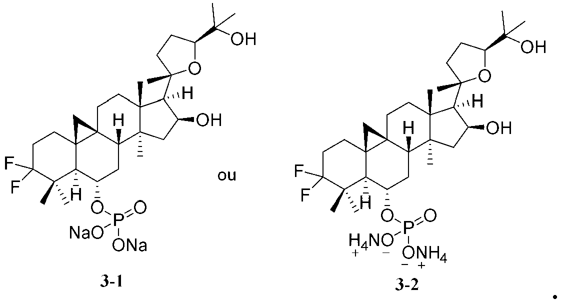

4. Dérivé triterpénoïde tétracyclique halogéné, **caractérisé en ce que** le dérivé est un composé 4: (2aR,3R,4S,5aS,5bS,7S,7aR,11aR,12aS)-9,9-difluoro-4-hydroxy-3-((2R,5S)-5-(2-hydroxypropan-2-yl)-2-méthyltétrahydrofuran-2-yl)-2a,5a,8,8-tétraméthyltétradécahydro-1H,12H-cyclopenta[a]cyclopropa[e]phénanthrène-7-ester, représenté par la formule structurale générale suivante :

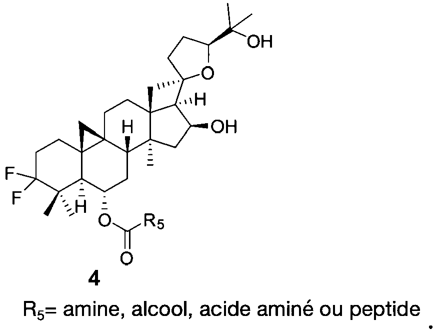

5. Dérivé triterpénoïde tétracyclique halogéné selon la revendication 4, **caractérisé en ce que** le dérivé est un composé choisi parmi

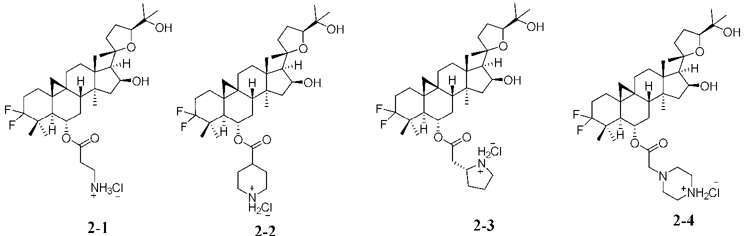

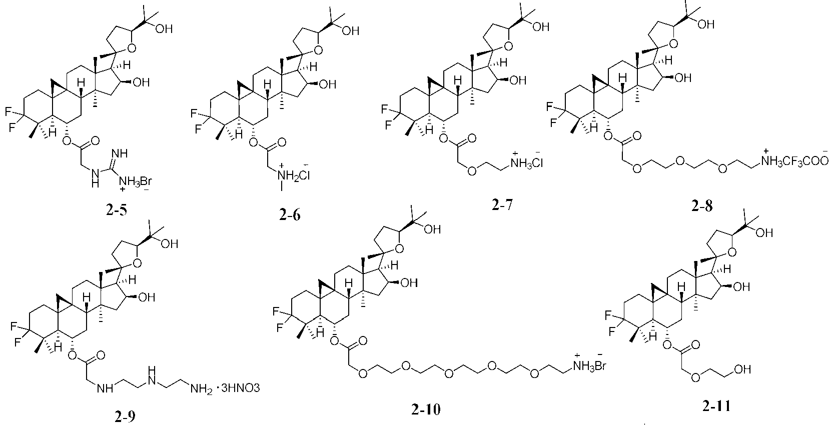

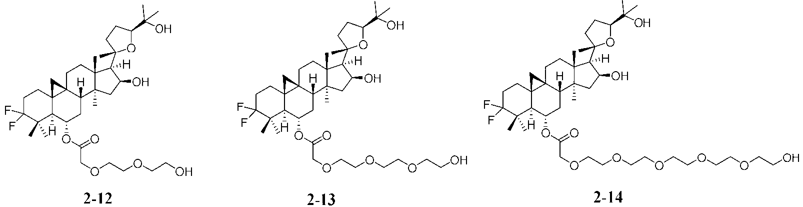

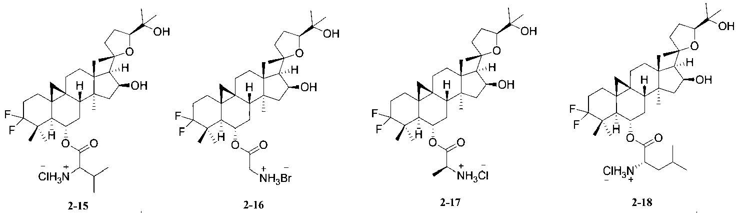

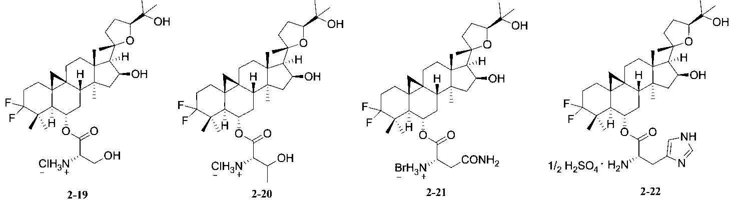

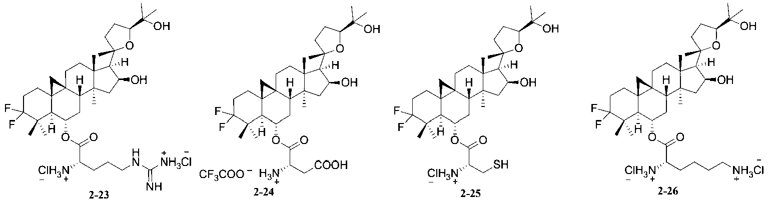

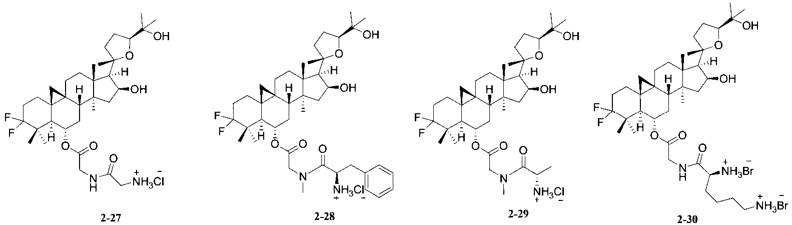

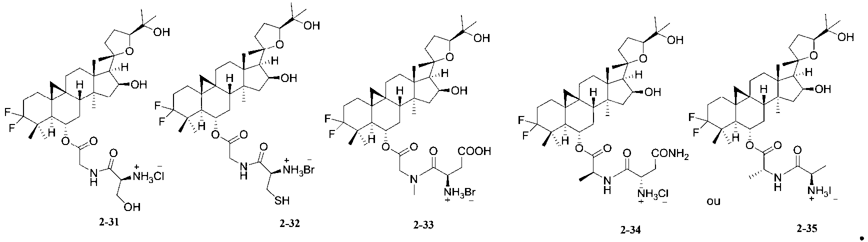

6. Procédé de préparation du dérivé triterpénoïde tétracyclique halogéné selon la revendication 2, **caractérisé en ce que** le procédé de préparation comprend les étapes suivantes et est représenté par la formule réactionnelle suivante : 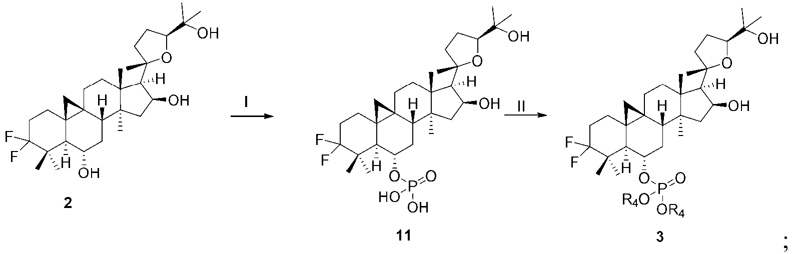 le procédé comprend les étapes suivantes :
(I). Phosphate de 6-hydroxy : le réactif est l'oxychlorure de phosphore, et le solvant est le phosphate de triméthyle ; le rapport molaire du composé 2 et de l'oxychlorure de phosphore est de 1:1 à 1:15, de préférence 1:3 ; la température de réaction est de -80 °C à -30 °C, de préférence de -5 °C à 10 °C ;
(II). Réaction de formation de sel : le réactif est une base organique ou une base inorganique, et la base organique est choisie parmi les acides aminés basiques, l'eau ammoniacale, la méthylamine, la diméthylamine, la diéthylamine, la triéthylamine, la pyridine, la pipéridine, le pyrrole, l'éthylènediamine ou la butanediamine, tandis que la base inorganique est choisie parmi l'hydroxyde de sodium, le méthylate de sodium, l'hydroxyde de potassium, le carbonate de potassium, le carbonate de sodium, le carbonate de césium, le bicarbonate de potassium, l'acétate de sodium, l'acétate de potassium, le carbonate de calcium, le carbonate de magnésium, le phosphate de sodium ou le phosphate de potassium ; le solvant est choisi parmi le tétrahydrofurane, le 2-méthyltétrahydrofurane, l'acétate d'éthyle, le dichlorométhane, le dichloroéthane, le méthanol, l'éthanol, l'isopropanol, le tert-butanol, l'éther diéthylique, le méthyl tert-butyl éther, l'acétonitrile ou le dioxane, de préférence le méthanol ; la température de réaction est de -20°C à 50°C, de préférence de 0°C à 20°C.

7. Procédé de préparation du dérivé triterpénoïde tétracyclique halogéné selon la revendication 3, **caractérisé en ce que** le procédé de préparation comprend les étapes suivantes, représentées par la formule réactionnelle suivante : 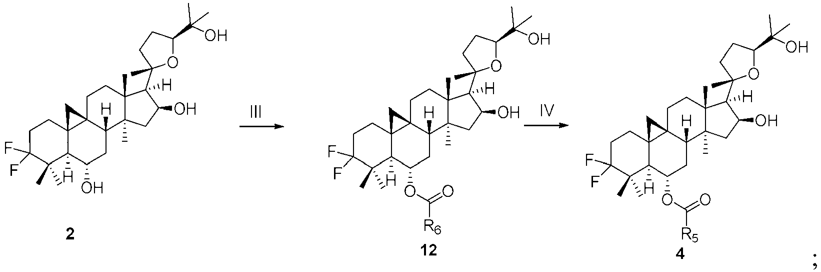 le procédé comprend les étapes suivantes :
(III). Estérification : Le composé 2 réagit avec les amines possédant un groupe acide carboxylique, le monoacide de polyéthylène glycol, le monoacide de polyéthylènediamine, l'acide aminé ou le peptide protégé par l'azote pour obtenir l'ester correspondant ; l'agent de condensation est l'anhydride n-propylphosphorique, le cyclohexylcarbodiimide (DCC), le 1-éthyl-3-(3-diméthylpropylamine)carbodiimide (EDCI), le diisopropylcarbodiimide (DIC), le carbonyldiimidazole ou le carbonate d'imine succinylique, de préférence l'EDCI ; la base est une base organique choisie parmi la diéthylamine, la triéthylamine, la pyridine, la pipéridine, l'imidazole, la N,N-diisopropyléthylamine, la 4-pyrrolidinopyridine, le 1,8-diazabicycloundéc-7-ène, la 4-diméthylaminopyridine ou le 1,4-diazabicyclo[2.2.2]octane, de préférence la 4-diméthylaminopyridine, le solvant est un solvant aprotique choisi parmi le toluène, le xylène, le tétrahydrofurane, le 2-méthyltétrahydrofurane, l'acétate d'éthyle, le dichlorométhane, le dichloroéthane, le diméthylformamide, le diméthylacétamide, le diméthylsulfoxyde, l'acétonitrile, le dioxane ou le chloroforme, de préférence le dichlorométhane ou le diméthylformamide, la température de réaction est de -50°C à -100°C, de préférence de -10°C à 40°C, en particulier de 0°C à 20°C ;
(IV). Déprotection : l'acide est un acide organique ou un acide inorganique, incluant l'acide chlorhydrique, l'acide bromhydrique, l'acide iodhydrique, l'acide sulfurique, l'acide nitrique ou l'acide trifluoroacétique, de préférence l'acide chlorhydrique ou l'acide trifluoroacétique ; le solvant de la réaction est choisi parmi le tétrahydrofurane, le 2-méthyltétrahydrofurane, le méthanol, l'éthanol, l'isopropanol, le tert-butanol, l'éther diéthylique, l'éther méthylique tert-butylique, l'acétonitrile ou un mélange d'un ou plusieurs solvants dans le dioxane, de préférence le méthanol ou un mélange méthanol-tétrahydrofurane.

8. Dérivé triterpénoïde tétracyclique halogéné selon l'une quelconque des revendications 1 à 5 pour être utilisé dans le traitement d'une maladie cardiovasculaire.

9. Dérivé triterpénoïde tétracyclique halogéné selon l'une quelconque des revendications 1 à 5 pour être utilisé dans le traitement de l'insuffisance cardiaque.
